# EUROPÄISCHE PATENTSCHRIFT

(11) **EP 1 404 656 B1**
(45) Veröffentlichungstag und Bekanntmachung des Hinweises auf die Patenterteilung: **18.05.2005**
(21) Anmeldenummer: 02740766.7
(22) Anmeldetag: 05.07.2002
(51) Int. Cl.: C07D 235/26, C07D 235/28, C07D 235/30, A61K 31/4184, A61P 31/00, A61P 37/06, A61P 37/08, C07D 401/12

(54) **BENZIMIDAZOLDERIVATE ZUR BEHANDLUNG VON MIT MIKROGLIA-AKTIVIERUNG ASSOZIIERTEN ERKRANKUNGEN WIE INFLAMMATORISCHE, ALLERGISCHE, INFEKTIÖSE ODER AUTOIMMUNE ERKRANKUNGEN**
BENZIMIDAZOLE DERIVATIVES FOR TREATING MICROGLIA-ACTIVATION ASSOCIATED DISEASES SUCH AS INFLAMMATORY, ALLERGIC, INFECTIOUS OR AUTOIMMUNE DISEASES
DERIVES DE BENZIMIDAZOLE POUR LE TRAITEMENT D'AFFECTIONS ASSOCIEES A UNE ACTIVATION DE LA MICROGLIE AINSI QUE D'AFFECTIONS INFLAMMATOIRES, ALLERGIQUES, INFECTIEUSES OU AUTO-IMMUNES

(30) Priorität: 09.07.2001 DE 10135050
(43) Veröffentlichungstag der Anmeldung: 07.04.2004
(73) Patentinhaber: SCHERING AKTIENGESELLSCHAFT, 13353 Berlin (DE)
(72) Erfinder: BLUME, Thorsten, 16552 Schildow (DE); HALFBRODT, Wolfgang, 13505 Berlin (DE); KUHNKE, Joachim, 14469 Potsdam (DE); MOENNING, Ursula, 15569 Woltersdorf (DE); SCHNEIDER, Herbert, 14129 Berlin (DE)
(74) Vertreter: Pohlman, Sandra M.
(86) Internationale Anmeldenummer: PCT/EP2002/007561
(87) Internationale Veröffentlichungsnummer: WO 2003/006438

(56) Entgegenhaltungen:
- EP-A- 0 251 536
- EP-A- 0 419 210
- EP-A- 0 531 883
- EP-A- 0 604 353
- WO-A-00/59886
- WO-A-01/00213
- WO-A-01/47883
- WO-A-01/51473
- WO-A-95/07263
- WO-A-97/10219
- DE-A- 19 816 915
- DE-A- 19 900 355
- JP-A- 2000 026 430
- US-A- 4 520 196
- US-A- 5 039 806
- US-A- 5 210 091
- US-A- 5 552 426

## Beschreibung

Die Erfindung betrifft neue Benzimidazolderivate und die Verwendung von Benzimidazolderivaten zur Herstellung von Arzneimitteln zur Behandlung und Prophylaxe von Erkrankungen, die mit einer Mikroglia-Aktivierung assoziiert sind, sowie pharmazeutische Präparate, die die neuen Benzimidazolderivate enthalten.

Nahezu alle degenerativen Erkrankungen des zentralen Nervensystems sind mit einer chronischen Entzündung verbunden. Ein zentraler Schritt des Entzündungsgeschehens ist die Aktivierung von mononukleären phagozytären Zellen, den Mikroglia. Dies erfolgt beispielsweise bei der Alzheimerschen Krankheit durch die senilen Plaques, bei der Creutzfeldt-Jakob-Krankheit durch ein Prion-Protein und beim ischämischen Schlaganfall durch abgestorbene Zellen. Die Mikroglia können über einen längeren Zeitraum in dem aktivierten Zustand bleiben, in dem sie verschiedene Entzündungsfaktoren, beispielsweise reaktive Sauerstoff/Stickstoff-Intermediate, Proteasen, Cytokine, Komplement-Faktoren und Neurotoxine produzieren und sekretieren. Diese wiederum bewirken neuronale Dysfunktion und Degeneration.

Zur Behandlung von Entzündungen und der Arteriosklerose sind u.a. Benzimidazolderivate als Wirkstoffe vorgeschlagen worden:

Beispielsweise sind in EP 0104 727 A1 Benzimidazolderivate angegeben, die in 1-Stellung nicht substituiert sind und in 2-Stellung eine Alkylgruppe aufweisen. Substituenten am Benzolring der Derivate sind u.a. Pyridyloxy-, Pyridylalkyl-, Pyridylalkyloxy- und Pyridyloxyalkandiyl-Reste.

In WO 01/21634 A1 sind ferner Benzimidazolderivate beschrieben, die in 1-Stellung eine Alkandiylamidogruppe, in 2-Stellung u.a. einen substituierten Phenyl- oder Heteroaryl-Rest und am anellierten Benzolring u.a. mindestens mit einem substituierten Alkoxy-, Alkylamino-, Alkylsulfonyl- und Alkylsulfoxid-Rest substituiert sein können. Es wird angegeben, dass diese Substanzen für eine Vielzahl möglicher Indikationen als Wirkstoff in Arzneimittelzubereitungen eingesetzt werden können.

In US-A-5,552,426 sind substituierte Benzimidazole angegeben, die in 1-Stellung u.a. einen Phenyl- oder Naphthylrest und in 2-Stellung u.a. einen Phenyl- oder Heterocyclusrest aufweisen. Der anellierte Benzolring der Benzimidazole ist vorzugsweise mit einem Alkoxy- oder Aminoalkoxyrest substituiert. Derartigen Verbindungen wird eine Wirksamkeit gegen Erkrankungen zugeschrieben, die auf einer mit einem β-Amyloid-Peptid assoziierten Neurotoxizität beruhen.

In WO 97/12613 A1 sind verschiedene entzündungshemmende und Arteriosklerose verhindernde Mittel beschrieben. Beispielsweise werden Benzimidazolderivate als Wirkstoffe angegeben, die in 1-Stellung u.a. mit einem Phenyl- oder substituierten Phenylrest und in 2-Stellung mit einem Alkoxyrest substituiert sind. Substituenten am Benzolring der Wirkstoffverbindungen können u.a. Alkyl-, Nitro-, Halogeno-, Alkoxy-, Amino-, Ester-, Amid-, Alkandiylalkoxy- und Alkandiylaminoreste sein.

In EP 0 520 200 A2 sind Benzimidazolderivate angegeben, die in 1-Stellung substituierte Arylreste und in 2-Stellung einfach-, zweifach-substituierte oder unsubstituierte Aminogruppen aufweisen. Der Benzolring des Benzimidazolgrundgerüsts kann mit Halogen, Trifluormethyl und/oder Cyano substituiert sein. Diese Verbindungen dienen zur Behandlung von Erkrankungen, die mit einer verstärkten Aktivierung von Ca-Kanälen verbunden sind.

In WO 97/33873 A1 sind ebenfalls Benzimidazolderivate angegeben, die zur Behandlung von Zystitis eingesetzt werden. Diese Verbindungen können in 1-Stellung u.a. Phenyl-, Naphthyl- und ungesättigte Heterocyclusreste aufweisen. In 2-Stellung können die Derivate mit Alkoxy-, Phenylalkoxy-, Naphthylalkoxy-, Heterocyclusalkoxy- oder ungesättigten Heterocyclusalkoxyresten substituiert sein. Der Benzolring des Grundgerüstes der Derivate kann mit Nitro-, Alkanoyl-, Amino-, Alkyl-, Alkoxy-, Cycloalkyl-, Heterocyclus-, ungesättigten Heterocyclus-, Halogeno-, Alkylthio-, Hydroxyalkylidenyl-, Hydroxyalkylidenylamino-, Aminoalkylidenyl-, Aminoalkoxy-, Hydroxyalkyl-, Heterocyclusalkoxy-, Aminoalkylidenyl- oder Trifluormethylresten substituiert sein.

In EP 0 531 883 A1 sind kondensierte 5-gliedrige Heterocyclen angegeben, beispielsweise substituierte Benzimidazolderivate, wobei diese Verbindungen gemäß der allgemeinen Beschreibung der Verbindungen in 1-Stellung vorzugsweise mit einem substituierten Alkylrest und in 2-Stellung beispielsweise mit einem O-Alkandiyl-, S-Alkandiyl-, NH-Alkandiyl-, N(Alkyl)-Alkandiyl-, SO-Alkandiyl- oder SO₂-Alkandiylrest substituiert sind. Der anellierte Benzolring kann danach u.a. mit einer Alkylenoxy-, Alkylenamino- oder Alkylenamidogruppe mit endständiger Carboxylgruppe substituiert sein. Bevorzugt sind solche Verbindungen umfasst, die in 1-Stellung unsubstituiert sind oder eine Alkylgruppe tragen. In den sehr zahlreichen Beispielen werden jedoch ausschliesslich Verbindungen genannt, die in 1-Stellung einen Aryl- oder Heterocyclusrest, insbesondere Phenylrest, oder einen Alkylrest tragen. Die beschriebenen Verbindungen sollen antithrombotische Wirksamkeit aufweisen.

In den vorstehend angegebenen Druckschriften wird ausschliesslich angegeben, dass die beschriebenen Wirkstoffe zur Behandlung von Thrombosen, der Arteriosklerose, der Zystitis und von mit einem β-Amyloid-Peptid sowie von mit einer verstärkten Aktivierung von Ca-Kanälen verbundenen Erkrankungen geeignet sind. Ein Effekt der Benzimidazolderivate auf Mikroglia ist aus den Dokumenten dagegen nicht bekannt.

Für eine mögliche Therapie der Neuroinflammation sind bisher nicht-steroidale Entzündungshemmer (COX II Inhibitoren) [McGeer, P.L., Roger, *Neurology*, 42, 447-449 (1992), Rogers, J., Kirby, L.C., Hempleman, S.R., Berry, D.L., McGeer, P.L., Kaszniak, A.W., Zalinski, J., Cofield, M., Mansukhani, L., Wilson, P., Kogan, F., *Neurology*, 43, 1609-1611 (1993), Andersen, K., Launer, L.J., Ott, A., Hoes, A.W., Breteler, M.M.B., Hofman, A., *Neurology,* 45, 1441-1445 (1995), Breitner, J.C.S., Gau, B.A., Welsh, K.A., Plassman, B.L., McDonald, W.M., Helms, M.J., Anthony, J.C., *Neurology,* 44, 227-232 (1994), The Canadian Study of Health and Aging, *Neurology,* 44, 2073-2079 (1994)], Cytokin-Modulatoren [McGeer, P.L., McGeer, E.G., *Brain Res. Rev.,* 21: 195-218 (1995), McGeer, E.G., McGeer, P.L., CNS *Drugs,* 7, 214-228 (1997), Barone, F.C. and Feuerstein, G.Z, *J. Cerebral Blood Flow and Metabolism*, 19, 819-834 (1999)] und Komplement-Kaskaden-Inhibitoren [Chen, S., Frederickson, R.C.A., and Brunden, K.R., *Neurobiol. Aging* (1996), McGeer, E.G., McGeer, P.L, *Drugs,* 55: 739-746 (1998)] beschrieben worden.

Der vorliegenden Erfindung liegt das Problem zugrunde, dass die bekannten Substanzen die Synthese oder die Wirkung einzelner Entzündungsfaktoren hemmen, ohne dass jedoch das Entzündungsgeschehen in einem früheren Schritt gehemmt wird. Von daher besteht die Aufgabe, Substanzen zu finden, die einen früheren Schritt im Entzündungsgeschehen hemmen und damit die Entstehung oder Wirkung vieler Entzündungsfaktoren verhindern.

Das Problem wird gelöst durch neuartige Benzimidazolderivate gemäß Anspruch 1, weiterhin durch eine Verwendung der erfindungsgemäßen Benzimidazolderivate zur Herstellung von Arzneimitteln zur Behandlung von mit Mikroglia-Aktivierung assoziierten Erkrankungen und zur Prophylaxe gegen diese Erkrankungen sowie pharmazeutische Präparate, enthaltend die erfindungsgemäßen Benzimidazolderivate.

Die erfindungsgemäßen Benzimidazolderivate weisen folgende allgemeine Strukturformel I auf:

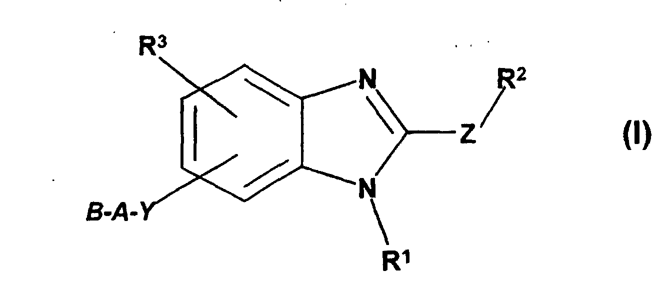

Darin sind:
- ***R***^{***1***}: eine Arylgruppe oder eine fünf- oder sechsgliedrige Heteroarylgruppe mit einem oder zwei Heteroatomen, ausgewählt aus der Gruppe, umfassend N, S und O, wobei die Aryl- oder Heteroarylgruppe mit bis zu drei Resten unabhängig voneinander substituiert sein kann, ausgewählt aus der Gruppe, umfassend:
F, Cl, Br,
C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4***}^{**'**},
C(N***R***^{***4***})N***R***^{***4***}***R***^{***4'***},
***X***-OH, ***X***-O***R***^{***4***}, ***X***-OCO***R***^{***4***}, ***X***-OCONH***R***^{***4***},
***X***-CO***R***^{***4***}, ***X***-C(NOH)***R***^{***4***},
***X***-CN, ***X***-COOH, ***X***-COO***R***^{***4***}, ***X***-CONH₂, ***X***-CON***R***^{***4***}***R***^{***4'***}, ***X***-CONH***R***^{***4***},
***X***-CONHOH,
***X***-S***R***^{***4***}, ***X***-SO***R***^{***4***}, ***X***-SO₂***R***^{***4***},
SO₂NH₂, SO₂NH***R***^{***4***},SO₂N***R***^{***4***}***R***^{***4'***},
NO₂, ***X***-NH₂, ***X***-NH***R***^{***4***}, ***X***-N***R***^{***4***}***R***^{***4'***}, ***X***-NHSO₂***R***^{***4***}, ***X***-N***R***^{***4***}SO₂***R***^{***4'***},
***X***-NHCO***R***^{***4***}, ***X***-NHCOO***R***^{***4***}, ***X***-NHCONH***R***^{***4***} und
***R***^{***4***},
wobei ***X*** eine Bindung, CH₂, (CH₂)₂ oder CH(CH₃) ist,
wobei ferner die Reste ***R***^{***4***} und ***R***^{***4'***} gemäß den weiter unten angegebenen Bedeutungen unabhängig voneinander gewählt werden und
wobei zwei Substituenten an ***R***^{***1***}, wenn sie zueinander orthoständig sind, jeweils so miteinander verknüpft sein können, dass sie gemeinsam eine Methandiylbisoxy-, Ethan-1,2-diylbisoxy-, Propan-1,3-diyl- oder Butan-1,4-diylgruppe bilden,
- ***Z***: NH, N***R***^{***2'***}, O, S, SO oder SO₂, wobei ***R***^{***2'***} die nachfolgend angegebene Bedeutung hat,
- ***R***^{***2***} und ***R***^{***2'***}: unabhängig voneinander jeweils ein Rest, ausgewählt aus der Gruppe, umfassend:
C₁₋₄-Perfluoralkyl, C₁₋₆-Alkyl, (C₀₋₃-Alkandiyl-C₃₋₇-Cycloalkyl),
(C₀₋₃-Alkandiyl-Aryl) und (C₀₋₃-Alkandiyl-Heteroaryl),
wobei die Heteroarylgruppe fünf- oder sechsgliedrig ist und ein oder zwei Heteroatome enthält, ausgewählt aus der Gruppe, umfassend N, S und O, und
wobei die Aryl- und Heteroarylgruppe jeweils mit bis zu zwei Resten, ausgewählt aus der Gruppe, umfassend F, Cl, Br, CH₃, C₂H₅, OH, OCH₃, OC₂H₅, NO₂, CF₃, C₂F₅ und SO₂NH₂, substituiert sein können und/oder auch eine anellierte Methandiylbisoxy- oder Ethan-1,2-diylbisoxygruppe tragen können, und
weiterhin ein Ringglied in einem fünfgliedrigen Cycloalkylring Ring-**N** oder Ring-O sein kann und ein oder zwei Ringglieder in einem sechs- oder siebengliedrigen Cycloalkylring Ring-**N**- und/oder Ring-O-Atome sein können, wobei die Ring-**N**-Atome gegebenenfalls mit C₁₋₃-Alkyl oder C₁₋₃-Alkanoyl substituiert sein können,
oder wenn ***Z*** N***R***^{***2'***} ist, ***R***^{***2***} und ***R***^{***2'***} gemeinsam mit ***Z*** einen fünf- bis siebengliedrigen heterocyclischen Ring bilden, wobei ferner der heterocyclische Ring ein weiteres **N**-, O- oder S-Atom enthalten und optional substituiert sein kann mit einem Rest, ausgewählt aus der Gruppe, umfassend C₁₋₄-Alkyl, (C₀₋₃-Alkandiyl-C₁₋₃-Alkoxy), C₁₋₄-Alkanoyl, C₁₋₄-Alkoxycarbonyl, Aminocarbonyl und Aryl,
- ***R***^{***3***}: unabhängig voneinander ein oder zwei Reste, ausgewählt aus der Gruppe, umfassend:
Wasserstoff,
F, Cl, Br,
OH, O***R***^{***4***}, OCO***R***^{***4***}, OCONH***R***^{***4***},
CO***R***^{***4***},
CN, COOH, COO***R***^{***4***}, CONH₂, CONH***R***^{***4***}, CON***R***^{***4***}***R***^{***4'***}, CONHOH,
CONHO***R***^{***4***},
S***R***^{***4***}, SO***R***^{***4***}, SO₂***R***^{***4***}, SO₂NH₂, SO₂NH***R***^{***4***}, SO₂N***R***^{***4***}***R***^{***4'***},
NO₂, NH₂, NH***R***^{***4***}, N***R***^{***4***}***R***^{***4'***},
NHSO₂***R***^{***4***}, N***R***^{***4***}SO₂***R***^{***4'***}, NHSO₂***R***^{***6***}, N***R***^{***4***}SO₂***R***^{***6***},
NHCO***R***^{***4***}, NHCOO***R***^{***4***}, NHCONH***R***^{***4***} und ***R***^{***4***},
wobei die Reste ***R***^{***4***}, ***R***^{***4'***} und ***R***^{***6***} gemäß den weiter unten angegebenen Bedeutungen unabhängig voneinander gewählt werden,
- ***A***: eine Gruppe, ausgewählt aus der Gruppe, umfassend C₁₋₁₀-Alkandiyl, C₂₋₁₀-Alkendiyl, C₂₋₁₀-Alkindiyl und (C₀₋₃-Alkandiyl-C₃₋₇-Cycloalkandiyl-C₀₋₃-Alkandiyl), wobei in einem fünfgliedrigen Cycloalkylring ein Ringglied Ring-**N** oder Ring-O sein kann und in einem sechs- oder siebengliedrigen Cycloalkylring ein oder zwei Ringglieder jeweils Ring-**N**- und/oder Ring-O-Atome sein können, wobei die Ring-**N**-Atome gegebenenfalls mit C₁₋₃-Alkyl oder
C₁₋₃-Alkanoyl substituiert sein können,
wobei in den oben genannten aliphatischen Ketten ein C-Atom gegen O, **N**H, **N**-C₁₋₃-Alkyl oder **N**-C₁₋₃-Alkanoyl ausgetauscht sein kann und wobei Alkyl- oder Cycloalkylgruppen optional mit einem Rest, ausgewählt aus der Gruppe, umfassend =O, OH, O-C₁₋₃-Alkyl, **N**H₂, **N**H-C₁₋₃-Alkyl, NH-C₁₋₃-Alkanoyl, **N**(C₁₋₃-Alkyl)₂ und **N**(C₁₋₃-Alkyl)(C₁₋₃-Alkanoyl), substituiert sein können,
- ***B***: ein Rest, ausgewählt aus der Gruppe, umfassend COOH, COO***R***^{***5***}, CONH₂, CONHNH₂, CONH***R***^{**5**}, CON***R***^{***5***}***R***^{***5'***}, CONHOH, CONHO***R***^{***5***} und
Tetrazolyl,
jeweils gebunden an ein C-Atom der Gruppe ***A,***
wobei die Reste ***R***^{***5***} und ***R***^{***5'***} unabhängig voneinander gemäß den weiter unten angegebenen Bedeutungen gewählt werden,
- ***Y***: für O steht
worin in den vorstehenden Resten die Reste ***R***^{***4***}, ***R***^{***4'***}, ***R***^{***5***}, ***R***^{***5'***} und ***R***^{***6***} die folgenden Bedeutungen haben; darin sind:
- ***R***^{***4***} und ***R***^{***4'***}: unabhängig voneinander jeweils ein Rest, ausgewählt aus der Gruppe, umfassend CF₃, C₂F₅, C₁₋₄-Alkyl, C₂₋₄-Alkenyl, C₂₋₃-Alkinyl und
(C₀₋₃-Alkandiyl-C₃₋₇-Cycloalkyl),
wobei in einem fünfgliedrigen Cycloalkylring ein Ringglied Ring-**N** oder Ring-O sein kann und in einem sechs- oder siebengliedrigen Cycloalkylring ein oder zwei Ringglieder jeweils Ring-**N**- und/oder Ring-O-Atome sein können, wobei die Ring-**N**-Atome gegebenenfalls mit C₁₋₃-Alkyl oder C₁₋₃-Alkanoyl substituiert sein können,
- ***R***^{***5***} und ***R***^{***5'***}: unabhängig voneinander jeweils ein Rest, ausgewählt aus der Gruppe, umfassend C₁₋₆-Alkyl, C₂₋₆-Alkenyl, C₂₋₆-Alkinyl, wobei ein C-Atom gegen O, S, SO, SO₂, **N**H, **N**-C₁₋₃-Alkyl oder **N**-C₁₋₃-Alkanoyl ausgetauscht sein kann, ferner (C₀₋₃-Alkandiyl-C₃₋₇-Cycloalkyl), wobei in einem fünfgliedrigen Cycloalkylring ein Ringglied Ring-**N** oder Ring-O sein kann und in einem sechs- oder siebengliedrigen Cycloalkylring ein oder zwei Ringglieder jeweils Ring-**N**- und/oder Ring-O-Atome sein können, wobei die Ring-**N**-Atome gegebenenfalls mit C₁₋₃-Alkyl oder C₁₋₃-Alkanoyl substituiert sein können, sowie ferner (C₀₋₃-Alkandiyl-Aryl) und (C₀₋₃-Alkandiyl-Heteroaryl), wobei die Heteroarylgruppe fünf- oder sechsgliedrig ist und ein oder zwei Heteroatome enthält, ausgewählt aus der Gruppe, umfassend N, S und O,
wobei alle zuvor genannten Alkyl- und Cycloalkylreste mit bis zu zwei Resten, ausgewählt aus der Gruppe, umfassend CF₃, C₂F₅, OH, O-C₁₋₃-Alkyl, NH₂, NH-C₁₋₃-Alkyl, NH-C₁₋₃-Alkanoyl, N(C₁₋₃-Alkyl)₂, N(C₁₋₃-Alkyl)(C₁₋₃-Alkanoyl), COOH, CONH₂ und COO-C₁₋₃-Alkyl, und alle zuvor genannten Aryl- und Heteroarylgruppen mit bis zu zwei Resten, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, substituiert sein können und/oder auch eine anellierte Methandiylbisoxy- oder Ethan-1,2-diylbisoxygruppe tragen können, oder ***R***^{***5***} und ***R***^{***5'***} gemeinsam mit dem Amid-**N**-Atom von **B** einen fünf- bis siebengliedrigen, gesättigten oder ungesättigten heterocyclischen Ring bilden, der ein weiteres **N**- oder O- oder S-Atom enthalten kann und der substituiert sein kann mit C₁₋₄-Alkyl, (C₀₋₂-Alkandiyl-C₁₋₄-Alkoxy), C₁₋₄-Alkoxycarbonyl, Aminocarbonyl oder Aryl,
- ***R***^{***6***}: ein Rest, ausgewählt aus der Gruppe, umfassend (C₀₋₃-Alkandiyl-Aryl) und (C₀₋₃-Alkandiyl-Heteroaryl), wobei die Heteroarylgruppe fünf- oder sechsgliedrig ist und ein oder zwei Heteroatome enthält, ausgewählt aus der Gruppe, umfassend **N**, S und O, und wobei die Aryl- und Heteroarylgruppen mit bis zu zwei Resten substituiert sein können, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, und/oder auch eine anellierte Methandiylbisoxy- oder Ethan-1,2-diylbisoxygruppe tragen können.

Bevorzugt sind solche Benzimidazolderivate, bei denen der Substituent ***B-A-Y*** an die 6-Position des Benzimidazols gebunden ist.

Bevorzugt sind ferner solche Benzimidazolderivate, bei denen ***Z*** die Bedeutung **N**H, N***R***^{***2'***}, S, SO oder SO₂ hat.

Die vorliegende Erfindung umfasst auch physiologisch verträgliche Salze sowie Ester der vorgenannten Verbindungen, insbesondere die Säuresalze der Stickstoffbasen der erfindungsgemäßen Benzimidazolderivate, ferner die Salze von Carbonsäuren der erfindungsgemäßen Derivate mit Basen sowie die Ester der Carbonsäuren der Derivate sowie von Carbonsäuren, die von Carbonsäure-Derivaten abgeleitet sind, etwa von Carbonsäureamiden.

Die erfindungsgemäßen Benzimidazolderivate können ein chirales Zentrum oder mehrere chirale Zentren aufweisen, so dass die Verbindungen in mehreren isomeren Formen auftreten können. Die Verbindungen der Formel t können auch als Tautomere, Stereoisomere oder geometrische Isomere vorliegen. Die Erfindung umfasst auch alle möglichen Isomeren, wie E- und Z-Isomere, S- und R-Enantiomere, Diastereomere, Razemate und Gemische derselben einschliesslich der tautomeren Verbindungen. Alle diese isomeren Verbindungen sind - auch wenn jeweils nicht ausdrücklich angegeben - Bestandteil der vorliegenden Erfindung. Die Isomerengemische können nach üblichen Methoden, wie beispielsweise Kristallisation, Chromatographie oder Salzbildung, in die Enantiomeren bzw. E/Z-Isomeren aufgetrennt werden.

Die in den erfindungsgemäßen Benzimidazol-Verbindungen enthaltenen Heteroarylgruppen sind aus fünf oder sechs Gerüstatomen aufgebaut und können ein oder zwei Heteroatome enthalten. Heteroatome sind Sauerstoff (O), Stickstoff (N) und Schwefel (S). Beispiele für Heteroarylgruppen sind Pyrrolyl, Thienyl, Furanyl, Imidazolyl, Thiazolyl, Isothiazolyl, Oxazolyl, Isoxazolyl, Pyrazolyl, Pyridyl, Pyrimidinyl, Pyrazinyl und Pyridazinyl. Wenn die Heteroarylgruppen Teil von ***R***^{***1***} oder ***R***^{***2***} sind, wird die Gruppe über ein C-Atom an das jeweilige **N**-Atom des Benzimidazolgerüsts bzw. an den Substituenten ***Z*** gebunden.

Als Arylreste kommen vor allem der Phenylrest, aber auch der Naphthylrest in Frage. Die Aryl- und Heteroarylreste können in beliebiger Weise an das Benzimidazol-Grundgerüst oder eine andere Gruppe gebunden sein, beispielsweise als 1- oder 2-Naphthyl, 2-, 3- oder 4-Pyridinyl, 2-Thienyl, 3-Thienyl, 3-Furyl oder 2-Pyridiminyl.

Alkylgruppen können geradkettig oder verzweigt sein. Beispiele für Alkylgruppen sind Methyl, Ethyl, *n*-Propyl, *iso*-Propyl, *n*-Butyl, *sek*-Butyl, *tert*-Butyl, *n*-Pentyl, *sek*-Pentyl, *tert*-Pentyl, *neo*-Pentyl, *n*-Hexyl, *sek*-Hexyl, Heptyl, Octyl, Nonyl, Decyl. Auch die höheren Homologen umfassen jeweils sowohl die linearen als auch die verzweigten Alkylgruppen, also beispielsweise 2-Ethylhexyl für Octyl und 3-Propyl-hexyl für Nonyl.

Perfluorierte Alkyle sind vorzugsweise CF₃ und C₂F₅.

Alkenylgruppen können geradkettig oder verzweigt sein. Beispielsweise sind Vinyl, 2-Propenyl, 1-Propenyl, 2-Butenyl, 1-Butenyl, 1-Methyl-1-propenyl, 2-Methyl-2-propenyl und 3-Methyl-2-propenyl Alkenylreste im erfindungsgemäßen Sinne.

Alkinylgruppen können geradkettig oder verzweigt sein. Beispiele hierfür sind Ethinyl, 1-Propinyl, 2-Propinyl, 1-Butinyl und 2-Butinyl.

Unter Cycloalkylgruppen sind jeweils vorzugsweise Cyclopropyl, Cyclobutyl, Cyclopentyl, Cyclohexyl oder Cycloheptyl zu verstehen (entspricht C₃₋₇-Cycloalkyl).

Als gesättigter heterocyclischer Ring bzw. als Cycloalkyl mit einem oder mehreren Heteroatomen werden beispielsweise genannt: Piperidin, Pyrrolidin, Tetrahydrofuran, Morpholin, Piperazin, Hexahydroazepin sowie 2,6-Dimethylmorpholin, N-Phenyl-piperazin, Methoxymethyl-pyrrolidin, wobei die Verknüpfung mit einem dem Ring benachbarten C-Atom über gegebenenfalls vorhandene Ring-**N**-Atome erfolgen kann.

In der Erfindungsbeschreibung genannte Alkandiyl-, Alkendiyl-, Alkindiyl- und Cyloalkandiyl-Reste sind gleichbedeutend mit Alkylen, Alkenylen, Alkinylen und Cycloalkylen. Soweit in den allgemeinen Formeln der Alkandiylreste die Anzahl der enthaltenen C-Atome angegeben ist und als untere Bereichsgrenze dieser Anzahl der Wert 0 angegeben ist, ist dieser Alkandiylrest im jeweiligen Falle nicht enthalten.

Als Alkane, Alkene und Alkine für ***A*** werden beispielsweise genannt: geradkettiges oder verzweiges Alkandiyl mit einem bis acht C-Atomen, beispielsweise Methandiyl, Ethandiyl, Propandiyl, Butandiyl, Pentandiyl, Hexandiyl, ferner 1-Methylethandiyl, 1-Ethylethandiyl, 1-Methylpropandiyl, 2-Methylpropandiyl, 1-Methylbutandiyl, 2-Methylbutandiyl, 1-Ethylbutandiyl, 2-Ethylbutandiyl, 1-Methylpentandiyl, 2-Methylpentandiyl, 3-Methylpentandiyl sowie analoge Verbindungen.

Geradkettiges oder verzweigtes Alkendiyl und Alkindiyl mit zwei bis acht C-Atomen sind Alkendiylgruppen bzw. Alkindiylgruppen mit Doppel- und Dreifachbindungen in allen möglichen Positionen sowie mit allen möglichen Methyl- oder Ethylsubstitutionen. In diesen Resten können jeweils ein oder zwei C-Atome gegen O, **N**H, N-C₁₋₃-Alkyl oder **N**-C₁₋₃-Alkanoyl ausgetauscht sein, wobei die ausgetauschte Gruppe durch mindestens zwei C-Atome von ***Y*** getrennt ist.

Wenn zwei Reste orthoständig stehen, können sie mit dem benachbarten Aromaten einen gemeinsamen Ring bilden. Verbindungen, in denen **N**-, O- oder S-Atome an olefinische oder acetylenische Mehrfachbindungen gebunden sind, oder in denen mehrere **N**-, O-, S- oder Halogenatome an das gleiche aliphatische C-Atom gebunden sind, oder in denen **N**-, O- oder S-Atome unmittelbar aneinander gebunden sind, sind ausgenommen, sofern diese Verknüpfungen nicht explizit, etwa in den im Anspruch genannten funktionellen Gruppen oder in Heteroaromaten definiert sind.

Die physiologisch verträglichen Säuresalze der Stickstoffbasen der erfindungsgemäßen Benzimidazolderivate können mit anorganischen und organischen Säuren gebildet werden, beispielsweise mit Oxalsäure, Milchsäure, Citronensäure, Fumarsäure, Essigsäure, Maleinsäure, Weinsäure, Phosphorsäure, Salzsäure, Bromwasserstoffsäure, Schwefelsäure, *p*-Toluolsulfonsäure und Methansulfonsäure.

Zur Salzbildung von Säuregruppen, insbesondere Carbonsäuregruppen, sind auch die anorganischen oder organischen Basen geeignet, die zur Bildung physiologisch verträglicher Salze bekannt sind, wie beispielsweise Alkalihydroxide, insbesondere **N**atrium- und Kaliumhydroxid, Erdalkalihydroxide, wie Calciumhydroxid, ferner Ammoniak, sowie Amine, wie Ethanolamin, Diethanolamin, Triethanolamin, **N**-Methylglucamin und Tris-(hydroxymethyl)-methylamin.

Zur Esterbildung sind alle niederen einwertigen, zweiwertigen und dreiwertigen Alkohole geeignet, insbesondere Methanol, Ethanol, *iso*-Propanol und *tert*-Butanol sowie Ethylenglykol und Glycerin.

Bevorzugt sind Benzimidazole mit der allgemeinen Formel I, in denen die nachfolgend angegebenen Reste und Gruppen unabhängig voneinander die folgenden Bedeutungen haben:
- ***R***^{***1***}: eine Phenylgruppe, die mit bis zu zwei Resten unabhängig voneinander substituiert sein kann, ausgewählt aus der Gruppe, umfassend:
F, Cl, Br,
C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4'***},
C(N***R***^{***4***})N***R***^{***4***}***R***^{***4'***},
OH, O***R***^{***4***}, OCO***R***^{***4***}, OCONH***R***^{***4***},
CO***R***^{***4***}, C(NOH)***R***^{***4***},
CN, COOH, COO***R***^{***4***}, CONH₂, CON***R***^{***4***}***R***^{***4'***}, CONH***R***^{***4***}, CONHOH,
S***R***^{***4***}, SO***R***^{***4***}, SO₂***R***^{***4***},
SO₂NH₂, SO₂NH***R***^{***4***},SO₂N***R***^{***4***}***R***^{***4'***},
NO₂, NH₂, NH***R***^{***4***}, N***R***^{***4***}***R***^{***4'***}, NHCONH***R***^{***4***} und
***R***^{***4***},
wobei die Reste ***R***^{***4***} und ***R***^{***4'***} gemäß nachstehend angegebenen Bedeutungen unabhängig voneinander gewählt werden und wobei zwei Substituenten an ***R***^{***1***} so miteinander verknüpft sein können, dass sie gemeinsam eine Methandiylbisoxy-, Ethan-1,2-diylbisoxy-, Propan-1,3-diyl- oder Butan-1,4-diylgruppe bilden, wenn sie zueinander orthoständig sind,
- ***Z***: dasselbe wie weiter oben angegeben,
- ***R***^{***2***} und **R**^{**2'**}: dasselbe wie weiter oben angegeben,
- ***R***^{***3***}: ein Rest, ausgewählt aus der Gruppe, umfassend Wasserstoff, F, Cl, Br, CH₃, C₂H₅, CF₃, C₂F₅, OH, O***R***^{***4***}, NHSO₂***R***^{***6***} und NHCO***R***^{***4***}, wobei ***R***^{***4***} und ***R***^{***6***} die weiter unten angegebenen Bedeutungen haben,
- ***A***: dasselbe wie weiter oben angegeben,
- ***B***: ein Rest, ausgewählt aus der Gruppe, umfassend COOH, COO***R***^{***5***}, CONH₂, CONH***R***^{***5***} und CON***R***^{***5***}***R***^{***5'***}, jeweils gebunden an ein C-Atom der Gruppe ***A***,
wobei die Reste ***R***^{***5***} und ***R***^{***5'***} gemäß den weiter unten angegebenen Bedeutungen unabhängig voneinander gewählt werden,
- ***Y***: O
worin in den vorstehenden Resten die Reste ***R***^{***4***}***, R***^{***4'***}***, R***^{***5***}***, R***^{***5'***} und ***R***^{***6***} die folgenden Bedeutungen haben; darin bedeuten:
- ***R***^{***4***} und ***R***^{***4'***}: dasselbe wie weiter oben angegeben,
- ***R***^{***5***} und ***R***^{***5'***}: unabhängig voneinander jeweils ein Rest, ausgewählt aus der Gruppe, umfassend C₁₋₆-Alkyl, C₂₋₆-Alkenyl, C₂₋₆-Alkinyl, wobei ein C-Atom gegen O, S, SO, SO₂, NH, N-C₁₋₃-Alkyl oder N-C₁₋₃-Alkanoyl ausgetauscht sein kann, ferner (C₀₋₃-Alkandiyl-C₃₋₇-Cycloalkyl),
wobei in einem fünfgliedrigen Cycloalkylring ein Ringglied Ring-**N** oder Ring-O sein kann und in einem sechs- oder siebengliedrigen Cycloalkylring ein oder zwei Ringglieder jeweils Ring-**N**- und/oder Ring-O-Atome sein können, wobei die Ring-**N**-Atome gegebenenfalls mit C₁₋₃-Alkyl oder C₁₋₃-Alkanoyl substituiert sein können, sowie ferner (C₀₋₃-Alkandiyl-Phenyl) und
(C₀₋₃-Alkandiyl-Heteroaryl), wobei die Heteroarylgruppe fünf- oder sechsgliedrig ist und ein oder zwei Heteroatome enthält, ausgewählt aus der Gruppe, umfassend N, S und O,
wobei alle zuvor genannten Alkyl- und Cycloalkylreste mit einem Rest, ausgewählt aus der Gruppe, umfassend CF₃, C₂F₅, OH, O-C₁₋₃-Alkyl, NH₂, NH-C₁₋₃-Alkyl, NH-C₁₋₃-Alkanoyl, N(C₁₋₃-Alkyl)₂, N(C₁₋₃-Alkyl)(C₁₋₃-Alkanoyl), COOH, CONH₂ und COO-C₁₋₃-Alkyl, und alle zuvor genannten Phenyl- und Heteroarylgruppen mit bis zu zwei Resten, ausgewählt aus der Gruppe, umfassend F, Cl, Br, CH₃, C₂H₅, OH, OCH₃, OC₂H₅, **N**O₂, **N**(C**H**₃)₂, CF₃, C₂F₅ und SO₂**NH**₂, substituiert sein können und/oder auch eine anellierte Methandiylbisoxy- oder Ethan-1,2-diylbisoxygruppe tragen können, oder ***R***^{***5***} und ***R***^{***5'***} gemeinsam mit dem Amid-N-Atom von ***B*** einen fünf- bis siebengliedrigen, gesättigten oder ungesättigten heterocyclischen Ring bilden, der ein weiteres **N**- oder O- oder S-Atom enthalten kann und der substituiert sein kann mit C₁₋₄-Alkyl, (C₀₋₂-Alkandiyl-C₁₋₄-Alkoxy), C₁₋₄-Alkoxycarbonyl, Aminocarbonyl oder Phenyl,
- ***R***^{***6***}: eine Phenyl- oder Heteroarylguppe, wobei die Heteroarylgruppe fünf- oder sechsgliedrig ist und ein oder zwei Heteroatome enthält, ausgewählt aus der Gruppe, umfassend N, S und O, und wobei die Phenyl- und Heteroarylgruppen mit bis zu zwei Resten substituiert sein können, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, oder auch eine anellierte Methandiylbisoxy- oder Ethan-1,2-diylbisoxygruppe tragen können.

Insbesondere bevorzugt sind Benzimidazole mit der allgemeinen Formel I, in denen die nachfolgend angegebenen Reste und Gruppen unabhängig voneinander die folgenden Bedeutungen haben:
- ***R***^{***1***}: eine Phenylgruppe, die mit bis zu zwei Resten unabhängig voneinander substituiert sein kann, ausgewählt aus der Gruppe, umfassend:
F, Cl, Br,
C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4'***},
C(N***R***^{***4***})N***R***^{***4***}***R***^{***4'***},
OH, O***R***^{***4***}, OCO***R***^{***4***}, OCONH***R***^{***4***},
CO***R***^{***4***}, C(NOH)***R***^{***4***},
CN, COOH, COO***R***^{***4***}, CONH₂, CON***R***^{***4***}***R***^{***4'***}, CONH***R***^{***4***}, CONHOH,
S***R***^{***4***}, SO***R***^{***4***}, SO₂***R***^{***4***},
SO₂NH₂, SO₂NH***R***^{***4***},SO₂N***R***^{***4***}***R***^{***4'***},
NO₂, NH₂, NH***R***^{***4***}, N***R***^{***4***}***R***^{***4'***}, NHCONH***R***^{***4***} und
***R***^{***4***},
wobei die Reste ***R***^{***4***} und ***R***^{***4'***} gemäß nachstehend angegebenen Bedeutungen unabhängig voneinander gewählt werden und
wobei zwei Substituenten an ***R***^{***1***} so miteinander verknüpft sein können, dass sie gemeinsam eine Methandiylbisoxy-, Ethan-1,2-diylbisoxy-, Propan-1,3-diyl- oder Butan-1,4-diylgruppe bilden, wenn sie zueinander orthoständig sind,
- ***Z***: dasselbe wie weiter oben angegeben,
- ***R***^{***2***} und ***R***^{***2'***}: unabhängig voneinander jeweils ein Rest, ausgewählt aus der Gruppe, umfassend:
C₁₋₄-Perfluoralkyl, C₁₋₆-Alkyl, (C₀₋₃-Alkandiyl-Aryl) und (C₀₋₃-Alkandiyl-Heteroaryl),
wobei die Heteroarylgruppe fünf- oder sechsgliedrig ist und ein oder zwei Heteroatome enthält, ausgewählt aus der Gruppe, umfassend N, S und O, und
wobei die Aryl- und Heteroarylgruppe jeweils mit bis zu zwei Resten, ausgewählt aus der Gruppe, umfassend F, Cl, Br, CH₃, C₂H₅, OH, OCH₃, OC₂H₅, NO₂, CF₃, C₂F₅ und SO₂NH₂, substituiert sein können
oder wenn ***Z* N*R***^{***2'***} ist, ***R***^{***2***} und ***R***^{***2'***} gemeinsam mit ***Z*** einen fünf- bis siebengliedrigen heterocyclischen Ring bilden, wobei ferner der heterocyclische Ring ein zusätzliches O- oder S-Atom enthalten und optional substituiert sein kann mit einem Rest, ausgewählt aus der Gruppe, umfassend C₁₋₄-Alkyl, (C₀₋₃-Alkandiyl-C₁₋₃-Alkoxy), C₁₋₄-Alkanoyl, C₁₋₄-Alkoxycarbonyl, Aminocarbonyl und Aryl,
- ***R***^{***3***}: Wasserstoff,
- ***A***: geradkettiges oder verzweigtes Alkandiyl mit bis zu 8 C-Atomen,
- ***B***: ein Rest, ausgewählt aus der Gruppe, umfassend COOH, COO***R***^{***5***}, CONH₂, CONH***R***^{***5***} und CON***R***^{***5***}***R***^{***5'***}, jeweils gebunden an ein C-Atom der Gruppe ***A***,
wobei die Reste ***R***^{***5***} und ***R***^{***5'***} gemäß den weiter unten angegebenen Bedeutungen unabhängig voneinander gewählt werden,
- ***Y***: O
worin in den vorstehenden Resten die Reste ***R***^{***4***}, ***R***^{***4'***}, ***R***^{***5***} und ***R***^{***5'***} die folgenden Bedeutungen haben; darin bedeuten:
- ***R***^{***4***} und ***R***^{***4'***}: unabhängig voneinander jeweils ein Rest, ausgewählt aus der Gruppe, umfassend CF₃, C₂F₅, C₁₋₄-Alkyl, C₂₋₄-Alkenyl, C₂₋₃-Alkinyl und (C₀₋₃-Alkandiyl-C₃₋₇-Cycloalkyl),
wobei Alkylreste optional mit einem Rest, ausgewählt aus der Gruppe, umfassend OH, OCH₃ und SCH₃, substituiert sein können,
- ***R***^{***5***} und ***R***^{***5'***}: unabhängig voneinander jeweils ein Rest, ausgewählt aus der Gruppe, umfassend C₁₋₆-Alkyl, C₂₋₆-Alkenyl, C₂₋₆-Alkinyl, (C₀₋₃-Alkandiyl-C₃₋₇-Cycloalkyl), (C₀₋₃-Alkandiyl-Phenyl) und (C₀₋₃-Alkandiyl-Heteroaryl), wobei die Heteroarylgruppe fünf- oder sechsgliedrig ist und ein oder zwei Heteroatome enthält, ausgewählt aus der Gruppe, umfassend N, S und 0,
wobei alle zuvor genannten Alkyl- und Cycloalkylreste mit einem Rest, ausgewählt aus der Gruppe, umfassend CF₃, C₂F₅, OH, O-C₁₋₃-Alkyl, NH₂, NH-C₁₋₃-Alkyl, NH-C₁₋₃-Alkanoyl, **N**(C₁₋₃-Alkyl)₂, **N**(C₁₋₃-Alkyl)(C₁₋₃-Alkanoyl), COOH, CONH₂ und COO-C₁₋₃-Alkyl, und alle zuvor genannten Phenyl- und Heteroarylgruppen mit bis zu zwei Resten, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, substituiert sein können und/oder auch eine anellierte Methandiylbisoxy- oder Ethan-1,2-diylbisoxygruppe tragen können, oder ***R***^{***5***} und ***R***^{***5'***} gemeinsam mit dem Amid-**N**-Atom von ***B*** einen fünf- bis siebengliedrigen, gesättigten oder ungesättigten heterocyclischen Ring bilden, der ein weiteres N- oder O- oder S-Atom enthalten kann und der substituiert sein kann mit C₁₋₄-Alkyl, (C₀₋₂-Alkandiyl-C₁₋₄-Alkoxy), C₁₋₄-Alkoxycarbonyl, Aminocarbonyl oder Phenyl.

***R***^{***1***} ist insbesondere Phenyl oder Methylphenyl. ***R***^{***2***} kann bevorzugt ein Rest sein, ausgewählt aus der Gruppe, umfassend C₁₋₃-Alkyl, Phenyl, Methylphenyl, Methandiylphenyl und Heteroaryl. ***Z*** kann zusammen mit ***R***^{***2***} auch einen gesättigten 5- oder 6-gliedrigen heterocyclischen Ring bilden, der ein zusätzliches O-oder S-Atom enthalten kann, beispielsweise einen Piperidin- oder Morpholinring.

***R***^{***3***} ist bevorzugt Wasserstoff. Die Gruppierung ***Y-A*** wird in einer bevorzugten Ausführungsform durch eine *n*-Pentandiyloxy-Gruppe (-C₅H₁₀-O-) wiedergegeben, die über das O-Atom an das Benzimidazolgerüst gebunden ist. Alternativ können auch Gruppierungen mit kürzeren oder längeren Alkandiyl-Resten eingesetzt werden, beispielsweise *n*-Butandiyloxy oder *n*-Hexandiyloxy.

Die Endgruppe ***B*** steht vorzugsweise für COOH, COO***R***, wobei ***R*** insbesondere C₁₋₄-Alkyl wie Methyl, Ethyl, *iso*-Propyl oder *tert*-Butyl sein kann, oder eine Amidgruppe, beispielsweise eine C₁₋₆-Alkylamidogruppe, wobei Alkyl insbesondere *iso*-Propyl oder *iso*-Butyl sein kann, eine Dialkylamidogruppe, wobei Alkyl insbesondere Methyl sein kann, oder eine 3-Alkoxy-propandiylaminogruppe, wobei Alkoxy eine Methyloxy, *iso*-Butyloxy oder *iso*-Pentyloxygruppe sein kann.

Die die einzelnen Derivate unterscheidenden Strukturmerkmale sind in Tabelle 2 wiedergegeben, wobei sich die angegebenen Strukturmerkmale auf die in der Tabelle ebenfalls angegebene allgemeine Formel II beziehen.

Die erfindungsgemäßen Benzimidazolderivate hemmen die Aktivierung der Mikroglia. Unter Mikroglia werden hier die Makrophagen des Gehirns verstanden. Daher betrifft die Erfindung auch die Verwendung dieser Derivate zur Herstellung von Arzneimitteln zur Behandlung von mit einer Mikroglia-Aktivierung assoziierten Erkrankungen sowie zur Prophylaxe gegen die Erkrankungen. Dabei ist auch eine entsprechende Verwendung von solchen Derivaten mit der allgemeinen Formel I eingeschlossen, bei denen ***B*** für Wasserstoff steht und worin die Reste ***R***^{***1***}***, R***^{***2***}***, R***^{***3***}***, A, B, Y*** und ***Z*** die oben angegebenen Bedeutungen haben. Die diese Reste bzw. Gruppen ebenfalls definierenden weiteren Reste ***R***^{***4***}***, R***^{***5***} und ***R***^{***6***} sind ebenfalls durch die weiter oben angegebenen Bedeutungen definiert. Zusätzlich zur Verwendung der erfindungsgemäßen neuen Benzimidazolderivate zur Herstellung der genannten Arzneimittel betrifft die Erfindung auch eine entsprechende Verwendung von Benzimidazolderivaten, in denen ***B*** für Wasserstoff steht. Die Verwendung dieser Derivate, die als Bestandteil von Arzneimitteln zur Behandlung von mit einer Mikroglia-Aktivierung assoziierten Erkrankungen sowie zur Prophylaxe gegen diese Erkrankungen eingesetzt werden, ist neu, auch wenn diese weiteren Verbindungen selbst nicht neu sind (US-A-5,552,426).

Die Verbindungen der Formel **I** inhibieren die Aktivierung der Mikroglia und die Produktion von Interleukin 12 (IL 12) und Interferon γ (IFNγ). Die Erfindung betrifft daher auch die Verwendung einer Verbindung der Formel **I**, sowie deren optischer oder geometrischer Isomere oder deren Tautomere oder eines physiologisch verträglichen Salzes zur Herstellung eines Arzneimittels zur Behandlung oder Prävention einer mit einer Mikroglia-Aktivierung assoziierten Erkrankung sowie einer durch Überproduktion von IL 12 und IFNγ ausgelösten Erkrankung und zur Induktion von Interleukin 10 (IL-10).

Auf Grund ihrer Fähigkeit, die Aktivierung der Mikroglia zu inhibieren und die Produktion von IL 12 und TNFα in Monozyten/Makrophagen und die INFγ Produktion in T-Zellen und NK-Zellen zu unterbrechen und die Induktion der IL-10-Produktion zu erhöhen, sind die erfindungsgemäßen Verbindungen zur Behandlung zahlreicher Erkrankungen geeignet, die durch die verstärkte Produktion von Cytokinen wie z.B. TNFα,β, IFNγ, IL 2 und IL 12 ausgelöst werden, wie inflammatorische Erkrankungen, Autoimmun-Erkrankungen, allergische und infektiöse Erkrankungen, Toxin-induzierte Entzündungen, pharmakologisch ausgelöste Entzündungsreaktionen sowie pathophysiologisch relevante Entzündungsreaktionen derzeit unklarer Genese.

Beispiele für inflammatorische und autoimmune Erkrankungen sind: chronische inflammatorische Darm-Erkrankungen (Inflammatory Bowel Diseases, Crohn's Disease, Ulcerative Colitis), Arthritis, allergisches Kontaktekzem, Psoriasis, Pemphigus, Asthma, Multiple Sklerose, Diabetes, Typ-I Insulin-abhängiger Diabetes Mellitus, Rheumatoide Arthritis, Lupus-Erkrankungen und andere Kollagenosen, Graves' Disease, Hashimoto's Disease, "Graft-versus-host-Disease" und Transplantatabstoßung.

Beispiele für allergische, infektiöse und Toxin-ausgelöste und Ischämieausgelöste Erkrankungen sind: Sarkoidose, Asthma, hypersensitive Pneumonitis, Sepsis, septischer Schock, Endotoxin-Schock, Toxisches Schocksyndrom, Toxisches Leberversagen, ARDS (Akutes Atemnot-Syndrom), Eklampsie, Kachexie, akute Virusinfektionen (z.B. Mononukleose, fulminante Hepatitis), Organschädigung nach Reperfusion.

Ein Beispiel für eine pharmakologisch ausgelöste Entzündung mit pathophysiologischer Relevanz ist die "first dose response" nach Gabe von Anti-T-Zellantikörpern wie OKT3.

Ein Beispiel für systemische Entzündungsreaktionen derzeit unklarer Genese ist die Eklampsie.

Beispiele für neuroinflammatorische Erkrankungen, die mit einer Mikroglia-Aktivierung assoziiert sind, sind AIDS-Dementia, Amyotrophe Lateralsklerose, Creutzfeldt-Jakob-Krankheit, Down's Syndrome, diffuse Lewy Body Krankheit, Huntington's Krankheit, Leukenzephalopathic Multiple Sklerose, Parkinsonsche Krankheit, Picksche Krankheit, Alzheimersche Krankheit, Schlaganfall, temporäre Lobe-Epilepsie und Tumore. Daher betrifft die Erfindung auch die Verwendung der angegebenen Benzimidazolderivate zur Behandlung dieser Erkrankungen sowie zur Prophylaxe gegen diese Erkrankungen.

Die Wirkung der erfindungsgemäßen Benzimidazolderivate bei der Behandlung und Prophylaxe von mit Mikroglia assoziierten Erkrankungen ist überraschend, da bisher Benzimidazolderivate nur für die Behandlung von Thrombosen und Artheriosklerose [EP 0 531 883 A1, EP 0104 727 A1, WO 97/12613 A1], Zystitis [WO 97/33873 A1] und Erkrankungen, die mit einem β-Amyloid-Peptid [US-A-5,552,426] sowie einer verstärkten Aktivierung von Ca-Kanälen [EP 0 520 200 A2] assoziiert sind, beschrieben worden sind, aber ein Effekt auf Mikroglia nicht bekannt ist.

In Beispiel 45 ist beschrieben, wie die Hemmung der Mikroglia-Aktivierung gemessen werden kann. Die Mikroglia können dabei durch verschiedene Stimuli aktiviert werden, wie beispielsweise mit Aβ-Peptid [β-Amyloid, Araujo, D.M. and Cotman, C.M., *Brain Res*., 569, 141-145 (1992)], mit Prion-Protein, Zytokinen oder durch Zellfragmente [Combs, C.K et al., *J. Neurosci*., 19, 928-939, (1999), Wood, P.L., Neuroinflammation: Mechanisms and Management , *Humana Press,* (1998)].

Die Stimulierung mit dem Aβ-Peptid entspricht der pathophysiologischen Situation bei der Alzheimerschen Krankheit. In diesem Test zeigten die erfindungsgemäßen Substanzen bei Stimulierung mit dem Aβ-Peptid eine Hemmung der Mikroglia-Aktivierung. Die Hemmung der Mikroglia-Aktivierung durch die erfindungsgemäßen Substanzen führt zu einer starken Reduktion der Cytokinproduktion und -sekretion, beispielsweise von II1β und TNFα (gemessen durch ELISA und mRNA-Expressionsanalyse), und zu einer verminderten Sekretion von reaktiven Sauerstoff/Stickstoff-Intermediaten. Es werden also gleich mehrere Entzündungsfaktoren gehemmt.

Die *in vivo*-Wirksamkeit der erfindungsgemäßen Substanzen wird in einem MCAO-Modell in Ratten gezeigt. Dieses Modell simuliert den Zustand eines Schlaganfalls. Die erfindungsgemäßen Substanzen reduzieren die Mikroglia-Aktivierung, die bei akuten Hirnläsionen in den Gehirnen der Tiere auftritt.

Die inhibition der Zytokin-Produktion wird beispielsweise durch Messung von TNFα und Interleukin 12 in Lipopolysaccharid (LPS)-stimulierten THP-1-Zellen dargestellt.

Die erfindungsgemäßen Verbindungen inhibieren die TNFα- und Interleukin-12 Produktion in Lipopolysaccharid (LPS)-stimulierten THP-1-Zellen. Zur Darstellung des Einflusses der Substanzen auf die T-Zell-Aktivierung wird beispielsweise die Messung der INFγ Sekretion eingesetzt. Die erfindungsgemäßen Verbindungen inhibieren die INFγ Produktion von peripheren mononukleären Blutzellen.

Ferner betrifft die Erfindung pharmazeutische Mittel, die eine oder mehrere erfindungsgemäße Verbindungen der allgemeinen Formel I sowie einen oder mehrere Trägerstoffe enthalten. Die pharmazeutischen Mittel bzw. Zusammensetzungen der Erfindung werden mit üblichen festen oder flüssigen Trägerstoffen oder Verdünnungsmitteln und üblichen pharmazeutischen und technischen Hilfsstoffen entsprechend der gewünschten Applikationsart mit einer geeigneten Dosierung in an sich bekannter Weise hergestellt. Bevorzugte Zubereitungen bestehen in einer Darreichungsform, die zur oralen, enteralen oder parenteralen, beispielsweise *i.p*. (intraperitonealen), *i.v.* (intravenösen), *i.m*. (intramuskulären), oder perkutanen Applikation geeignet ist. Solche Darreichungsformen sind beispielsweise Tabletten, Filmtabletten, Dragees, Pillen, Kapseln, Pulver, Cremes, Salben, Lotionen, Flüssigkeiten, wie Sirupe, Gele, injiierbare Flüssigkeiten, beispielsweise zur *i.p., i.v., i.m*., oder perkutanen Injektion usw. Weiterhin sind auch Depotformen, wie implantierbare Zubereitungen, sowie Suppositorien geeignet. Dabei geben die einzelnen Zubereitungen die erfindungsgemäßen Derivate je nach deren Art allmählich oder die gesamte Menge in kurzer Zeit an den Körper ab.

Zur oralen Verabreichung können Kapseln, Pillen, Tabletten, Dragees und Flüssigkeiten oder andere bekannte orale Darreichungsformen als pharmazeutische Präparate eingesetzt werden. In diesem Falle können die Arzneimittel in der Weise formuliert sein, dass sie die Wirkstoffe entweder in kurzer Zeit freisetzen und an den Körper abgeben oder eine Depotwirkung aufweisen, so dass eine länger anhaltende, langsame Zufuhr von Wirkstoff zum Körper erreicht wird. Die Dosierungseinheiten können neben dem mindestens einen Benzimidazolderivat einen oder mehrere pharmazeutisch verträgliche Träger enthalten, beispielsweise Stoffe zur Einstellung der Rheologie des Arzneimittels, oberflächenaktive Stoffe, Lösungsvermittler, Mikrokapseln, Mikropartikel, Granulate, Verdünner, Bindemittel, wie Stärke, Zucker, Sorbit und Gelatine, ferner Füllstoffe, wie Kieselsäure und Talkum, Gleitmittel, Farbstoffe, Duftstoffe und andere Stoffe.

Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog zu den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Die erfindungsgemäßen Benzimidazolderivate können auch in Form einer Lösung formuliert werden, die für die orale Verabreichung bestimmt ist und die neben dem aktiven Benzimidazolderivat als Bestandteile ein pharmazeutisch verträgliches Öl und/oder eine pharmazeutisch verträgliche lipophile, oberflächenaktive Substanz und/oder eine pharmazeutisch verträgliche hydrophile, oberflächenaktive Substanz und/oder ein pharmazeutisch verträgliches wassermischbares Lösungsmittel enthält.

Um eine bessere Bioverfügbarkeit der erfindungsgemäßen Wirkstoffe zu erreichen, können die Verbindungen auch als Cyclodextrinchlatrate formuliert werden. Hierzu werden die Verbindungen mit α-, β- oder γ-Cyclodextrin oder deren Derivaten umgesetzt.

Falls Cremes, Salben, Lotionen und äußerlich anwendbare Flüssigkeiten eingesetzt werden sollen, müssen diese so beschaffen sein, dass die erfindungsgemäßen Verbindungen dem Körper in ausreichender Menge zugeführt werden. In diesen Darreichungsformen sind Hilfsstoffe enthalten, beispielsweise Stoffe zur Einstellung der Rheologie der Arzneimittel, oberflächenaktive Mittel, Konservierungsmittel, Lösungsvermittler, Verdünner, Stoffe zur Erhöhung der Permeationsfähigkeit für die erfindungsgemäßen Benzimidazolderivate durch die Haut, Farbstoffe, Duftstoffe und Hautschutzmittel, wie Konditionierer und Feuchteregulatoren. Zusammen mit den erfindungsgemäßen Verbindungen können auch andere Wirkstoffe in dem Arzneimittel enthalten sein [*Ullmanns Enzyklopädie der technischen Chemie,* Band 4 (1953), Seiten 1 - 39; *J. Pharm. Sci.,* 52, 918 ff. (1963); H. v.Czetsch-Lindenwald, *Hilfsstoffe für Pharmazie und angrenzende Gebiete; Pharm. lnd., 2,* 72 ff (1961); Dr. H. P. Fiedler, *Lexikon der Hilfsstoffe für Pharmazie, Kosmetik und angrenzende Gebiete,* Cantor AG, Aulendorf/Württ., 1971].

Die erfindungsgemäßen Substanzen können auch in geeigneten Lösungen wie beispielsweise physiologischer Kochsalzlösung, als Infusions- oder Injektionslösung zur Anwendung kommen. Für die parenterale Applikation können die Wirkstoffe in einem physiologisch verträglichen Verdünnungsmittel gelöst oder suspendiert sein. Als Verdünnungsmittel sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Zur Formulierung eines injizierbaren Präparats kann ein beliebiger flüssiger Träger verwendet werden, in dem die erfindungsgemäßen Verbindungen gelöst oder emulgiert sind. Diese Flüssigkeiten enthalten häufig auch Stoffe zur Regulation der Viskosität, oberflächenaktive Stoffe, Konservierungsstoffe, Lösungsvermittler, Verdünner und weitere Zusatzstoffe, mit denen die Lösung isotonisch eingestellt wird. Zusammen mit den Benzimidazolderivaten können auch andere Wirkstoffe verabreicht werden.

Es ist auch möglich, die erfindungsgemäßen Substanzen in ein transdermales System einzuarbeiten und sie damit transdermal zu applizieren. Hierzu werden die Benzimidazolderivate in Form einer Depotinjektion oder eines Implantatpräparats, beispielsweise subkutan, angewendet. Derartige Präparate können so formuliert sein, dass eine verzögerte Wirkstoff-Freigabe ermöglicht wird. Hierzu können bekannte Techniken eingesetzt werden, beispielsweise sich auflösende oder mit einer Membran arbeitende Depots. Implantate können als inerte Materialien beispielsweise biologisch abbaubare Polymere oder synthetische Silikone, beispielsweise Silikonkautschuk, enthalten. Die Benzimidazolderivate können ferner zur perkutanen Verabreichung beispielsweise in ein Pflaster eingearbeitet werden.

Die Dosierung der erfindungsgemäßen Substanzen der allgemeinen Formel **I** wird vom behandelnden Arzt bestimmt und hängt unter anderem von der verabreichten Substanz, dem Verabreichungsweg, der zu behandelnden Erkrankung und von der Schwere der Erkrankung ab. Die tägliche Dosis beträgt nicht mehr als 1000 mg, vorzugsweise nicht mehr als 100 mg, wobei die Dosis als einmal zu verabreichende Einzeldosis oder unterteilt in zwei oder mehrere Tagesdosen gegeben werden kann.

1-Aryl-2N-, -S- oder -O-substituierte Benzimidazole ***(G, H, K, L, N, P)*** *-* **s**. Schemata 2 bis 4 - sind auf unterschiedlichen Wegen nach an sich literaturbekannten Verfahren zugänglich.

Als mögliche Verfahren neben anderen seien die folgenden erwähnt:

Durch Reaktion von Arylaminen ***(B)*** mit ortho-abgangsgruppensubstituierten, bevorzugt halogensubstituierten, Nitrobenzolderivaten ***(A)*** können *N*-Aryl-2-nitrobenzole ***(C)*** unter diversen Reaktionsbedingungen erzeugt werden, wie zum Beispiel durch Erwärmen der Reaktanden ohne oder mit einem geeigneten inerten Lösungsmittel wie beispielsweise Alkyl- oder Halogenbenzolen. Auch kann das als Reaktand verwendete Amin im Überschuss als Lösungsmittel eingesetzt werden. Die Umsetzungen werden sowohl ohne als auch mit Basen, beispielsweise Kaliumcarbonat oder Natriumhydrid, durchgeführt. Auch finden weitere Hilfsstoffe wie beispielsweise Kupfersalze Verwendung. Beispiele für die hier angegebene Vorgehensweise finden sich in zahlreichen Arbeiten, wie etwa in: D. Jerchel, H. Fischer, M. Graft, *Ann. Chem*., 575, 162 (1952), CAS, 53 (2138); R-A. Abramovitch, *Can. J. Chem.,* 38, 2273 (1960). Die Nitrogruppe ***(C* → *D)*** wird bevorzugt durch Hydrierung in polaren Lösungsmitteln, wie Essigsäure, niederen Alkoholen oder Essigestern, unter Zugabe von Katalysatoren, wie Raney-Nickel oder Palladium auf Kohle, oder durch chemische Reduktion, beispielsweise mit Zinn in Salzsäure, SnCl₂ [F.D. Bellamy, *Tet. Lett*. (1984)] oder Fe/Essigsäure [D.C. Owslly, J.J. Bloomfield, *Synthesis,* 118, 150 (1977)] reduziert.

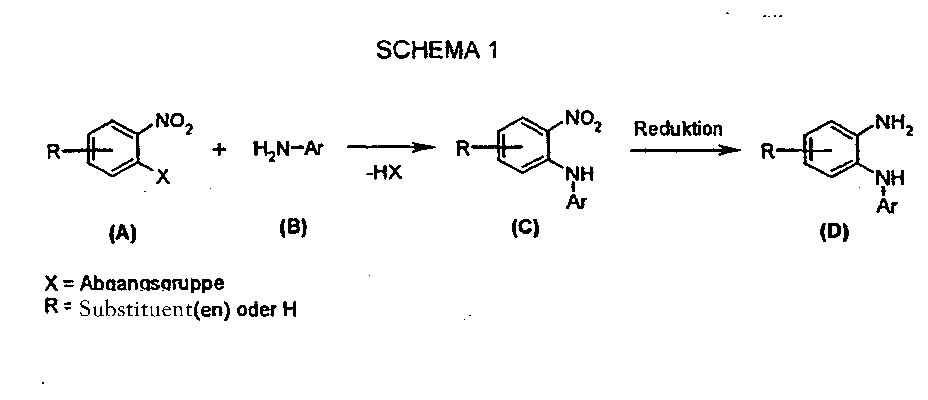

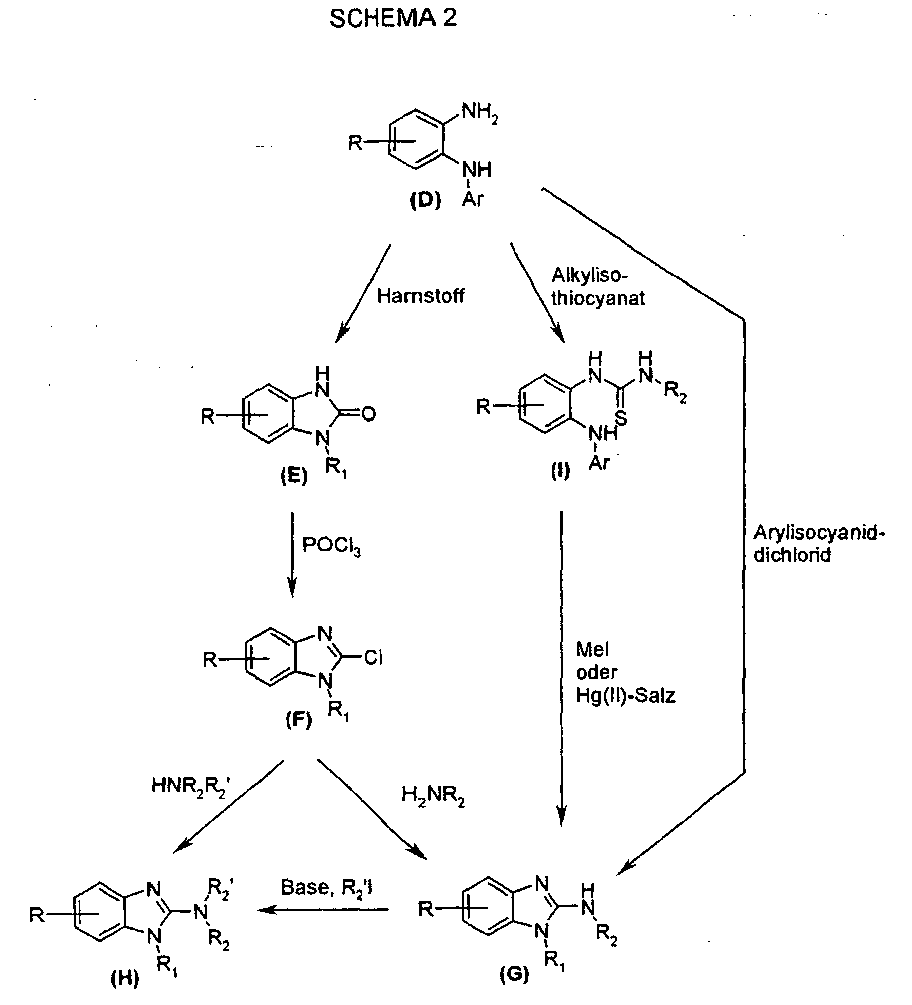

Die so erhaltenen Diaminobenzole vom Typ ***D*** lassen sich auf verschiedenen Wegen in 1-Aryl-2N-, -S- oder -O-substituierte Benzimidazole ***(G, H, K, L, N, P)*** überführen:

Durch Umsetzung der Diamine des Typs ***D*** mit Kohlensäurederivaten wie etwa Harnstoff [H. Goeker, G. Ayhan-Kilcigil, M. Tuncbilek, C. Kus, R. Ertan, E. Kendi, S. Oezbey, M. Fort, C. Garcia, A. Farre, *J. Heterocycles*, 1999, 2561] sind Benzimidazolone vom Typ ***E*** darstellbar, deren Behandlung mit Phosphoroxychlorid [M.J. Kukla, H.J. Breslin, C.J. Diamond, P.P. Grous, C.Y. Ho et al., *J. Med. Chem.,* 1991, 3187; J. Turner; *J. Chem. Soc.* 1950, 1515] 2-Chlorbenzimidazole des Typs ***F*** liefert. Umsetzung dieser Benzimidazole mit primären oder sekundären Aminen [M.J. Kukla, H.J. Breslin, C.J. Diamond, P.P. Grous, C.Y. Ho et al., *J. Med. Chem.,* 1991, 3187; Efros et al., *Zh. Obshch. Khim*., 1953, 1691; J. Turner, *J. Chem.* Soc., 1950, 1515; Z. Zhu, B. Lippa, J.C. Drach, L.B. Townsend, *J. Med. Chem.,* 2000, 2430; H. Goeker, G. Ayhan-Kilcigil, M. Tuncbilek, C. Kus, R. Ertan, E. Kendi, S. Oezbey, M. Fort, C. Garcia, A. Farre, *J. Heterocycles,* 1999, 2561; J. Musco, D.B. Murphy, *J. Org. Chem.,* 1971, 3469] führt zu 2-aminosubstituierten Benzimidazolen der Typen ***G*** und ***H.*** Die Benzimidazole des Typs ***G*** lassen sich auch durch basenvermittelte Alkylierung [K. Kubo, *Y.* Kohara, *E.* Imamiya, *Y.* Sugiura, *Y.* Inada et al., *J. Med. Chem.,* 1993, 2182] zu den Benzimidazolen ***H*** umsetzen. Benzimidazole des Typs ***G*** sind beispielsweise auch dadurch zugänglich, dass man Phenylendiamine vom Typ ***D*** mit Alkyl- oder Arylisothiocyanaten zu den Thioharnstoffderivaten ***I*** umsetzt, die bei nachfolgender Behandlung mit Methyljodid [M.J. Kukla, H.J. Breslin, C.J. Diamond, P.P. Grous, C.Y. Ho et al., *J. Med. Chem*., 1991, 3187] oder mit Quecksilber(II)-Salzen [F. Merchan, J. Garin, V. Martinez, E. Melendez, *Synthesis,* 1982, 482; K.C. Nicolaou, J.I. Trujillo, B. Jandeleit, K. Chibale, M. Rosenfeld et al., *Bioorg. Med. Chem*., 1998, 1185] eine Zyklisierung zu den Benzimidazolen ***G*** eingehen. Die Umsetzung von Phenylendiaminen des Typs ***D*** mit Arylisocyaniddichloriden [J. Musco, D.B. Murphy, *J. Org.* Chem., 1971, 3469] stellt einen weiteren Zugang zu Benzimidazolen des Typs ***G*** dar.

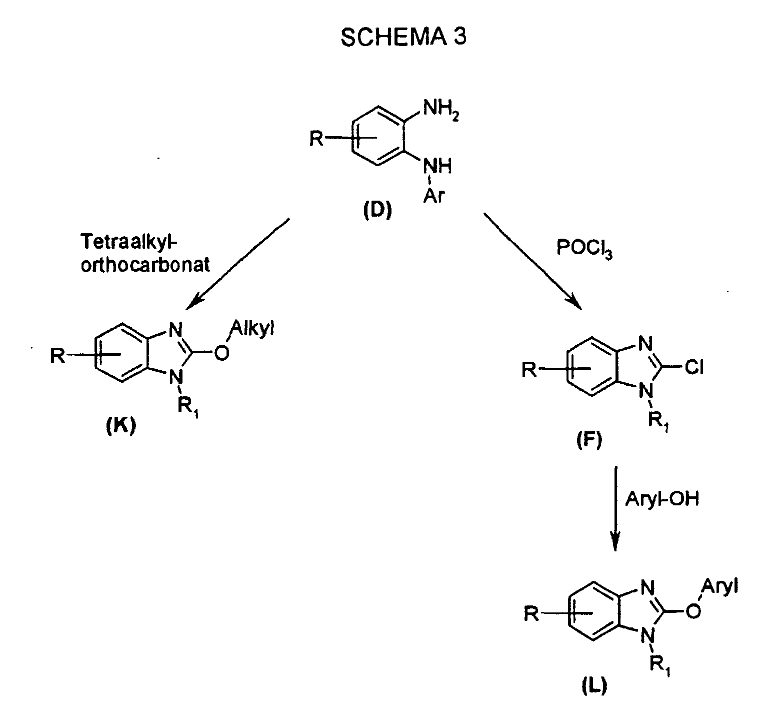

Aus den Diaminen vom Typ ***D*** sind beispielsweise durch Umsetzung mit Tetraalkylorthocarbonaten [M.J. Kukla, H.J. Breslin, C.J. Diamond,P.P. Grous, C.Y. Ho et al., *J. Med. Chem.,* 1991, 3187; Y. Abe, H. Kayakiri, S. Satoh, T. Inoue, Y. Sawada et al., *J. Med. Chem*., 1998, 4062] 2-Alkoxybenzimidazole ***(K)*** zugänglich. 2-Aryloxybenzimidazole ***(L)*** können beispielsweise durch basenvermittelte Reaktion von Phenolen mit 2-Chlorbenzimidazolen ***(F)*** [M. V. Kulkarni, V.D. Patil, *Arch. Pharm.,* 1981; 440] dargestellt werden.

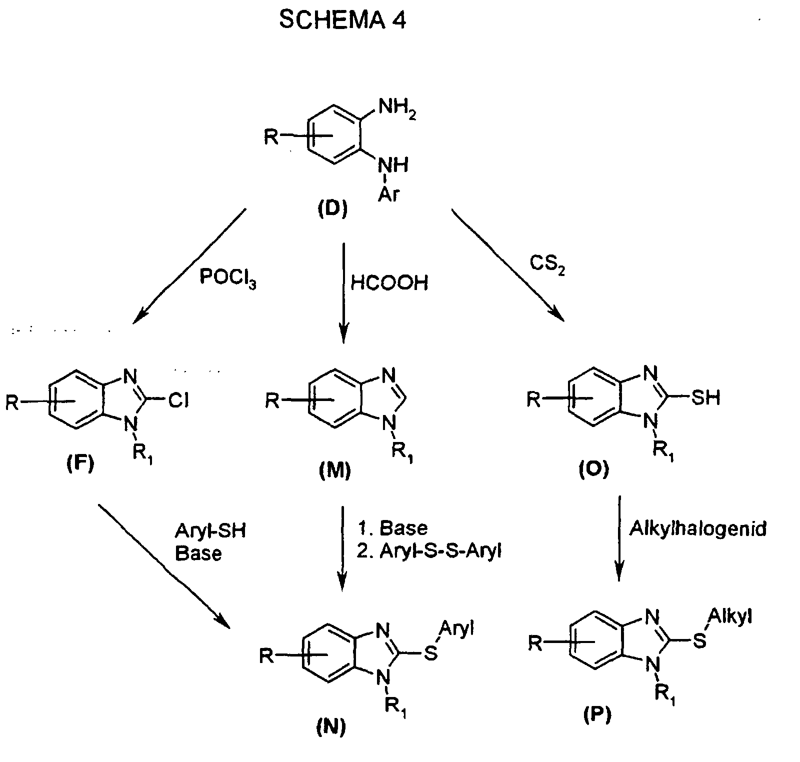

Aus 2-Chlorbenzimidazolderivaten ***(F)*** lassen sich durch Behandlung mit Arylthiolen und Basen 2-Arylmercaptobenzimidazole ***(N)*** darstellen [K. Hirai, H. Koike, T. Ishiba, S. Ueda, I. Makino et al., *Eur. J. Med. Chem. Chim. Ther.,* 1991, 143]. Diese 2-Arylthiobenzimidazole sind auch zugänglich, indem man Phenylendiamine des Typs ***D*** beispielsweise mit Trialkylorthoformiaten [A.J. Freyer, C.K. Lowe-Ma, R.A. Nissan, W.S. Wilson, *Aust. J. Chem*., 1992, 525] oder mit Ameisensäure [S. Abuzar, S. Sharma, *Z. Naturforsch. B Anorg. Chem. Org. Chem*., 1981, 108] zu 2-unsubstituierten Benzimidazolen ***(M)*** zyklisiert und diese Benzimidazole dann mit starken Basen deprotoniert und mit Diaryldisulfiden zur Reaktion bringt [S. Ohta, et al., *J. Chem. Soc. Perkin Trans.,* 1, 2001, 429]. 2-Alkylmercaptobenzimidazole ***(P)*** sind beispielsweise dadurch zugänglich, dass man Phenylendiamine des Typs ***D*** mit Kohlenstoffdisulfid zu 2-Mercaptobenzimidazolen ***(O)*** zyklisiert [E.R. Lavagnino, D.C. Thompson, *J. Heterocycl. Chem.,* 1972, 149; E.L. Ellsworth, J. Domagala, J.V.N. Prasad, S. Hagen, D. Ferguson et al., *Bioorg. Med. Chem. Lett*.; 1999, 2019], die man dann durch basenvermittelte S-Alkylierung [E. Nicolai, J. Goyard, T. Benchetrit, J.-M. Teulon, F. Caussade et al., *J. Med. Chem.,* 1993, 1176] zu den 2-Alkylmercaptobenzimidazolen ***(P)*** umsetzt. Die 2-Arylmercapto- und 2-Alkylmercaptobenzimidazole ***(N)*** und ***(P)*** lassen sich nach bekannten Verfahren, etwa durch Reaktion mit *m*-Chlorperbenzoesäure in die Sulfoxide [J.C. Sih, W.B. Im, A. Robert, D.R. Graber, D.P. Blakeman, *J. Med. Chem*., 1991, 1049; S.C. Yoon, K Kim, *J. Org. Chem.,* 1996, 793] oder Sulfone [D.E. Beattie, R. Crossley, K.H. Dickinson, M. Dover, *Eur. J. Med. Chem. Chim. Ther*., 1983, 277-285] überführen.

Für den Fachmann ist es selbstverständlich, dass die Substituenten ***R*** mit den im Verlauf der Synthesesequenz verwendeten Reagenzien und unter den Reaktionsbedingungen verträglich sein müssen. Gegebenenfalls können die Substituenten später modifiziert werden.
Letztlich sei erwähnt, dass in einigen Fällen die Möglichkeit der direkten N-Arylierung von vorgefertigten Benzimidazolen besteht, beispielsweise nach M.J. Sansone, M.S. Kwiatek, US-A-4,933,397 oder D.M.T. Chan, K.L. Monaco, R.-P. Wang, M. P. Winters, *Tet. Lett*., 39 (1998) 2933 oder A.P. Combs, S. Saubern, M. Rafalski, P.Y.S. Lam, *Tet. Lett*., 40 (1999) 1623.

Wird das Strukturelement ***B-A-Y*** (Formel I) in geschützter oder ungeschützter Form wegen Unverträglichkeit mit den Reaktionsbedingungen während der jeweiligen Benzimidazolsynthese oder aus sonstigen synthetischen Gründen erst nach abgeschlossener Benzimidazolsynthese etabliert, so sind je nach mitgebrachten Substituenten ***R***^{***3***} am Benzolring des Benzimidazolgerüstes verschiedene Vorgehensweisen zur Etablierung des ***B-A-Y***-Strukturelements Formel I) möglich, wobei, was für den Fachmann selbstverständlich ist, eine Verträglichkeit der verwendeten Methoden mit den Arylsubstituenten und weiteren Resten ***R***^{***3***} berücksichtigt werden muss.

Im folgenden sind einige Möglichkeiten zur Etablierung des ***B-A-Y***-Strukturelements aufgezeigt:

Sauerstoff kann in freier Form oder auch in geschützter Form, beispielsweise als Alkylether [vgl. zum Beispiel: B.D. Jerchel, H. Fischer, M. Graft, *Ann. Chem*., 575,162 (1952)] von vornherein als Substituent in eine Benzimidazolsynthese mitgebracht werden. Durch Alkyletherspaltung beispielsweise mit konzentrierter Bromwasserstoffsäure unter eventueller Zuhilfenahme von Lösungsvermittlern wie halogenierten Kohlenwasserstoffen oder auch mit Bortribromid in inerten Lösungsmitteln wie etwa Dichlormethan lässt sich die Hydroxylgruppe freisetzen. Die Hydroxylfunktion lässt sich nach bekannten Methoden mit gegebenenfalls einer endständigen Gruppe ***B*** (Formel I) oder eine Vorstufe davon enthaltenden Alkyl- und Allylhalogeniden zu den Ethern umsetzen, wobei die Umsetzung mit den Alkylierungsmitteln bevorzugt in polaren Lösungsmitteln, wie etwa Dimethylformamid, Dimethylsulfoxid, Ethern, wie etwa Tetrahydrofuran oder auch niederen Ketonen, wie Aceton oder Methylethylketon, unter Zugabe von Basen, wie Alkali- und Erdalkalihydriden, bevorzugt jedoch Natriumhydrid, oder unter Zugabe von Alkalicarbonaten, wie Kalium- oder Cäsiumcarbonat, durchgeführt wird in einem Temperaturbereich von 0°C bis 120°C. Des weiteren kann eine Umsetzung in einem Zweiphasensystem unter Phasentransferkatalyse erfolgen, wobei die Reaktanden in einem geeigneten inerten organischen Lösungsmittel gelöst werden, wie beispielsweise in Halogenalkanen, bevorzugt jedoch in Dichlormethan. Die andere Phase ist ein festes Alkalihydroxid, bevorzugt Natrium- oder Kaliumhydroxid, oder auch eine konzentrierte wässrige Lösung des betreffenden Hydroxids. Als Phasentransferkatalysatoren werden beispielsweise quartäre Ammoniumsalze verwendet. Reaktionen unter Phasentransferkatalyse werden bevorzugt bei Raumtemperatur durchgeführt.

Beispielsweise wird ein Phenolderivat in Dimethylformamid gelöst und unter Zugabe von Cäsiumcarbonat mit 6-Bromhexansäuremethylester bei Temperaturen von 0°C bis 50°C zu einer Verbindung der Formel I umgesetzt.

Die Spaltung des Esters durch saure oder alkalische Hydrolyse lässt sich nach den dem Fachmann bekannten Methoden durchführen, wie beispielsweise mit basischen Katalysatoren wie zum Beispiel mit Alkali- oder Erdalkalicarbonaten oder -hydroxiden in einem Alkohol oder der wässrigen Lösung eines Alkohols. Als Alkohole kommen aliphatische Alkohole, wie beispielsweise Methanol, Ethanol, Butanol etc. in Betracht, vorzugsweise jedoch Methanol. Auch wässrige Lösungen von Ethern wie Tetrahydrofuran finden Verwendung. Als Alkalicarbonate und -hydroxide seien Lithium-, Natrium- und Kaliumsalze genannt. Bevorzugt sind die Lithium- und Natriumsalze. Als Erdalkalicarbonate und -hydroxide eignen sich beispielsweise Calciumcarbonat, Calciumhydroxid und Bariumcarbonat. Die Umsetzung wird allgemein bei -10°C bis 70°C, vorzugsweise jedoch bei 25°C, durchgeführt. Die Esterspaltung kann jedoch auch unter sauren Bedingungen wie etwa in wässriger Salzsäure, gegebenenfalls unter Zuhilfenahme eines Lösungsvermittlers wie etwa einem niederen Alkohol, bevorzugt Methanol, erfolgen.

Des weiteren können die Alkylierungsreagenzien neben dem Halogenatom als weitere funktionelle Gruppe ein Tetrazol in geschützter Form, zum Beispiel trityliert, tragen, woraus dann nach der Alkylierung das Tetrazol freigesetzt wird. Aus einem im Alkylierungsreagenz vorhandenen oder auch nachträglich generiertem Nitril lässt sich später ebenfalls ein Tetrazol herstellen. Hierzu wird das Alkylierungsprodukt mit einem Azid wie etwa Tributylzinnazid oder Natriumazid in einem geeigneten Lösungsmittel wie beispielsweise in aromatischen Kohlenwasserstoffen durch Erwärmung zur Reaktion gebracht. Auch lässt sich ein Nitril durch Hydrolyse in eine Carbonsäurefunktion überführen. Die Alkylierungsreagenzien können auch funktionelle Gruppen wie etwa Hydroxylfunktionen in freier oder geschützter Form enthalten, welche sich nach Überführung in Abgangsgruppen wie zum Beispiel Tosylat, Mesylat, Bromid oder Jodid beispielsweise gegen Cyanide, Amine, Alkyl-, Aryl-, oder Heteroarylbausteine austauschen lassen. Auch können die Alkylierungsreagenzien funktionelle Gruppen, wie beispielsweise Halogene oder gegebenenfalls geschützte Amino- oder Mercaptogruppen, enthalten.

Die Einführung von Stickstoff gelingt beispielsweise, indem Nitrobenzimidazole, die nach literaturbekannten Verfahren [siehe zum Beispiel: K. Bougrin, M. Soufiaoui, *Tet. Lett*., 36, 21, 1995, 3683-3686; J.J.V. Eynde, F. Delfosse, P. Lor, Y.V. Haverbeke, *Tetrahedron,* 51, 20, 1995, 5813-5818; Q. Sun, B. Yan, *Bioorg. Med. Chem. Lett*., 1998, 361-364; Sandera et al., *J. Amer. Chem. Soc.* 76, 1954, 5173] zugänglich sind, am Stickstoff des Benzimidazols N-aryliert werden (siehe oben) und dann die Nitrogruppe etwa durch Hydrierung in polaren Lösungsmitteln, wie Essigsäure, niederen Alkoholen oder Essigestern, unter Zugabe von Katalysatoren, wie Raney-Nickel oder Palladium auf Kohle, oder durch chemische Reduktion beispielsweise mit Zinn in Salzsäure oder SnCl₂ (siehe oben) zur Aminogruppe reduziert wird. Derart etablierte Aminogruppen lassen sich dann analog zu den Hydroxygruppen (siehe oben) mit Alkylhalogeniden wie beispielsweise mit ω-Bromalkansäureestern mono- oder bisalkylieren oder nach literaturbekannten Verfahren in Sulfonamide überführen. Auch gelingt es beispielsweise nach Standardverfahren, die N-monoalkylierten Aminobenzimidazole mit Säurederivaten, wie etwa Säureanhydriden oder Säurehalogeniden, zu acylieren oder mit Sulfonsäureanhydriden oder Sulfonsäurehalogeniden in die entsprechenden Sulfonsäureamide zu überführen.

Auch können *N*-Aryl-2-nitrobenzole ***(C)***, in denen ***R*** für Cl oder F steht, wie beispielsweise (5-Chlor-2-nitrophenyl)phenylamin, durch Umsetzung mit primären oder sekundären Aminen in die entsprechenden aminsubstituierten *N*-Aryl-2-nitrobenzole überführt werden [siehe beispielsweise: D. Evans, T.A. Hicks, W.R.N. Williamson, W. Dawson, S.C.R. Meacock, E.A. Kitchen, *Eur. J. Med. Chem. Chim. Ther*., 31, 7-8, 1996,635-642]. Nach Reduktion der Nitrogruppe und Ringschluss zum Benzimidazol (vgl. Schemata 2-4) lassen sich so aminsubstituierte Benzimidazole der Typen ***G, H, K, L, N*** und ***P*** gewinnen.

Je nach angestrebter Substitution sind die Substituenten ***R***^{***3***} von vornherein in den Synthesebausteinen enthalten oder werden nach Bedarf an geeigneter Stelle der betreffenden Synthesesequenz etabliert beziehungsweise aus mitgebrachten geeigneten Vorläufern generiert. So lassen sich mitgebrachte Nitrogruppen zu den entsprechenden Aminen nach bereits oben beschriebenen Verfahren reduzieren und in Carboxyaminogruppen überführen. Sulfonylaminogruppen sind aus den Aminoverbindungen nach Standardverfahren zugänglich. So wird zum Beispiel ein Amin oder sein Hydrochlorid in einem geeigneten inerten Lösungsmittel wie einem aromatischen Kohlenwasserstoff, zum Beispiel Toluol, oder einem Halogenalkan, beispielsweise Dichlormethan, unter Zuhilfenahme einer Base, wie etwa Triethylamin oder Pyridin, mit einem Sulfonsäurehalogenid bei 0°C bis 120°C umgesetzt. Nitrile können zum Beispiel mit Grignardreagenzien oder Lithiumorganylen in Ketone überführt oder zu Säuren beziehungsweise Amiden hydrolysiert werden. Es ist für den Fachmann selbstverständlich, dass die hier verwendeten Reaktionsbedingungen mit den restlichen im Molekül befindlichen Gruppierungen verträglich sein müssen.

Die freien Säurederivate der Formel I können nach diversen, literaturbekannten Verfahren in Amidderivate oder Esterderivate der Formel I überführt werden.

Die freien Säurederivate der Formel I lassen sich auch mit geeigneten Mengen der entsprechenden anorganischen Basen unter Neutralisierung in Salze überführen. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, zum Beispiel von Alkohol oder Aceton, das feste Salz.

Die Aminsalze werden in üblicher Weise hergestellt. Dazu löst man die entsprechende Säure in einem geeigneten Lösungsmittel, wie beispielsweise Ethanol, Aceton, Diethylether oder Benzol, und setzt dieser Lösung ein bis fünf Äquivalente des jeweiligen Amins zu. Dabei fällt das Salz gewöhnlich in fester Form an oder wird nach Verdampfen des Lösungsmittels in üblicher Weise isoliert.

Die Clathrate mit α-, β- oder γ-Cyclodextrin werden analog der Vorschrift in WO-A-87/05294 erhalten. Bevorzugt wird β-Cyclodextrin verwendet.

Liposomen werden nach dem in *Pharmazie in unserer Zeit,* 11, 98 (1982) beschriebenen Verfahren hergestellt.

Die erfindungsgemäßen Benzimidazolderivate werden analog zu bekannten Verfahren hergestellt: Verfahren zu deren Herstellung sind beispielsweise in EP 0 531 883 A1 beschrieben. Soweit die Herstellung der Ausgangsverbindungen nicht beschrieben wird, sind die Ausgangsverbindungen bekannt und käuflich, oder die Verbindungen werden analog zu den beschriebenen Verfahren synthetisiert. Nachfolgend wird die Herstellung einiger Vorstufen, Zwischenprodukte und Produkte exemplarisch beschrieben.

Bei der Herstellung der erfindungsgemäßen Substanzen bedient man sich beispielsweise folgender Verfahren:

### Allgemeine Arbeitsvorschrift 1:

### Reduktion Nitrogruppen

Die zu hydrierende Verbindung wird in Ethylacetat, Tetrahydrofuran oder Ethanol oder Gemischen der Lösungsmittel gelöst und an 2 bis 5% (bezogen auf die Nitroverbindung) Palladium auf Kohle (10%) bei normalem Druck hydriert. Nach Ende der Wasserstoffaufnahme wird abgesaugt, der Rückstand mit Ethylacetat oder Ethanol gewaschen und das Filtrat im Vakuum eingeengt. Das Rohprodukt wird ohne weitere Reinigung umgesetzt.

### Allgemeine Arbeitsvorschrift 2:

### Alkylierung von Phenolderivaten mit Alkylhalogeniden

Eine Lösung von 1,85 mmol des Phenolderivats in 12 ml *N,N*-Dimethylformamid wird mit 1,85 mmol Caesiumcarbonat, und 2,24 mmol Alkyljodid versetzt. Man rührt 12 bis 96 Stunden lang, giesst dann auf Wasser, nimmt mit Ethylacetat auf, wäscht die organische Phase viermal mit Wasser, trocknet diese über Natriumsulfat und engt im Vakuum ein.

Alternativ zu dieser wässrigen Aufarbeitung kann man das Reaktionsgemisch mit Dichlormethan versetzen, von den ausfallenden Salzen durch Filtration trennen und das Filtrat im Vakuum einengen.

Unabhängig von der Aufarbeitungsmethode wird der Rückstand durch Kristallisation oder Säulenchromatographie an Kieselgel gereinigt

### Allgemeine Arbeitsvorschrift 3:

### Verseifung von Carbonsäurealkylestern

0,77 mmol des Carbonsäurealkylesters werden in 5 ml Methanol und 5 ml Tetrahydrofuran gelöst und mit 5 ml einer 0,5 N wässrigen Lithium- oder Natriumhydroxidlösung versetzt. Nach 2 bis 12 Stunden langem Rühren wird im Vakuum weitestgehend eingeengt, durch Zusatz von wässriger Salzsäure neutralisiert und mit Ethylacetat extrahiert. Man trocknet über Natriumsulfat und engt im Vakuum ein. Der Rückstand wird falls erforderlich durch Säulenchromatographie an Kieselgel oder durch Kristallisation gereinigt.

### Allgemeine Arbeitsvorschrift 4:

### Überführung von Carbonsäureestern in Carbonsäureamide

0,36 mmol eines Amins werden in 3 ml Toluol gelöst und unter Kühlung im Eisbad tropfenweise mit 0,18 ml einer 2 M Lösung von Trimethylaluminium in Toluol versetzt. Man versetzt mit einer Lösung aus 0,33 mmol des Carbonsäuremethylesters in 3 ml Toluol und rührt 2 bis 8 Stunden lang bei 95°C. Zur Aufarbeitung gibt man nach dem Erkalten Wasser zu, extrahiert dreimal mit Ethylacetat, wäscht die vereinigten organischen Phasen mit ges.

Natriumchloridlösung, trocknet über Natriumsulfat und engt im Vakuum ein. Der Rückstand wird durch Säulenchromatographie an Kieselgel gereinigt.

**Tabelle 1:**

| Erfindungsgemäße Benzimidazolderivate | |
|---|---|
| Nr. | Benzimidazolderivat |
| | |
| 1 | 6-[[1-Phenyl-2-propylmercapto-1*H*-benzimidazol-6-yl]oxy]hexansäuremethylester |
| 2 | 6-[[1-Phenyl-2-propansulfinyl-1*H*-benzimidazol-6-yl]oxy]hexansäuremethylester |
| 3 | 6-[[1-Phenyl-2-propansulfonyl-1*H*-benzimidazol-6-yl]oxy]hexansäuremethylester |
| 4 | 6-[[1-(4-Methylphenyl)-2-propylmercapto-1*H*-benzimidazol-6-yl]oxy]hexansäuremethylester |
| 5 | 6-[[1-(4-Methylphenyl)-2-propansulfinyl-1*H*-benzimidazol-6-yl]oxy]hexansäuremethylester |
| 6 | 6-[[1-(4-Methylphenyl)-2-propansulfonyl-1*H*-benzimidazol-6-yl]oxy]hexansäuremethylester |
| 7 | 6-[[2-Benzylmercapto-1-(4-methylphenyl)-1*H*-benzimidazol-6-yl]oxy]hexansäuremethylester |
| 8 | 6-[[1-(4-Methylphenyl)-2-(phenylmethansulfinyl)-1*H*-benzimidazol-6-yl]oxy]hexansäuremethylester |
| 9 | 6-[[1-(4-Methylphenyl)-2-(phenylmethansulfonyl)-1*H*-benzimidazol-6-yl]oxy]hexansäuremethylester |
| 10 | 6-[[1-(4-Methylphenyl)-2-(2-pyridinyl)mercapto-1*H*-benzimidazol-6-yl]oxy]hexansäuremethylester |
| 11 | *N*-(3-Methoxypropyl)-6-[[1-(4-methylphenyl)-2-propylmercapto-1*H*-benzimidazol-6-yl]oxy]hexanamid |
| 12 | *N*-(3-Methoxypropyl)-6-[[1-(4-methylphenyl)-2-propansulfonyl-1*H*-benzimidazol-6-yl]oxy]hexanamid |
| 13 | *N*-(3-Methoxypropyl)-6-[[2-benzylmercapto-1-(4-methylphenyl)-1*H*-benzimidazol-6-yl]oxy] |
| 14 | N-(3-Methoxypropyl)-6-[[1-(4-Methylphenyl)-2(-phenylmethansulfonyl)-1 H-benzimidazol-6-yl]oxy]hexanamid |
| 15 | 6-[[2-(Morpholin-4-yl)-1-phenyl-1*H*-benzimidazol-6-yl]oxy]hexansäuremethylester |
| 16 | 6-[[2-(Piperidin-1-yl)-1-phenyl-1*H*-benzimidazol-6-yl]oxy]hexansäuremethylester |
| 17 | 6-[[1-(4-Methylphenyl)-2-(morpholin-4-yl)-1*H*-benzimidazol-6-yl]oxy]hexansäuremethylester |
| 18 | 6-[[1-(4-Methylphenyl)-2-(piperidin-1-yl)-1*H*-benzimidazol-6-yl]oxy]hexansäuremethylester |
| 19 | *N*-(3-Methoxypropyl)-6-[[2-(morpholin-4-yl)-1-phenyl-1*H*-benzimidazol-6-yl]oxy]hexanamid |
| 20 | *N*-(3-Methoxypropyl)-6-[[2-(piperidin-1-yl)-1-phenyl-1*H*-benzimidazol-6-yl]oxy]hexanamid |
| 21 | 6-[[2-Methoxy-1-phenyl-1*H*-benzimidazol-6-yl]oxy]hexansäuremethylester |
| 22 | 6-[[2-Methoxy-1-phenyl-1*H*-benzimidazol-6-yl]oxy]hexansäure |
| 23 | 6-[[2-Ethoxy-1-phenyl-1*H*-benzimidazol-6-yl]oxy]hexansäuremethylester |
| 24 | 6-[[2-Ethoxy-1-phenyl-1*H*-benzimidazol-6-yl]oxy]hexansäure |
| 25 | 6-[[1-Phenyl-2-phenylamino-1*H*-benzimidazol-6-yl]oxy]hexansäuremethylester |
| 26 | 6-[[1-Phenyl-2-phenylamino-1*H*-benzimidazol-6-yl]oxy]hexansäure |
| 27 | 6-[[1-(4-Methylphenyl)-2-phenylamino-1*H*-benzimidazol-6-yl]oxy]hexansäuremethylester |
| 28 | 6-[[1-Phenyl-2-propylamino-1*H*-benzimidazol-6-yl]oxy]hexansäuremethylester |
| 29 | 6-[[1-Phenyl-2-propylamino-1*H*-benzimidazol-6-yl]oxy]hexansäure |
| 30 | 6-[[2-(*N*-Methyl-*N*-propyl)amino-1-phenyl-1*H*-benzimidazol-6-yl]oxy]hexansäuremethylester |
| 31 | 6-[[1-(4-Methylphenyl)-2-phenyloxy-1*H*-benzimidazol-6-yl]oxy]hexansäure |
| 32 | 6-[[1-(4-Methylphenyl)-2-phenylmercapto-1*H*-benzimidazol-6-yl]oxy]hexansäuremethylester |
| 33 | 6-[[1-(4-Methylphenyl)-2-(phenylsulfinyl)-1*H*-benzimidazol-6-yl]oxy]hexansäuremethylester |
| 34 | 6-[[1-(4-Methylphenyl)-2-(phenylsulfonyl)-1*H*-benzimidazol-6-yl]oxy]hexansäuremethylester |
| 35 | 6-[[1-Phenyl-2-propylmercapto-1*H*-benzimidazol-6-yl]oxy]hexansäure |
| 36 | 6-[[1-Phenyl-2-propansulfonyl-1*H*-benzimidazol-6-yl]oxy]hexansäure |
| 37 | 6-[(2-Benzylmercapto-1-phenyl-1*H*-benzimidazol-6-yl)oxy]hexansäuremethylester |
| 38 | 6-[[1-Phenyl-2-(phenylmethansulfonyl)-1*H*-benzimidazol-6-yl]oxy]hexansäuremethylester |
| 39 | 6-[(2-Benzylmercapto-1-phenyl-1*H*-benzimidazol-6-yl)oxy]hexansäure |
| 40 | 6-[[1-Phenyl-2-(phenylmethansulfonyl)-1*H*-benzimidazol-6-yl]oxy]hexansäure |
| 41 | 6-[[1-(4-Methylphenyl)-2-propylmercapto-1*H*-benzimidazol-6-yl]oxy]hexansäure |
| 42 | 6-[[1-(4-Methylphenyl)-2-propansulfonyl-1*H*-benzimidazol-6-yl]oxy]hexansäure |
| 43 | 6-[[2-Benzylmercapto-1-(4-methylphenyl)-1*H*-benzimidazol-6-yl]oxy]hexansäure |
| 44 | 6-[[1-(4-Methylphenyl)-2-(phenylmethansulfonyl)-1*H*-benzimidazol-6-yl]oxy]hexansäure |

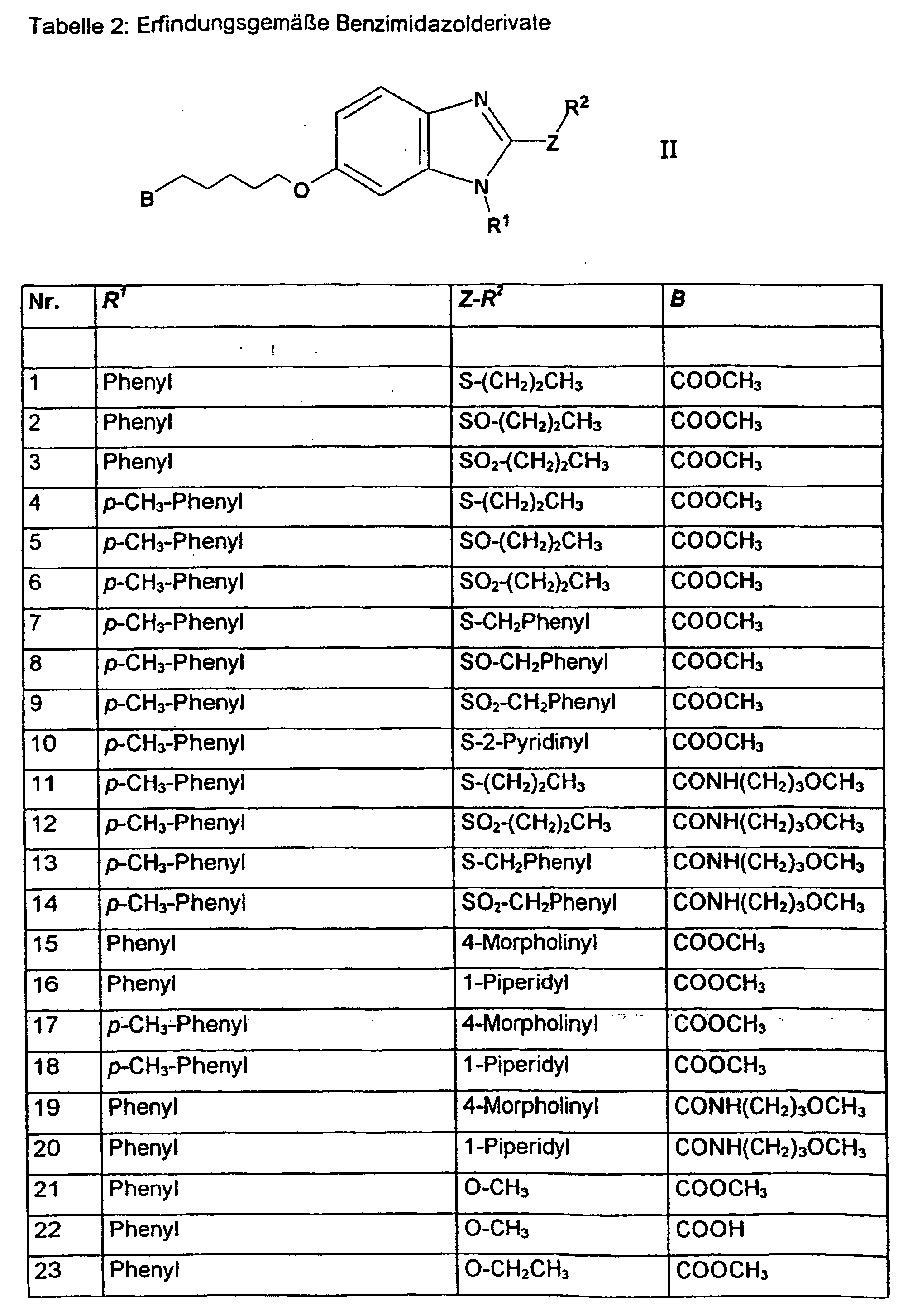

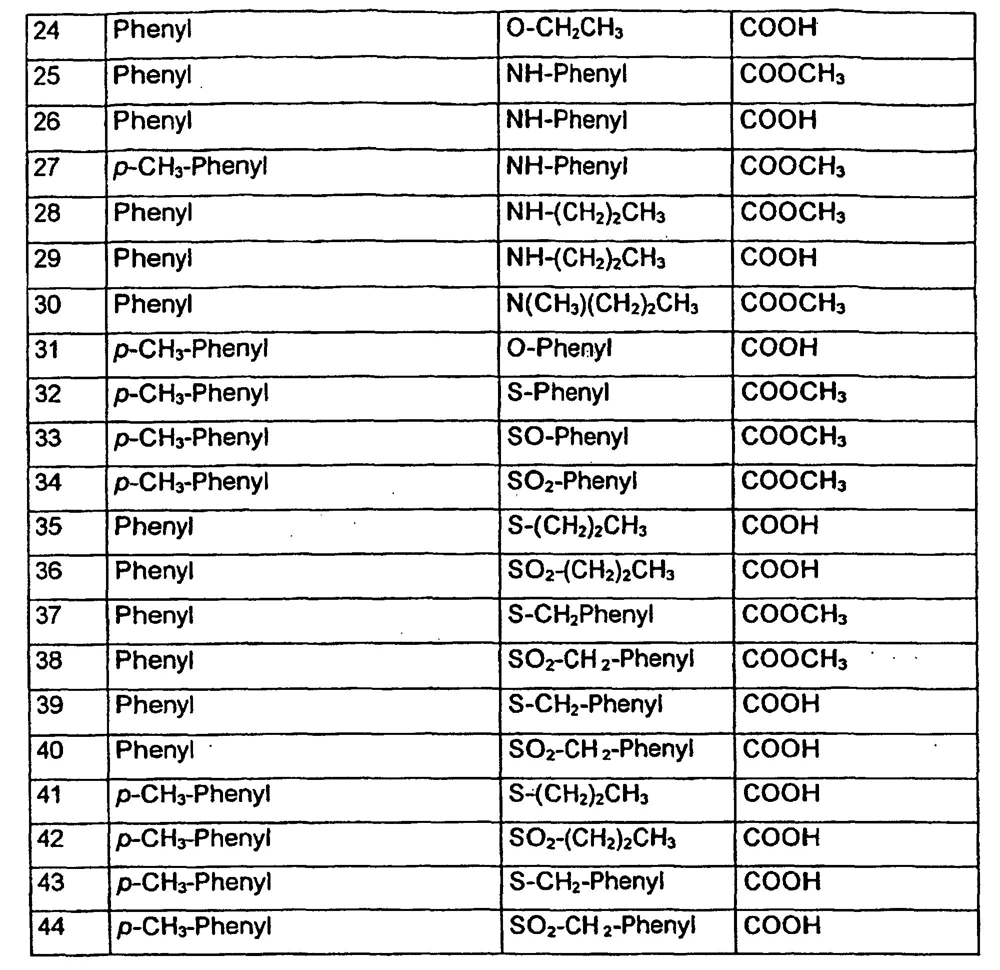

### Beispiel 1

### 6-[[1-Phenyl-2-propylmercapto-1H-benzimidazol-6-yl]oxy]hexansäuremethylester

### a) 3-Phenylamino-4-nitrophenol

14 g 3-Fluor-4-nitrophenol und 24 ml Anilin wurden vermischt und 3 Stunden bei 140°C gerührt. Nach dem Erkalten wurde in Ethylacetat gelöst und sechsmal mit 2 **N** wässriger Salzsäure extrahiert. Die organische Phase wurde mit gesättigter **N**atriumchloridlösung gewaschen, über Natriumsulfat getrocknet, im Vakuum eingeengt und der Rückstand aus Diisopropylether kristallisiert.
Man erhielt 20,8 g.
Fp. 170-172°C

### b) 6-(3-Phenylamino-4-nitrophenyl)oxyhexansäuremethylester

13,3 g 3-Phenylamino-4-nitrophenol wurden mit 6-Bromhexansäuremethylester gemäß der allgemeinen Arbeitsvorschrift 2 umgesetzt. Man erhielt 11,2 g.
¹H-NMR (CDCl₃): δ = 1,38-1,52 ppm m (2H); 1,59-1,80 m (4H); 2,33 t (J = 7,5 Hz, 2H); 3,67 s (3H); 3,87 t (J = 7,5 Hz, 2H); 6,32 dd (J = 8, 2 Hz, 1H); 6,52 d (J = 2 Hz, 1H); 7,20-7,32 m (3H); 7,40-7,48 m (2H); 8,18 d (J = 8 Hz, 1H); 9,75 s (breit) (1H).

### c) 6-(-3-Phenylamino-4-aminophenyl)oxyhexansäuremethylester

4,5 g 6-(3-Phenylamino-4-nitrophenyl)oxyhexansäuremethylester wurden durch Umsetzung gemäß der allgemeinen Arbeitsvorschrift 1 reduziert. Man erhielt 4,3 g.
¹H-NMR (CDCl₃): δ = 1,42-1,52 ppm m (2H); 1,62-1,83 m (4H); 2,34 t (J = 7,5 Hz, 2H); 3,68 s (3H); 3,86 t (J = 7,5 Hz, 2H); 5,32 s(breit) (1H); 6,54 dd (J = 8, 2 Hz, 1H); 6,74 d (J = 8 Hz, 1H); 6,78 d (J = 2 Hz, 1H); 6,80-6,90 m (3H); 7,20-7,29 m (2H).

### d) 6-[[2-Mercapto-1-phenyl-1H-benzimidazol-6-yl]oxy]hexansäuremethylester

0,4 g 6-(3-Phenylamino-4-aminophenyl)oxyhexansäuremethylester wurden in 5 ml Pyridin gelöst, mit 75 µl Kohlenstoffdisulfid versetzt und die Mischung 12 Stunden bei 20°C gerührt. Man versetzte mit ges. Natriumhydrogencarbonatlösung, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 368 mg.
Fp. 122-123°C

### e) 6-[[1-Phenyl-2-propylmercapto-1H-benzimidazol-6-yl]oxy]hexansäuremethylester

100 mg 6-[[2-Mercapto-1-phenyl-1*H*-benzimidazol-6-yl]oxy]hexansäuremethylester wurden in 2 ml *N,N*-Dimethylformamid gelöst, mit 40 µl Propyljodid, 57 mg Kaliumhydrogencarbonat und 10 mg Dicyclohexano-18-krone-6 versetzt und die Mischung 15 Stunden bei 20°C gerührt. Man filtrierte und engte das Filtrat im Vakuum ein. Der Rückstand wurde durch Säulenchromatographie an Kieselgel gereinigt. Es wurden 103 mg erhalten.
Fp. 45.5-47°C

### Beispiel 2

### 6-[[1-Phenyl-2-propansulfinyl-1H-benzimidazol-6-yl]oxy]hexansäuremethylester

80 mg 6-[[1-Phenyl-2-propylmercapto-1*H*-benzimidazol-6-yl]oxy]hexansäuremethylester wurden in 2 ml Dichlormethan gelöst, mit 56 mg *m*-Chlorperbenzoesäure (ca. 60%ig) versetzt und die Mischung 10 Minuten bei 20°C gerührt. Man versetzte mit Natriumdisulfitlösung, extrahierte zweimal mit Dichlormethan, wusch die vereinigten organischen Phasen mit ges.
Natriumhydrogencarbonatlösung, Wasser und ges. Natriumchloridlösung, trocknete über Natriumsulfat und engte im Vakuum ein. Der Rückstand wurde durch Säulenchromatographie an Kieselgel gereinigt. Es wurden 30 mg erhalten.
¹H-NMR (CDCl₃): δ = 1,06 ppm t (J = 8 Hz, 3H); 1,43-1,55 m (2H); 1,62-1,85 m (6H); 2,33 t (J = 7,5 Hz, 2H); 3,24-3,36 m (1H); 3,47-3,58 m (1H); 3,67 s (3H); 3,90 t (J = 7,5 Hz, 2H); 6,67 d (J = 2 Hz, 1H); 7,02 dd (J = 8, 2 Hz, 1H); 7,50-7,69 m (5H); 7,78 d (J = 8 Hz, 1H).

### Beispiel 3

### 6-[[1-Phenyl-2-propansulfonyl-1H-benzimidazol-6-yl]oxy]hexansäuremethylester

85 mg 6-[[1-Phenyl-2-propylmercapto-1*H*-benzimidazol-6-yl]oxy]hexansäuremethylester wurden in 2 ml Dichlormethan gelöst, mit 59 mg m-Chlorperbenzoesäure (ca. 60%ig) versetzt und die Mischung 9 Stunden bei 20°C gerührt. Man versetzte mit Natriumdisulfitlösung, extrahierte zweimal mit Dichlormethan, wusch die vereinigten organischen Phasen mit ges. Natriumhydrogencarbonatlösung, Wasser und ges. Natriumchloridlösung, trocknete über Natriumsulfat und engte im Vakuum ein. Der Rückstand wurde durch Säulenchromatographie an Kieselgel gereinigt. Es wurden 30 mg erhalten.
MS (EI): 444 (Molekülionpeak)

### Beispiel 4

### 6-[[1-(4-Methylphenyl)-2-propylmercapto-1H-benzimidazol-6-yl]oxy]hexansäuremethylester

### a) 3-(4-Methylphenyl)amino-4-nitrophenol

1.3 g g 3-Fluor-4-nitrophenol und 2.7 g 4-Methylanilin wurden vermischt und 14 Stunden bei 140°C gerührt. Nach dem Erkalten wurde in Ethylacetat gelöst und dreimal mit 4 N wässriger Salzsäure extrahiert. Die organische Phase wurde mit ges. Natriumchloridlösung gewaschen, über Natriumsulfat getrocknet, im Vakuum eingeengt und der Rückstand aus Diisopropylether kristallisiert. Man erhielt 1.70 g.
¹H-NMR (CDCl₃): δ = 2,34 ppm s (3H); 6,25 dd (J = 8, 2 Hz, 1H); 6,48 d (J = 2 Hz, 1H); 7,12-7,20 m (4H); 8,08 d (J = 8 Hz, 1H); 9,64 s (breit) (1H); 9,72 s (breit) (1H).

### b) 6-[3-(4-Methylphenyl)amino-4-nitrophenyl]oxyhexansäuremethylester

990 mg 3-(4-Methylphenyl)amino-4-nitrophenol wurden mit 6-Bromhexansäuremethylester gemäß der allgemeinen Arbeitsvorschrift 2 umgesetzt. Man erhielt 1.5 g.
¹H-NMR (CDCl₃): δ = 1,38-1,50 ppm m (2H); 1,60-1,80 m (4H); 2,33 t (J = 7,5 Hz, 2H); 2,39 s (3H); 3,68 s (3H); 3,85 t (J = 7,5 Hz, 2H); 6,28 dd (J = 8, 2 Hz, 1H); 6,45 d (J = 2 Hz, 1H); 7,15 d (J = 8 Hz, 2H); 7,24 d (J = 8 Hz, 2H); 8,18 d (J = 8 Hz, 1H); 9,70 s (breit) (, 1H).

### c) 6-[3-(4-Methylphenyl)amino-4-aminophenyl]oxyhexansäuremethylester

1,3 g 6-[3-(4-Methylphenyl)amino-4-nitrophenyl]oxyhexansäuremethylester wurden durch Umsetzung gemäß der allgemeinen Arbeitsvorschrift 1 reduziert. Man erhielt 1,19 g.
¹H-NMR (CDCl₃): δ = 1,40-1,54 ppm m (2H); 1,62-1,78 m (4H); 2,30 s (3H); 2,33 t (J = 7,5 Hz, 2H); 3,68 s (3H); 3,85 t (J = 7,5 Hz, 2H); 5,27 s (breit) (1H); 6,50 dd (J = 8, 2 Hz, 1H); 6,72 d (J = 2 Hz, 1H); 6,74 d (J = 8 Hz, 1H); 6,80 d (J = 8 Hz, 2H); 7,04 d (J = 8 Hz, 2H).

### d) 6-[[2-Mercapto-1-(4-methylphenyl)-1H-benzimidazol-6-yl]oxy]hexansäuremethylester

3 g 6-[3-(4-Methylphenyl)-4-aminophenyl]oxyhexansäuremethylester wurden in 15 ml Pyridin gelöst, mit 0,6 ml Kohlenstoffdisulfid versetzt und die Mischung 20 Stunden bei 20°C gerührt. Man versetzte mit ges. Natriumhydrogencarbonatlösung, extrahierte dreimal mit Ethylacetat, wusch die vereinigten organischen Phasen mit ges. Natriumchloridlösung, trocknete über Natriumsulfat und engte im Vakuum ein. Der Rückstand wurde aus Diisopropylether kristallisiert. Man erhielt 2,6 g.
¹H-NMR (CDCl₃): δ = 1,40-1,52 ppm m (2H); 1,60-1,82 m (4H); 2,33 t (J = 7,5 Hz, 2H); 2,48 s (3H); 3,66 s (3H); 3,89 t (J = 7,5 Hz, 2H); 6,46 d (J = 2 Hz, 1H); 6,80 dd (J = 8, 2 Hz, 1H); 7,16 d (J = 8 Hz, 1H); 7,40 s (4H).

### e) 6-[[1-(4-Methylphenyl)-2-propylmercapto-1H-benzimidazol-6-yl]oxy]hexansäuremethylester

1 g 6-[[2-Mercapto-1-(4-methylphenyl)-1*H*-benzimidazol-6-yl]oxy]hexansäuremethylester wurden in 15 ml *N,N*-Dimethylformamid gelöst, mit 0,3 ml Propyljodid, 0,55 g Kaliumhydrogencarbonat und 97 mg Dicyclohexano-18-krone-6 versetzt und die Mischung 48 Stunden bei 20°C gerührt. Man filtrierte und engte das Filtrat im Vakuum ein. Der Rückstand wurde durch Säulenchromatographie an Kieselgel gereinigt. Man erhielt 1,06 g.
¹H-NMR (CDCl₃): δ = 1,02 ppm t (J = 8 Hz, 3H); 1,43-1,52 m (2H); 1,62-1,83 m (6H); 2,32 t (J = 7,5 Hz, 2H); 2,48 s (3H); 3,30 t (J = 8 Hz, 2H); 3,67 s (3H); 3,90 t (J = 7,5 Hz, 2H); 6,56 d (J = 2 Hz, 1H); 6,83 dd (J = 8, 2 Hz, 1H); 7,32 d (J = 7,5 Hz, 2H); 7,39 d (J = 7,5 Hz, 2H); 7,58 d (J = 8 Hz, 1H).

### Beispiel 5

### 6-[[1-(4-Methylphenyl)-2-propansulfinyl-1H-benzimidazol-6-yl]oxy]hexansäuremethylester

### Beispiel 6

### 6-[[1-(4-Methylphenyl)-2-propansulfonyl-1H-benzimidazol-6-yl]oxy]hexansäuremethylester

600 mg 6-[[1-(4-Methylphenyl)-2-propylmercapto-1*H*-benzimidazol-6-yl]oxy]hexansäuremethylester wurden in 10 ml Dichlormethan gelöst, mit 405 mg *m*-Chlorperbenzoesäure (ca. 60%ig) versetzt und die Mischung 15 Minuten bei 20°C gerührt. Man versetzte mit Natriumdisulfitlösung, extrahierte zweimal mit Dichlormethan, wusch die vereinigten organischen Phasen mit ges. Natriumhydrogencarbonatlösung, Wasser und ges. Natriumchloridlösung, trocknete über Natriumsulfat und engte im Vakuum ein. Der Rückstand wurde durch Säulenchromatographie an Kieselgel gereinigt. Man erhielt 525 mg 6-[[1-(4-Methylphenyl)-2-propansulfinyl-1*H*-benzimidazol-6-yl]oxy]hexansäuremethylester,
¹H-NMR (CDCl₃): δ = 1,05 ppm t (J = 8 Hz, 3H); 1,43-1,54 m (2H); 1,62-1,78 m (6H); 2,33 t (J = 7,5 Hz, 2H); 2,50 s (3H); 3,25-3,35 m (1H); 3,43-3,53 m (1H); 3,67 s (3H); 3,90 t (J = 7,5 Hz, 2H); 6,62 d (J = 2 Hz, 1H); 7,00 dd (J = 8, 2 Hz, 1H); 7,40 s (4H); 7,78 d (J = 8 Hz, 1H),
und 239 mg 6-[[1-(4-Methylphenyl)-2-propansulfonyl-1*H*-benzimidazol-6-yl]oxy]hexansäuremethylester,
¹H-NMR (CDCl₃): δ = 1,04 ppm t (J = 8 Hz, 3H); 1,42-1,54 m (2H); 1,62-1,92 (6H); 2,34 t (J = 7,5 Hz, 2H); 2,48 s (3H); 3,48 t (J = 8 Hz, 2H); 3,67 s (3H); 3,88 t (J = 7,5 Hz, 2H); 6,52 d (J = 2 Hz, 1H); 7,00 dd (J = 8, 2 Hz, 1H); 7,39 s (4H); 7,76 d (J = 8 Hz, 1H).

### Beispiel 7

### 6-[[2-Benzylmercapto-1-(4-methylphenyl)-1H-benzimidazol-6-yl]oxy]hexansäuremethylester

800 mg 6-[[2-Mercapto-1-(4-methylphenyl)-1*H*-benzimidazol-6-yl]oxy]hexansäuremethylester wurden in 8 ml *N,N*-Dimethylformamid gelöst, mit 0,3 ml Benzylbromid, 438 mg Kaliumhydrogencarbonat und 75 mg Dicyclohexano-18-krone-6 versetzt und die Mischung 15 Stunden bei 20°C gerührt. Man filtrierte und engte das Filtrat im Vakuum ein. Der Rückstand wurde durch Säulenchromatographie an Kieselgel gereinigt. Man erhielt 976 mg.
MS (EI): 474 (Molekülionpeak)

### Beispiel 8

### 6-[[1-(4-Methylphenyl)-2-(phenylmethansulfinyl)-1H-benzimidazol-6-yl]oxy]hexansäuremethylester

### Beispiel 9

### 6-[[1-(4-Methylphenyl)-2-(phenylmethansulfonyl)-1H-benzimidazol-6-yl]oxy]hexansäuremethylester

500 mg 6-[[2-Benzylmercapto-1-(4-Methylphenyl)-1*H*-benzimidazol-6-yl]oxy]hexansäuremethylester wurden in 9 ml Dichlormethan gelöst, mit 303 mg *m*-Chlorperbenzoesäure (ca. 60%ig) versetzt und die Mischung 2 Stunden bei 20°C gerührt. Man versetzte mit Natriumdisulfitlösung, extrahierte zweimal mit Dichlormethan, wusch die vereinigten organischen Phasen mit ges. Natriumhydrogencarbonatlösung, Wasser und ges. Natriumchloridlösung, trocknete über Natriumsulfat und engte im Vakuum ein. Der Rückstand wurde durch Säulenchromatographie an Kieselgel gereinigt. Man erhielt 232 mg 6-[[1-(4-Methylphenyl)-2-(phenylmethansulfinyl)-1*H*-benzimidazol-6-yl]oxy]hexansäuremethylester,
¹H-NMR (CDCl₃): δ = 1,42-1,53 ppm m (2H); 1,62-1,84 m (4H); 2,32 t (J = 7,5 Hz, 2H); 2,42 s (3H); 3,64 s (3H); 3,88 t (J = 7,5 Hz, 2H); 4,58 d (J = 12 Hz, 1H); 4,88 d (J = 12 Hz, 1H); 6,50 d (J = 2 Hz, 1H); 6,75-6,93 m (2H); 7,01 dd (J = 8, 2 Hz, 1H); 7,12 d (J = 8 Hz, 2H); 7,20-7,33 m (5H); 7,82 d (J = 8 Hz, 1H),
und 189 mg 6-[[1-(4-Methylphenyl)-2-(phenylmethansulfonyl)-1*H*-benzimidazol-6-yl]oxy]hexansäuremethylester,
¹H-NMR (CDCl₃): δ = 1,42-1,54 ppm m (2H); 1,58-1,82 m (4H); 2,34 t (J = 7,5 Hz, 2H); 2,42 s (3H); 3,67 s (3H); 3,86 t (J = 7,5 Hz, 2H); 4,75 s (2H); 6,38 d (J = 2 Hz, 1H); 6,86 d (J = 8 Hz, 2H); 7,03 dd (J = 8, 2 Hz, 1H); 7,15-7,38 m (7H); 7,82 d (J = 8 Hz, 1H).

### Beispiel 10

### 6-[[1-(4-Methylphenyl)-2-(2-pyridinyl)mercapto-1H-benzimidazol-6-yl]oxy]hexansäuremethylester

200 mg 6-[[1-(4-Methylphenyl)-2-mercapto-1*H*-benzimidazol-6-yl]oxy]hexansäuremethylester wurden mit 80 µl 2-Chlorpyridin 14 Stunden auf 150°C erhitzt. Nach Zugabe von weiteren 200 µl 2-Chlorpyridin wurde 3 Stunden auf 170°C erhitzt. Nach dem Erkalten wurde durch Säulenchromatographie an Kieselgel gereinigt. Man erhielt 40 mg.
MS (EI): 461 (Molekülionpeak)

### Beispiel 11

### N-(3-Methoxypropyl)-6-[[1-(4-methylphenyl)-2-propylmercapto-1H-benzimidazol-6-yl]oxy]hexanamid

100 mg 6-[[1-(4-Methylphenyl)-2-propylmercapto-1*H*-benzimidazol-6-yl]oxy]hexansäuremethylester wurden gemäß der allgemeinen Arbeitsvorschrift 4 mit 3-Methoxypropylamin umgesetzt. Man erhielt 38 mg.
MS (EI): 483 (Molekülionpeak)

### Beispiel 12

### N-(3-Methoxypropyl)-6-[[1-(4-methylphenyl)-2-propansulfonyl-1H-benzimidazol-6-yl]oxy]hexanamid

100 mg 6-[[1-(4-Methylphenyl)-2-propansulfonyl-1*H*-benzimidazol-6-yl]oxy]hexansäuremethylester wurden gemäß der allgemeinen Arbeitsvorschrift 4 mit 3-Methoxypropylamin umgesetzt. Man erhielt 105 mg.
MS (EI): 515 (Molekülionpeak)

### Beispiel 13

### N-(3-Methoxypropyl)-6-[[1-(4-methylphenyl)-2-benzylmercapto-1H-benzimidazol-6-yl]oxy]hexanamid

100 mg 6-[[2-Benzylmercapto-1-(4-methylphenyl)-1*H*-benzimidazol-6-yl]oxy]hexansäuremethylester wurden gemäß der allgemeinen Arbeitsvorschrift 4 mit 3-Methoxypropylamin umgesetzt. Man erhielt 53 mg.
MS (EI): 531 (Molekülionpeak)

### Beispiel 14

### N-(3-Methoxypropyl)-6-[[1-(4-Methylphenyl)-2(-phenylmethansulfonyl)-1H-benzimidazol-6-yl]oxy]hexanamid

100 mg 6-[[1-(4-Methylphenyl)-2-(phenylmethansulfonyl)-1*H*-benzimidazol-6-yl]oxy]hexansäuremethylester wurden gemäß der allgemeinen Arbeitsvorschrift 4 mit 3-Methoxypropylamin umgesetzt. Man erhielt 22 mg.
MS (EI): 563 (Molekülionpeak)

### Beispiel 15

### 6-[[2-(Morpholin-4-yl)-1-phenyl-1H-benzimidazol-6-yl]oxy]hexansäuremethylester

### a) 6-[[1-Phenyl-2-oxo-2,3-dihydro-1H-benzoimidazol-6-yl]oxy]hexansäuremethylester

7,5 g 6-(3-Phenylamino-4-aminophenyl)oxyhexansäuremethylester wurden mit 8,23 g Harnstoff versetzt und die Mischung 4 Stunden auf 140°C erhitzt. Man versetzte nach dem Erkalten mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde aus Diisopropylether kristallisiert. Man erhielt 4,27 g. Fp. 146,5-148°C **b) 6-[[2-Chlor-1-phenyl-1*H*-benzimidazol-6-yl]oxy]hexansäuremethylester** 3 g 6-[[1-Phenyl-2-oxo-2,3-dihydro-1*H*-benzoimidazol-6-yl]oxy]hexansäuremethylester
wurden mit 12 ml Phosphoroxychlorid versetzt und die Mischung 4 Stunden zum Rückfluss erhitzt. Man rührte nach dem Erkalten in ges. Natriumhydrogencarbonatlösung ein, extrahierte dreimal mit Ethylacetat, trocknete die vereinigten organischen Phasen über Natriumsulfat und engte im Vakuum ein. Der Rückstand wurde durch Säulenchromatographie an Kieselgel gereinigt. Man erhielt 1,28 g.
MS (EI): 372 (Molekülionpeak)

### c) 6-[[2-(Morpholin-4-yl)-1-phenyl-1H-benzimidazol-6-yl]oxy]hexansäuremethylester

657 mg 6-[[2-Chlor-1-phenyl-1*H*-benzimidazol-6-yl]oxy]hexansäuremethylester wurden in 13 ml Morpholin gelöst und die Mischung 6 Stunden bei 120°C gerührt. Man engte im Vakuum weitestgehend ein, versetzte mit Wasser, extrahierte mit Ethylacetat, trocknete die organische Phase über Natriumsulfat und engte im Vakuum ein. Der Rückstand wurde durch Säulenchromatographie an Kieselgel gereinigt. Man erhielt 271 mg.
¹H-NMR (CDCl₃): δ = 1,42-1,54 ppm m (2H); 1,55-1,80 (4H); 2,34 t (J = 7,5 Hz, 2H); 3,20 t (J = 7,5 Hz, 4H); 3,66 s (3H); 3,66 t (J = 7,5 Hz, 4H); 3,92 t (J = 7,5 Hz, 2H); 6,62 d (J = 2 Hz, 1H); 6,82 dd (J = 8, 2 Hz, 1H); 7,45-7,61 m (6H).

### Beispiel 16

### 6-[[2-(Piperidin-1-yl)-1-phenyl-1H-benzimidazol-6-yl]oxy]hexansäuremethylester

100 mg 6-[[2-Chlor-1-phenyl-1*H*-benzimidazol-6-yl]oxy]hexansäuremethylester wurden in 2,5 ml Piperidin gelöst und die Mischung 5 Stunden bei 100°C gerührt. Man engte im Vakuum weitestgehend ein, versetzte mit Wasser, extrahierte mit Ethylacetat, trocknete die organische Phase über Natriumsulfat und engte im Vakuum ein. Der Rückstand wurde durch Säulenchromatographie an Kieselgel gereinigt. Man erhielt 30 mg.
¹H-NMR (CDCl₃): δ = 1,42-1,57 ppm m (8H); 1,63-1,80 m (4H); 2,32 t (J = 7,5 Hz, 2H); 3,10-3,18 m (4H); 3,65 s (3H); 3,90 t (J = 7,5 Hz, 2H); 6,62 d (J = 2 Hz, 1H); 6,79 dd (J = 8, 2 Hz, 1H); 7.40-7.58 m (6H).

### Beispiel 17

### 6-[[1-(4-Methylphenyl)-2-(morpholin-4-yl)-1H-benzimidazol-6-yl]oxy]hexansäuremethylester

### a) 6-[[1-(4-Methylphenyl)-2-oxo-2,3-dihydro-1H-benzoimidazol-6-yl]oxy]hexansäuremethylester

5 g 6-[3-(4-Methylphenyl)-4-aminophenyl]oxyhexansäuremethylester wurden mit 5,28 g Harnstoff versetzt und die Mischung 5 Stunden auf 150°C erhitzt. Man versetzte nach dem Erkalten mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde aus Diisopropylether kristallisiert. Man erhielt 2,54 g.
Fp. 99-100°C

### b) 6-[[2-Chlor-1-(4-methylphenyl)-1H-benzimidazol-6-yl]oxy]hexansäuremethylester

2,5 g 6-[[1-(4-Methylphenyl)-2-oxo-2,3-dihydro-1*H*-benzoimidazol-6-yl]oxy]hexansäuremethylester wurden mit 10 ml Phosphoroxychlorid versetzt und die Mischung 2 Stunden zum Rückfluss erhitzt. Man rührte nach dem Erkalten in ges. Natriumhydrogencarbonatlösung ein, extrahierte dreimal mit Ethylacetat, trocknete die vereinigten organischen Phasen über Natriumsulfat und engte im Vakuum ein. Der Rückstand wurde durch Säulenchromatographie an Kieselgel gereinigt. Man erhielt 1,28 g.
¹H-NMR (CDCl₃): δ = 1,42-1,54 ppm m (2H); 1,60-1,84 m (4H); 2,34 t (J = 7,5 Hz, 2H); 2,50 s (3H); 3,66 s (3H); 3,88 t (J = 7,5 Hz, 2H); 6,58 d (J = 2 Hz, 1H); 6,92 d (J = 8 Hz, 1H); 7,30 d (J = 7,5 Hz, 2H); 7,40 d (J = 7,5 Hz, 2H); 7,60 d (J = 8 Hz, 1H).

### c) 6-[[1-(4-Methylphenyl)-2-(morpholin-4-yl)-1H-benzimidazol-6-yl]oxy]hexansäuremethylester

240 mg 6-[[2-Chlor-1-(4-methylphenyl)-1*H*-benzimidazol-6-yl]oxy]hexansäuremethylester wurden mit 0,2 ml *N,N*-Dimethylformamid und 0,7 ml Morpholin versetzt und das Gemisch 7,5 Stunden bei 110°C gerührt. Man setzte weitere 0,7 ml Morpholin zu und erhitzte während weiterer 4 Stunden bei 110°C. Man versetzte mit Wasser, extrahierte mit Ethylacetat, trocknete die organische Phase über Natriumsulfat und engte im Vakuum ein. Der Rückstand wurde durch Säulenchromatographie an Kieselgel gereinigt. Man erhielt 7 mg.
¹H-NMR (CDCl₃): δ = 1,42-1,53 ppm m (2H); 1,60-1,82 m (4H); 2,34 t (J = 7,5 Hz, 2H); 2,50 s (3H); 3,20 t (J = 7,5 Hz, 4H); 3,68 s (3H); 3,68 t (J = 7,5 Hz, 4H); 3,90 t (J = 7,5 Hz, 2H); 6,60 d (J = 2 Hz, 1H); 6,80 dd (J = 8, 2 Hz, 1H); 7,38 s (4H); 7,48 d (J = 8 Hz, 1H).

### Beispiel 18

### 6-[[1-(4-Methylphenyl)-2-(piperidin-1-yl)-1H-benzimidazol-6-yl]oxy]hexansäuremethylester

240 mg 6-[[2-Chlor-1-(4-methylphenyl)-1*H*-benzimidazol-6-yl]oxy]hexansäuremethylester wurden mit 0,2 ml *N,N*-Dimethylformamid und 0,75 ml Piperidin versetzt und das Gemisch 7,5 Stunden bei 110°C gerührt. Man setzte weitere 0,75 ml Piperidin zu und erhitzte während weiterer 4 Stunden bei 110°C. Man versetzte mit Wasser, extrahierte mit Ethylacetat, trocknete die organische Phase über Natriumsulfat und engte im Vakuum ein. Der Rückstand wurde durch Säulenchromatographie an Kieselgel gereinigt. Man erhielt 16 mg.
¹H-NMR (CDCl₃): δ = 1,40-1,58 ppm m (8H); 1,62-1,82 m (4H); 2,32 t (J = 7,5 Hz, 2H); 2,48 s (3H); 3,14-3,22 m (4H); 3,68 s (3H); 3,88 t (J = 7,5 Hz, 2H); 6,58 d (J = 2 Hz, 1H); 6,78 dd (J = 8, 2 Hz, 1H); 7.36 s (4H); 7.50 d (J = 8 Hz, 1H).

### Beispiel 19

### N-(3-Methoxypropyl)-6-[[2-(morpholin-4-yl)-1-phenyl-1H-benzimidazol-6-yl]oxy]hexanamid

135 mg 6-[[2-(Morpholin-4-yl)-1-phenyl-1*H*-benzimidazol-6-yl]oxy]hexansäuremethylester wurden gemäß der allgemeinen Arbeitsvorschrift 4 mit 3-Methoxypropylamin umgesetzt. Man erhielt 94 mg.
MS (EI): 480 (Molekülionpeak)

### Beispiel 20

### N-(3-Methoxypropyl)-6-[[2-(piperidin-1-yl)-1-phenyl-1H-benzimidazol-6-yl]oxy]hexanamid

100 mg 6-[[2-(Piperidin-1-yl)-1-phenyl-1*H*-benzimidazol-6-yl]oxy]hexansäuremethylester wurden gemäß der allgemeinen Arbeitsvorschrift 4 mit 3-Methoxypropylamin umgesetzt. Man erhielt 47 mg.
¹H-NMR (CDCl₃): δ = 1,40-1,55 ppm m (8H); 1,60-1,80 m (4H); 2,18 t (J = 7,5 Hz, 2H); 3,14-3,24 m (4H); 3,36 s (3H); 3,36-3,42 m (2H); 3,45 t (J = 7,5 Hz, 2H); 3,90 t (J = 7,5 Hz, 2H); 6,00 s (breit) (1H); 6,62 d (J = 2 Hz, 1H); 6,80 dd (J = 8, 2 Hz, 1H); 7,40-7,62 m (6H).

### Beispiel 21

### 6-[[2-Methoxy-1-phenyl-1H-benzimidazol-6-yl]oxy]hexansäuremethylester

200 mg 6-(3-Phenylamino-4-aminophenyl)oxyhexansäuremethylester wurden mit 0,12 ml Tetramethylorthocarbonat und 40 µl Essigsäure versetzt und die Mischung 4 Stunden auf 80°C erhitzt. Man versetzte nach dem Erkalten mit 1 N Natronlauge; extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 104 mg.
Fp. 104-106°C

### Beispiel 22

### 6-[[2-Methoxy-1-phenyl-1H-benzimidazol-6-yl]oxy]hexansäure

56 mg 6-[[2-Methoxy-1-phenyl-1*H*-benzimidazol-6-yl]oxy]hexansäuremethylester wurden gemäß der allgemeinen Arbeitsvorschrift 3 umgesetzt. Man erhielt 33 mg.
Fp. 134-136°C

### Beispiel 23

### 6-[[2-Ethoxy-1-phenyl-1H-benzimidazol-6-yl]oxy]hexansäuremethylester

200 mg 6-(3-Phenylamino-4-aminophenyl)oxyhexansäuremethylester wurden mit 0,19 ml Tetraethylorthocarbonat und 40 µl Essigsäure versetzt und die Mischung 4 Stunden auf 80°C erhitzt. Man versetzte nach dem Erkalten mit 1 N Natronlauge, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 124 mg.
¹H-NMR (CDCl₃): δ = 1,45 ppm t (J = 8 Hz, 3H); 1,45-1,55 m (2H); 1,63-1,85 m (4H); 2,35 t (J = 7,5 Hz, 2H); 3,67 s (3H); 3,95 t (J = 7,5 Hz, 2H); 4,60 t (J = 8 Hz, 2H); 6,72 d (J = 2 Hz, 1H); 6,83 dd (J = 8, 2 Hz, 1H); 7,40-7,60 m (6H).

### Beispiel 24

### 6-[[2-Ethoxy-1-phenyl-1H-benzimidazol-6-yl]oxy]hexansäure

50 mg 6-[[2-Ethoxy-1-phenyl-1*H*-benzimidazol-6-yl]oxy]hexansäuremethylester wurden gemäß der allgemeinen Arbeitsvorschrift 3 umgesetzt. Man erhielt 44 mg.
Fp. 127-130°C

### Beispiel 25

### 6-[[1-Phenyl-2-phenylamino-1H-benzimidazol-6-yl]oxy]hexansäuremethylester

200 mg 6-(3-Phenylamino-4-aminophenyl)oxyhexansäuremethytester wurden in 1 ml 1,2-Dichlorethan gelöst, die Lösung mit 85 µl Phenylisocyaniddichlorid versetzt und die Mischung 6 Stunden auf 65°C erhitzt. Man versetzte nach dem Erkalten mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 55 mg.
MS (EI): 429 (Molekülionpeak)

### Beispiel 26

### 6-[[1-Phenyl-2-phenylamino-1H-benzimidazol-6-yl]oxy]hexansäure

50 mg 6-[[1-Phenyl-2-phenylamino-1*H*-benzimidazol-6-yl]oxy]hexansäuremethylester wurden gemäß der allgemeinen Arbeitsvorschrift 3 umgesetzt. Man erhielt 33 mg.
Fp. 148-149,5°C

### Beispiel 27

### 6-[[1-(4-Methylphenyl)-2-phenylamino-1H-benzimidazol-6-yl]oxy]hexansäuremethylester

250 mg 6-[3-(4-Methylphenyl)-4-aminophenyl]oxyhexansäuremethylester wurden in 1 ml 1,2-Dichlorethan gelöst, die Lösung mit 0,1 ml Phenylisocyaniddichlorid versetzt und die Mischung 8 Stunden auf 65°C erhitzt. Man versetzte nach dem Erkalten mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde aus einem Gemisch aus Ethylacetat und Diethylether kristallisiert. Man erhielt 147 mg.
Fp. 142-143,5°C

### Beispiel 28

### 6-[[1-Phenyl-2-propylamino-1H-benzimidazol-6-yl]oxy]hexansäuremethylester

200 mg 6-(3-Phenylamino-4-aminophenyl)oxyhexansäuremethylester wurden in 2 ml Methanol gelöst, die Lösung mit 90 µl Propylisothiocyanat versetzt und die Mischung 3 Stunden auf 50°C erhitzt. Man versetzte nach dem Erkalten mit ges. Ammoniumchloridlösung, verdünnte mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 140 mg eines Thiohamstoffderivats, die in in 1,5 ml Methanol gelöst wurden. Zu dieser Lösung gab man 0,16 ml Jodmethan und erhitzte 4 Stunden zum Rückfluss. Man versetzte nach dem Erkalten mit 1 N wässriger Salzsäure, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 72 mg. MS (EI): 395 (Molekülionpeak)

### Beispiel 29

### 6-[[1-Phenyl-2-propylamino-1H-benzimidazol-6-yl]oxy]hexansäure

30 mg 6-[[1-Phenyl-2-propylamino-1*H*-benzimidazol-6-yl]oxy]hexansäuremethylester wurden gemäß der allgemeinen Arbeitsvorschrift 3 umgesetzt. Man erhielt 17 mg.
Fp. 123-125°C

### Beispiel 30

### 6-[[2-(N-Methyl-N-propyl)amino-1-phenyl-1H-benzimidazol-6-yl]oxy]hexansäuremethylester

20 mg 6-[[1-Phenyl-2-propylamino-1*H*-benzimidazol-6-yl]oxy]hexansäuremethylester wurden in 1 ml Tetrahydrofuran gelöst. Man setzte 10 mg Natriumhydrid (80%ig in Mineralöl) zu, rührte 30 Minuten bei 20°C, setzte dann bei 0°C 50 µl Jodmethan zu und rührte 1 Stunde bei 0°C. Man versetzte mit ges. Ammoniumchloridlösung, verdünnte mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 6 mg.
¹H-NMR (CDCl₃): δ = 0,70 ppm t (J = 7,5 Hz, 3H); 1,38-1,53 m (4H); 1,63-1,83 m (4H); 2,31 t (J = 7,5 Hz, 2H); 2,82 s (3H); 3,02 t (J = 7,5 Hz; 2H); 3,67 s (3H); 3,88 t (J = 7,5 Hz, 2H); 6,53 d (J = 2 Hz, 1H); 6,77 dd (J = 8, 2 Hz, 1H); 7,40-7,60 m (6H).

### Beispiel 31

### 6-[[1-(4-Methylphenyl)-2-phenyloxy-1H-benzimidazol-6-yl]oxy]hexansäure

150 mg 6-[[2-Chlor-1-(4-methylphenyl)-1*H*-benzimidazol-6-yl]oxy]hexansäuremethylester wurden in 2 ml Dimethylformamid gelöst, mit 54 mg Kaliumcarbonat und 37 mg Phenol versetzt und das Gemisch für 7 Tage bei 150°C gerührt. Nach dem Erkalten versetzte man mit ges. Ammoniumchloridlösung, verdünnte mit Wasser, extrahierte dreimal mit Ethylacetat, wusch die vereinigten organischen Phasen mit ges. Natriumchloridlösung, trocknete über Natriumsulfat und engte im Vakuum ein. Der Rückstand wurde durch Säulenchromatographie an Kieselgel gereinigt. Man erhielt 78 mg.
MS (EI): 430 (Molekülionpeak)

### Beispiel 32

### 6-[[1-(4-Methylphenyl)-2-phenylmercapto-1H-benzimidazol-6-yl]oxy]hexansäuremethylester

150 mg 6-[[2-Chlor-1-(4-methylphenyl)-1*H*-benzimidazol-6-yl]oxy]hexansäuremethylester wurden in 2 ml Dimethylformamid gelöst, mit 54 mg Kaliumcarbonat und 40µl Thiophenol versetzt und das Gemisch für 5 Stunden bei 140°C gerührt. Nach dem Erkalten versetzte man mit ges. Ammoniumchloridlösung, verdünnte mit Wasser, extrahierte dreimal mit Ethylacetat, wusch die vereinigten organischen Phasen mit ges. Natriumchloridlösung, trocknete über Natriumsulfat und engte im Vakuum ein. Man erhielt 158 mg.
¹H-NMR (CDCl₃): δ = 1,40-1.52 ppm m (2H); 1,60-1,82 m (6H); 2,32 t (J = 7,5 Hz, 2H); 2,45 s (3H); 3,65 s (3H); 3,91 t (J = 7,5 Hz, 2H); 6,52 d (J = 2 Hz, 1H); 6,90 dd (J = 8, 2 Hz, 1H); 7,10-7,44 m (9H); 7,68 d (J = 8 Hz, 1H).

### Beispiel 33

### 6-[[1-(4-Methylphenyl)-2-(phenylsulfinyl)-1H-benzimidazol-6-yl]oxy]hexansäuremethylester

### Beispiel 34

### 6-[[1-(4-Methylphenyl)-2-(phenylsulfonyl)-1H-benzimidazol-6-yl]oxy]hexansäuremethylester

180 mg 6-[[1-(4-Methylphenyl)-2-phenylmercapto-1*H*-benzimidazol-6-yl]oxy]hexansäuremethylester wurden in 4 ml Dichlormethan gelöst, mit 112 mg *m*-Chlorperbenzoesäure (ca. 55%ig) versetzt und die Mischung 5 Stunden bei 20°C gerührt. Man versetzte mit Natriumdisulfitlösung, extrahierte zweimal mit Dichlormethan, wusch die vereinigten organischen Phasen mit ges. Natriumhydrogencarbonatlösung, Wasser und ges. Natriumchloridlösung, trocknete über Natriumsulfat und engte im Vakuum ein. Der Rückstand wurde durch Säulenchromatographie an Kieselgel gereinigt. Man erhielt 56 mg 6-[[1-(4-Methylphenyl)-2-(phenylsulfinyl)-1*H*-benzimidazol-6-yl]oxy]hexansäuremethylester,
¹H-NMR (CDCl₃): δ = 1,40-1.50 ppm m (2H); 1,60-1,80 m (6H); 2,30 t (J = 7,5 Hz, 2H); 2,48 s (3H); 3,64 s (3H); 3,85 t (J = 7,5 Hz, 2H); 6,48 d (J = 2 Hz, 1H); 6,95 dd (J = 8, 2 Hz, 1H); 7,02-7,12 m (2H); 7,20-7,45 m (7H); 7,78 d (J = 8 Hz, 1H).
und 39 mg 6-[[1-(4-Methylphenyl)-2-(phenylsulfonyl)-1*H*-benzimidazol-6-yl]oxy]hexansäuremethylester,
¹H-NMR (CDCl₃): δ = 1,38-1.52 ppm m (2H); 1,60-1,80 m (6H); 2,30 t (J = 7,5 Hz, 2H); 2,50 s (3H); 3,64 s (3H); 3,83 t (J = 7,5 Hz, 2H); 6,38 d (J = 2 Hz, 1H); 6,98 dd (J = 8, 2 Hz, 1H); 7,10 d (J = 8 Hz, 2H); 7,27 d (J = 8 Hz, 2H); 7,43 dd (J = 8, 8 Hz, 2H); 7,58 m (1H); 7,72 d (J = 8, 8 Hz, 2H); 7,78 d (J = 8 Hz, 1H).

### Beispiel 35

### 6-[[1-Phenyl-2-propylmercapto-1H-benzimidazol-6-yl)oxy]hexansäure

109 mg 6-[[1-Phenyl-2-propylmercapto-1*H*-benzimidazol-6-yl]oxy]hexansäuremethylester wurden gemäß der allgemeinen Arbeitsvorschrift 3 mit Lithiumhydroxid umgesetzt. Man erhielt 104 mg.
MS (EI): 398 (Molekülionpeak)

### Beispiel 36

### 6-[[1-Phenyl-2-propansulfonyl-1H-benzimidazol-6-yl]oxy]hexansäure

228 mg 6-[[1-Phenyl-2-propansulfonyl-1*H*-benzimidazol-6-yl]oxy]hexansäuremethylester wurden gemäß der allgemeinen Arbeitsvorschrift 3 mit Lithiumhydroxid umgesetzt. Man erhielt 152 mg.
¹H-NMR (CDCl₃): δ = 1,03 ppm t (J = 8 Hz, 3H); 1,45-1,60 m (2H); 1,62-1,95 m (6H); 2,36 t (J = 7,5 Hz, 2H); 3,45-3,56 m (2H); 3,90 t (J = 7,5 Hz, 2H); 6,51 d (J = 2 Hz, 1H); 7,02 dd (J = 8, 2 Hz, 1H); 7,48-7,66 m (5H); 7,77 d (J = 8 Hz, 1H).

### Beispiel 37

### 6-[(2-Benzylmercapto-1-phenyl-1H-benzimidazol-6-yl)oxy]hexansäuremethylester

490 mg 6-[(2-Mercapto-1-phenyl-1*H*-benzimidazol-6-yl)oxy]hexansäuremethylester wurden in 7 ml *N,N*-Dimethylformamid gelöst, mit 0,19 ml Benzylbromid, 278 mg Kaliumhydrogencarbonat und 49 mg Dicyclohexano-18-krone-6 versetzt und die Mischung 15 Stunden bei 20°C gerührt. Man filtrierte und engte das Filtrat im Vakuum ein. Der Rückstand wurde durch Säulenchromatographie an Kieselgel gereinigt. Man erhielt 548 mg.
¹H-NMR (CDCl₃): δ = 1,43-1,55 ppm m (2H); 1,62-1,83 m (6H); 2,32 t (J = 7,5 Hz, 2H); 3,68 s (3H); 3,90 t (J = 7,5 Hz, 2H); 4,58 s (2H); 6,69 d (J = 2 Hz, 1H); 6,90 dd (J = 8, 2 Hz, 1H); 7,20-7,59 m (10H); 7,69 d (J = 8 Hz, 1H).

### Beispiel 38

### 6-[[1-Phenyl-2-(phenylmethansulfonyl)-1H-benzimidazol-6-yl]oxy]hexansäuremethylester

300 mg 6-[(2-Benzylmercapto-1-phenyl-1*H*-benzimidazol-6-yl)oxy]hexansäuremethylester wurden in 6 ml Dichlormethan gelöst, mit 468 mg *m*-Chlorperbenzoesäure (ca. 60%ig) versetzt und die Mischung 48 Stunden bei 20°C gerührt. Man versetzte mit Natriumdisulfitlösung, extrahierte zweimal mit Dichlormethan, wusch die vereinigten organischen Phasen mit ges. Natriumhydrogencarbonatlösung, Wasser und ges. Natriumchloridlösung, trocknete über Natriumsulfat und engte im Vakuum ein. Der Rückstand wurde durch Säulenchromatographie an Kieselgel gereinigt. Man erhielt 179 mg.
¹H-NMR (CDCl₃): δ = 1,42-1,55 ppm m (2H); 1,58-1,84 m (4H); 2,33 t (J = 7,5 Hz, 2H); 3,66 s (3H); 3,85 t (J = 7,5 Hz, 2H); 4,75 s (2H); 6,38 d (J = 2 Hz, 1H); 6,96 dd (J = 8, 2 Hz, 2H); 7,04 dd (J = 8, 2 Hz, 1H); 7,20-7,50 m (8H); 7,82 d (J = 8 Hz, 1H).

### Beispiel 39

### 6-[(2-Benzylmercapto-1-phenyl-1H-benzimidazol-6-yl)oxy]hexansäure

235 mg 6-[(2-Benzylmercapto-1-phenyl-1*H*-benzimidazol-6-yl)oxy]hexansäuremethylester wurden gemäß der allgemeinen Arbeitsvorschrift 3 mit Lithiumhydroxid umgesetzt. Man erhielt 179 mg.
MS (EI): 446 (Molekülionpeak)

### Beispiel 40

### 6-[[1-Phenyl-2-(phenylmethansulfonyl)-1H-benzimidazol-6-yl]oxy]hexansäure

175 mg 6-[[1-Phenyl-2-(phenylmethansulfonyl)-1*H*-benzimidazol-6-yl]oxy]hexansäuremethylester wurden gemäß der allgemeinen Arbeitsvorschrift 3 mit Lithiumhydroxid umgesetzt. Man erhielt 143 mg.
¹H-NMR (CDCl₃): δ = 1,42-1,88 m (6H); 2,38 t (J = 7,5 Hz, 2H); 3,85 t (J = 7,5 Hz, 2H); 4,75 s (2H); 6,38 d (J = 2 Hz, 1H); 6,97 dd (J = 8, 2 Hz, 2H); 7,06 dd (J = 8, 2 Hz, 1H); 7,18-7,50 m (8H); 7,83 d (J = 8 Hz, 1H).

### Beispiel 41

### 6-[[1-(4-Methylphenyl)-2-propylmercapto-1H-benzimidazol-6-yl]oxy]hexansäure

128 mg 6-[[1-(4-Methylphenyl)-2-propylmercapto-1*H*-benzimidazol-6-yl]oxy]hexansäuremethylester wurden gemäß der allgemeinen Arbeitsvorschrift 3 mit Lithiumhydroxid umgesetzt. Man erhielt 98 mg.
MS (EI): 412 (Molekülionpeak)

### Beispiel 42

### 6-[[1-(4-Methylphenyl)-2-propansulfonyl-1H-benzimidazol-6-yl]oxy]hexansäure

147 mg 6-[[1-(4-Methylphenyl)-2-propansulfonyl-1*H*-benzimidazol-6-yl]oxy]hexansäuremethylester wurden gemäß der allgemeinen Arbeitsvorschrift 3 mit Lithiumhydroxid umgesetzt. Man erhielt 71 mg.
MS (EI): 444 (Molekülionpeak)

### Beispiel 43

### 6-[[2-Benzylmercapto-1-(4-methylphenyl)-1H-benzimidazol-6-yl]oxy]hexansäure

130 mg 6-[[2-Benzylmercapto-1-(4-methylphenyl)-1*H*-benzimidazol-6-yl]oxy]hexansäuremethylester wurden gemäß der allgemeinen Arbeitsvorschrift 3 mit Lithiumhydroxid umgesetzt. Man erhielt 120 mg.
MS (EI): 460 (Molekülionpeak)

### Beispiel 44

### 6-[[1-(4-Methylphenyl)-2-(phenylmethansulfonyl)-1H-benzimidazol-6-yl]oxy]hexansäure

293 mg 6-[[1-(4-Methylphenyl)-2-(phenylmethansulfonyl)-1*H*-benzimidazol-6-yl]oxy]hexansäuremethylester wurden gemäß der allgemeinen Arbeitsvorschrift 3 mit Lithiumhydroxid umgesetzt. Man erhielt 114 mg.
MS (EI): 492 (Molekülionpeak)

### Beispiel 45

### Hemmung der Mikroglia-Aktivierung

Zur *in vitro* Darstellung von Aβ-aktivierten Mikroglia werden primäre Ratten-Mikroglia mit synthetischem Aβ Peptid inkubiert:

Zur Simulierung von Aβ-Ablagerungen wird synthetisches Aβ Peptid auf 96-Loch Gewebekulturplatten eingetrocknet. Dazu wird eine Peptidstammlösung von 2mg/ml H₂O 1:50 in H₂O verdünnt. Zur Beschichtung der 96-Loch Platten werden 30 µL dieser verdünnten Peptidlösung/Loch eingesetzt und über Nacht bei Raumtemperatur eingetrocknet.

Primäre Rattenmikroglia werden von gemischten Gliakulturen geerntet, die von P3 Rattenhirnen gewonnen wurden. Zur Herstellung von gemischten Gliakulturen werden die Hirne von 3 Tage alten Ratten entnommen und von Hirnhäuten befreit. Die Zellvereinzelung wird durch Trypsinisierung erreicht (0,25 % Trypsinlösung, 15 Minuten, 37°C). Nach Abtrennung von nichtverdauten Gewebefragmenten mit Hilfe eines 40µm Nylonnetzes werden die isolierten Zellen abzentrifugiert (800 Umdrehungen/10 Minuten). Das Zellpellet wird in Kulturmedium resuspendiert und in 100 ml Gewebekulturflaschen überführt. (1 Hirn/ Gewebekulturflasche). Die Zellen werden über einen Zeitraum von 5 bis 7 Tagen in Dulbeccos modified Eagle Medium (DMEM, mit Glutamin), supplementiert mit Penicillin (50 U/ml), Streptomycin (40 µg/ml) und 10 % (v/v) fötalem Kälber Serum (FCS) bei 37°C und 5 % CO₂ kultiviert. Während dieser Inkubation wird ein adhäsiver Zellrasen gebildet, der hauptsächlich aus Astrozyten besteht. Mikroglia proliferieren als nicht- oder schwachadhesive Zellen auf diesem und werden über Schüttelinkubation abgeerntet (420 Umdrehungen/Minute, 1 Stunde).

Zur Aktivierung der Mikroglia durch Aβ-Peptid werden 2,5 mal 10⁴ Mikroglia/Loch auf Aβ-beschichtete Gewebekulturplatten ausgesät und über einen Zeitraum von 7 Tagen in DMEM (mit Glutamin), supplementiert mit Penicillin (50 U/ml), Streptomycin (40 µg/ml) und 10 % (v/v) fötalem Kälber Serum (FCS) bei 37°C und 5 % CO₂ inkubiert. Am Tag 5 wird eine erfindungsgemäße Verbindung in verschiedenen Konzentrationen (0,1, 0,3, leerzeichen 1,3 und 10 µM) zugegeben.

Zur Quantifizierung der Mikroglia-Reaktivität wird am Kultivierungstag 7 die metabolische Aktivität über die Reduktion von MTS (3-(4,5-Dimethylthiazol-2-yl)-5-(3-carboxymethoxyphenyl)-2-(sulfophenyl)-2H-tetrazolium), Owen's Reagenz, Baltrop, J.A. et al. *Bioorg*. & *Med. Chem. Lett*., 1, 6111 (1991)) gemessen. Die Prozent Inhibition bezieht sich auf eine nur mit DMSO behandelte Kontrolle. Die erfindungsgemäßen Verbindungen inhibieren die Mikroglia-Aktivierung. Die Verbindung des Beispiels 9 (6-[[1-(4-Methylphenyl)-2-(phenylmethansulfonyl)-1*H*-benzimidazol-6-yl]oxy]hexansäuremethylester zeigt eine Hemmung von
IC₅₀ = 0,46 µM und die Verbindung des Beispiels 15 6-[[2-(Morpholin-4-yl)-1-phenyl-1*H*-benzimidazol-6-yl]oxy]hexansäuremethylester eine Hemmung von IC₅₀ = 0,87 µM.

## Patentansprüche

1. Benzimidazolderivate mit der allgemeinen Formel I 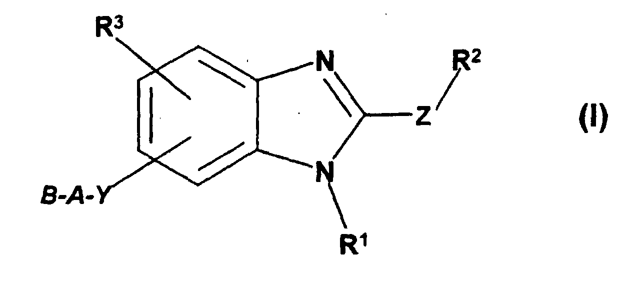 worin
***R***^{***1***} für eine Arylgruppe oder eine fünf- oder sechsgliedrige Heteroarylgruppe mit einem oder zwei Heteroatomen, ausgewählt aus der Gruppe, umfassend N, S und O, steht,
wobei die Aryl- oder Heteroarylgruppe mit bis zu drei Resten unabhängig voneinander substituiert sein kann, ausgewählt aus der Gruppe, umfassend F, Cl, Br, C(NH)NH₂, C(NH)NH***R***^{***4***},
C(NH)N***R***^{***4***}***R***^{***4'***}, C(N***R***^{***4***})NH₂, C(N***R***^{***4***})NH***R***^{***4'***}, C(N***R***^{***4***})N***R***^{***4***}***R***^{***4'***}, ***X***-OH, ***X***-O***R***^{***4***}, ***X***-OCO***R***^{***4***}, ***X***-OCONH***R***^{***4***},
***X***-CO***R***^{***4***}, ***X***-C(NOH)***R***^{***4***}, ***X***-CN, ***X***-COOH, ***X***-COO***R***^{***4***}, ***X***-CONH₂,
***X***-CON***R***^{***4***}***R***^{***4'***}, ***X***-CONH***R***^{***4***},
***X***-CONHOH, ***X***-S***R***^{***4***}, ***X***-SO***R***^{***4***}, ***X***-SO₂***R***^{***4***}, SO₂NH₂, SO₂NH***R***^{***4***},
SO₂N***R***^{***4***}***R***^{***4'***}, NO₂, ***X***-NH₂,
***X***-NH***R***^{***4***}, ***X***-N***R***^{***4***}***R***^{***4'***}, ***X***-NHSO₂***R***^{***4***}, ***X***-N***R***^{***4***}SO₂***R***^{***4'***}, ***X***-NHCO***R***^{***4***}, ***X***-NHCOO***R***^{***4***}, ***X***-NHCONH***R***^{***4***} und ***R***^{***4***},
wobei ***X*** eine Bindung, CH₂, (CH₂)₂ oder CH(CH₃) ist und
wobei ferner ***R***^{***4***} und ***R***^{***4'***} unabhängig voneinander die weiter unten angegebenen Bedeutungen haben, und
wobei zwei Substituenten an ***R***^{***1***} jeweils so miteinander verknüpft sein können, dass sie gemeinsam eine Methandiylbisoxy-, Ethan-1,2-diylbisoxy-, Propan-1,3-diyl- oder Butan-1,4-diylgruppe bilden, wenn die Substituenten an ***R***^{***1***} zueinander orthoständig sind,
***Z*** für eine Gruppierung steht, ausgewählt aus der Gruppe, umfassend **N**H, N***R***^{***2'***}, O, S, SO und SO₂, worin ***R***^{***2'***} die nachstehend angegebene Bedeutung hat,
***R***^{***2***} und ***R***^{***2'***} für jeweils einen Rest stehen, ausgewählt aus der Gruppe, umfassend:
C₁₋₄-Perfluoralkyl, C₁₋₆-Alkyl, (C₀₋₃-Alkandiyl-C₃₋₇ Cycloalkyl), (C₀₋₃-Alkandiyl-Aryl) und (C₀₋₃-Alkandiyl-Heteroaryl),
wobei die Heteroarylgruppe fünf- oder sechsgliedrig ist und ein oder zwei Heteroatome enthält, ausgewählt aus der Gruppe, umfassend N, S und O, und wobei ***R***^{***2***} und ***R***^{***2'***} unabhängig voneinander gewählt sind,
wobei die Aryl- und/oder Heteroarylgruppe jeweils mit bis zu zwei Resten substituiert sein kann, ausgewählt aus der Gruppe, umfassend F, Cl, Br, CH₃, C₂H₅, OH, OCH₃, OC₂H₅, NO₂, CF₃, C₂F₅ und SO₂NH₂, und/oder eine anellierte Methandiylbisoxy- und/oder Ethan-1,2-diylbisoxygruppe tragen kann,
wobei ein Ringglied im Cycloalkylring ferner Ring-**N** oder Ring-O sein kann, wenn der Cycloalkylring fünfgliedrig ist, oder ein oder zwei Ringglieder im Cycloalkylring jeweils Ring-**N**- und/oder Ring-O-Atome sein können, wenn der Cycloalkylring sechs- oder siebengliedrig ist,
wobei ferner die Ring-N-Atome mit C₁₋₃-Alkyl oder C₁₋₃-Alkanoyl substituiert sein können,
oder wenn ***Z*** N***R***^{***2'***} ist, ***R***^{***2***} und ***R***^{***2'***} gemeinsam mit ***Z*** einen fünf- bis siebengliedrigen heterocyclischen Ring bilden können, wobei der heterocyclische Ring gesättigt sein kann, der ein weiteres N-, O- oder S-Atom enthalten kann und der mit einem Rest substituiert sein kann, ausgewählt aus der Gruppe, umfassend C₁₋₄-Alkyl, (C₀₋₃-Alkandiyl-C₁₋₃-Alkoxy), C₁₋₄-Alkanoyl, C₁₋₄-Alkoxycarbonyl, Aminocarbonyl und Phenyl,
***R***^{***3***} für einen oder zwei unabhängig voneinander wählbare Substituenten steht, ausgewählt aus der Gruppe, umfassend: Wasserstoff, F, Cl, Br, OH, O***R***^{***4***}, OCO***R***^{***4***}, OCONH***R***^{***4***}, CO***R***^{***4***}, CN, COOH, COO***R***^{***4***}, CONH₂, CONH***R***^{***4***}, CON***R***^{***4***}***R***^{***4'***}, CONHOH, CONHO***R***^{***4***}, S***R***^{***4***}, SO***R***^{***4***}, SO₂***R***^{***4***}, SO₂NH₂, SO₂NH***R***^{***4***}, SO₂N***R***^{***4***}***R***^{***4'***}, NO₂, NH₂, NH***R***^{***4***}, N***R***^{***4***}***R***^{***4'***}, NHSO₂***R***^{***4***}, N***R***^{***4***}SO₂***R***^{***4'***}, NHSO₂***R***^{***6***}, N***R***^{***4***}SO₂***R***^{***6***}, NHCO***R***^{***4***}, NHCOO***R***^{***4***}, NHCONH***R***^{***4***} und ***R***^{***4***},
wobei die Reste ***R***^{***4***}, ***R***^{***4'***} und ***R***^{***6***} unabhängig voneinander gewählt werden und die weiter unten angegebenen Bedeutungen haben,
***A*** für eine Gruppe steht, ausgewählt aus der Gruppe, umfassend C₁₋₁₀-Alkandiyl, C₂₋₁₀-Alkendiyl, C₂₋₁₀-Alkindiyl und (C₀₋₃-Alkandiyl-C₃₋₇-Cycloalkandiyl-C₀₋₃-Alkandiyl),
wobei ein Ringglied im Cycloalkylring Ring-**N** oder Ring-O sein kann, wenn der Cycloalkylring fünfgliedrig ist, oder ein oder zwei Ringglieder im Cycloalkylring jeweils Ring-**N**- und/oder Ring-O-Atome sein können, wenn der Cycloalkylring sechs- oder siebengliedrig ist, wobei die Ring-**N**-Atome mit mindestens einem Rest, ausgewählt aus der Gruppe, umfassend
C₁₋₃-Alkyl- und C₁₋₃-Alkanoyl-Reste, substituiert sein können,
wobei in den aliphatischen Ketten der C₁₋₁₀-Alkandiyl-, C₂₋₁₀-Alkendiyl-, C₂₋₁₀-Alkindiyl- und (C₀₋₃-Alkandiyl-C₃₋₇-Cycloalkandiyl-C₀₋₃-Alkandiyl)-Gruppen ferner ein C-Atom gegen O, NH, N-C₁₋₃-Alkyl oder N-C₁₋₃-Alkanoyl ausgetauscht sein kann und
wobei mindestens eine der Alkyl- und Cycloalkylgruppen mit einem Rest, ausgewählt aus der Gruppe, umfassend =O, OH, O-C₁₋₃-Alkyl, NH₂, NH-C₁₋₃-Alkyl, NH-C₁₋₃-Alkanoyl, N(C₁₋₃-Alkyl)₂ und N(C₁₋₃-Alkyl)(C₁₋₃-Alkanoyl), substituiert sein kann,
***B*** für einen Rest steht, ausgewählt aus der Gruppe, umfassend COOH, COO***R***^{***5***}, CONH₂, CONHNH₂, CONH***R***^{***5***}, CON***R***^{***5***}***R***^{***5'***}, CONHOH, CONHO***R***^{***5***} und Tetrazolyl,
wobei ***B*** an ein C-Atom der Gruppe ***A*** gebunden ist,
wobei die Reste ***R***^{***5***} und ***R***^{***5'***} unabhängig voneinander gewählt werden und die weiter unten angegebenen Bedeutungen haben,
***Y*** für O steht,
***R***^{***4***} und ***R***^{***4'***} jeweils für einen Rest stehen, ausgewählt aus der Gruppe, umfassend CF₃, C₂F₅, C₁₋₄-Alkyl, C₂₋₄-Alkenyl, C₂₋₃-Alkinyl und (C₀₋₃-Alkandiyl- C₃₋₇-Cyclo-alkyl),
wobei ein Ringglied im Cycloalkylring Ring-N oder Ring-O sein kann, wenn der Cycloalkylring fünfgliedrig ist, oder dass ein oder zwei Ringglieder im Cycloalkylring jeweils Ring-N- und/oder Ring-O-Atome sein können, wenn der Cycloalkylring sechs- oder siebengliedrig ist, wobei die Ring-N-Atome mit C₁₋₃-Alkyl oder C₁₋₃-Alkanoyl substituiert sein können,
***R***^{***5***} und ***R***^{***5'***} jeweils für einen Rest stehen, ausgewählt aus der Gruppe, umfassend
C₁₋₆-Alkyl, C₂₋₆-Alkenyl, C₂₋₆-Alkinyl, wobei ein C-Atom in mindestens einem der Reste ***R***^{***5***} und ***R***^{***5'***} gegen O, S, SO, SO₂, NH, N-C₁₋₃-Alkyl oder N-C₁₋₃-Alkanoyl ausgetauscht sein kann,
ferner (C₀₋₃-Alkandiyl-C₃₋₇-Cycloalkyl), wobei ein Ringglied im Cycloalkylring Ring-N oder Ring-O sein kann, wenn der Cycloalkylring fünfgliedrig ist, oder dass ein oder zwei Ringglieder im Cycloalkylring jeweils ein oder zwei Ring-N- und/oder Ring-O-Atome sein können, wenn der Cycloalkylring sechs- oder siebengliedrig ist, wobei die Ring-**N**-Atome mit mindestens einem Rest substituiert sein können, ausgewählt aus der Gruppe, umfassend C₁₋₃-Alkyl-Reste und C₁₋₃-Alkanoyl-Reste,
ferner (C₀₋₃-Alkandiyl-Aryl) und (C₀₋₃-Alkandiyl-Heteroaryl), wobei die Heteroarylgruppe fünf- oder sechsgliedrig sein kann und ein oder zwei Heteroatome enthalten kann, ausgewählt aus der Gruppe, umfassend **N**, S und O,
wobei ferner mindestens einer der Alkyl- und Cycloalkylreste von ***R***^{***5***} und ***R***^{***5'***} mit bis zu zwei Resten substituiert sein kann, ausgewählt aus der Gruppe, umfassend CF₃, C₂F₅, OH, O-C₁₋₃-Alkyl, NH₂, NH-C₁₋₃-Alkyl, NH-C₁₋₃-Alkanoyl, N(C₁₋₃-Alkyl)₂, N(C₁₋₃-Alkyl)(C₁₋₃-Alkanoyl), COOH, CONH₂ und COO-C₁₋₃-Alkyl, und dass mindestens einer der Aryl- und Heteroarylreste von ***R***^{***5***} und ***R***^{***5'***} mit bis zu zwei Resten substituiert sein kann, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, und/oder dass mindestens einer der Alkyl- Cycloalkyl-, Aryl- und/oder Heteroarylreste von ***R***^{***5***} und ***R***^{***5'***} eine anellierte Methandiylbisoxy- oder Ethan-1,2-diylbisoxygruppe tragen kann,
oder wobei ***R***^{***5***} und ***R***^{***5'***} gemeinsam mit dem Amid-N-Atom von ***B*** einen fünf- bis siebengliedrigen, gesättigten oder ungesättigten heterocyclischen Ring bilden können, der ein weiteres N- oder O- oder S-Atom enthalten kann und der substituiert sein kann mit C₁₋₄-Alkyl, (C₀₋₂-Alkandiyl-C₁₋₄-Alkoxy), C₁₋₄-Alkoxycarbonyl, Aminocarbonyl oder Aryl,
***R***^{***6***} für einen Rest steht, ausgewählt aus der Gruppe, umfassend (C₀₋₃-Alkandiyl-Aryl) und (C₀₋₃-Alkandiyl-Heteroaryl), wobei die Heteroarylgruppe fünf- oder sechsgliedrig ist und ein oder zwei Heteroatome enthält, ausgewählt aus der Gruppe, umfassend N, S und O, und
wobei mindestens eine der Aryl- und Heteroarylgruppen mit jeweils bis zu zwei Resten substituiert sein kann, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, oder dass mindestens eine der Aryl- oder Heteroarylgruppen auch eine anellierte Methandiylbisoxy- oder Ethan-1,2-diylbisoxygruppe tragen kann.

2. Benzimidazolderivate nach Anspruch 1, **dadurch gekennzeichnet, dass *R***^{***1***} für Phenyl steht und mit bis zu zwei Resten unabhängig voneinander substituiert sein kann, ausgewählt aus der Gruppe, umfassend F, Cl, Br, C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4'***}, C(N***R***^{***4***})N***R***^{***4***}***R***^{***4'***}, OH, O***R***^{***4***}, OCO***R***^{***4***}, OCONH***R***^{***4***}, CO***R***^{***4***}, C(NOH)***R***^{***4***}, CN, COOH, COO***R***^{***4***}, CONH₂, CON***R***^{***4***}***R***^{***4'***}, CONH***R***^{***4***}, CONHOH, S***R***^{***4***}, SO***R***^{***4***}, SO₂***R***^{***4***}, SO₂NH₂, SO₂NH***R***^{***4***},SO₂N***R***^{***4***}***R***^{***4'***}, NO₂, NH₂, NH***R***^{***4***}, N***R***^{***4***}***R***^{***4'***}, NHCONH***R***^{***4***} und ***R***^{***4***}.

3. Benzimidazolderivate nach Anspruch 1, **dadurch gekennzeichnet, dass *R***^{***3***} ein Rest ist, ausgewählt aus der Gruppe, umfassend Wasserstoff, F, Cl, Br, CH₃, C₂H₅, CF₃, C₂F₅, OH, O***R***^{***4***}, NHSO₂***R***^{***6***} und NHCO***R***^{***4***}.

4. Benzimidazolderivate nach Anspruch 1, **dadurch gekennzeichnet, dass *B*** für einen Rest steht, ausgewählt aus der Gruppe, umfassend COOH, COO***R***^{***5***}, CONH₂, CONH***R***^{***5***} und CON***R***^{***5***}***NR***^{***5'***}.

5. Benzimidazolderivate nach Anspruch 1, **dadurch gekennzeichnet, dass *B-A-Y*** in 6-Position des Benzimidazols steht.

6. Benzimidazolderivate nach Anspruch 1, **dadurch gekennzeichnet, dass *R***^{***6***} für eine Phenyl- oder Heteroarylguppe steht, wobei die Heteroarylgruppe fünf- oder sechsgliedrig ist und ein oder zwei Heteroatome enthält, ausgewählt aus der Gruppe, umfassend N, S und O.

7. Benzimidazole der allgemeinen Formel I nach Anspruch leerzeichen, worin
***R***^{***1***} eine Phenylgruppe ist, die mit bis zu zwei Resten unabhängig voneinander substituiert sein kann, ausgewählt aus der Gruppe, umfassend:
F, Cl, Br,
C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4'***}, C(N***R***^{***4***})N***R***^{***4***}***R***^{***4'***},
OH, O***R***^{***4***}, OCO***R***^{***4***}, OCONH***R***^{***4***},
CO***R***^{***4***}, C(NOH)***R***^{***4***},
CN, COOH, COO***R***^{***4***}, CONH₂, CON***R***^{***4***}***R***^{***4'***}, CONH***R***^{***4***}, CONHOH,
S***R***^{***4***}, SO***R***^{***4***}, SO₂***R***^{***4***},
SO₂NH₂, SO₂NH***R***^{***4***}, SO₂N***R***^{***4***}***R***^{***4'***},
NO₂, NH₂, NH***R***^{***4***}, N***R***^{***4***}***R***^{***4'***}, NHCONH***R***^{***4***} und
***R***^{***4***},
wobei die Reste ***R***^{***4***} und ***R***^{***4'***} gemäß nachstehend angegebenen Bedeutungen unabhängig voneinander gewählt werden und
wobei zwei Substituenten an ***R***^{***1***} so miteinander verknüpft sein können, dass sie gemeinsam eine Methandiylbisoxy-, Ethan-1,2-diylbisoxy-, Propan-1,3-diyl- oder Butan-1,4-diylgruppe bilden, wenn sie zueinander orthoständig sind,
***Z*** *NH, NR*^{*2'*}, O, *SO oder* SO₂,
***R***^{***2***} und ***R***^{***2'***} unabhängig voneinander jeweils ein Rest, ausgewählt aus der Gruppe, umfassend:
C₁₋₄-Perfluoralkyl, C₁₋₆-Alkyl, (C₀₋₃-Alkandiyl-Aryl) und (C₀₋₃-Alkandiyl-Heteroaryl),
wobei die Heteroarylgruppe fünf- oder sechsgliedrig ist und ein oder zwei Heteroatome enthält, ausgewählt aus der Gruppe, umfassend N, S und O, und
wobei die Aryl- und Heteroarylgruppe jeweils mit bis zu zwei Resten, ausgewählt aus der Gruppe, umfassend F, Cl, Br, CH₃, C₂H₅, OH, OCH₃, OC₂H₅, NO₂, CF₃, C₂F₅ und SO₂NH₂, substituiert sein können
oder wenn ***Z*** N***R***^{***2'***} ist, ***R***^{***2***} und ***R***^{***2'***} gemeinsam mit ***Z*** einen fünf- bis siebengliedrigen heterocyclischen Ring bilden, wobei ferner der heterocyclische Ring ein zusätzliches O- oder S-Atom enthalten und optional substituiert sein kann mit einem Rest, ausgewählt aus der Gruppe, umfassend C₁₋₄-Alkyl, (C₀₋₃-Alkandiyl-C₁₋₃-Alkoxy), C₁₋₄-Alkanoyl, C₁₋₄-Alkoxycarbonyl, Aminocarbonyl und Aryl,
***R***^{***3***} Wasserstoff,
***A*** geradkettiges oder verzweigtes Alkandiyl mit bis zu 8 C-Atomen,
***B*** ein Rest, ausgewählt aus der Gruppe, umfassend COOH, COO***R***^{***5***}, CONH₂, CONH***R***^{***5***} und CON***R***^{***5***}***R***^{***5'***}, jeweils gebunden an ein C-Atom der Gruppe ***A***,
wobei die Reste ***R***^{***5***} und ***R***^{***5'***} gemäß den weiter unten angegebenen Bedeutungen unabhängig voneinander gewählt werden,
***Y*** O
worin in den vorstehenden Resten die Reste ***R***^{***4***}***, R***^{***4'***}***, R***^{***5***} und ***R***^{***5'***} die folgenden Bedeutungen haben:
***R***^{***4***} und ***R***^{***4'***} unabhängig voneinander jeweils ein Rest, ausgewählt aus der Gruppe, umfassend CF₃, C₂F₅, C₁₋₄-Alkyl, C₂₋₄-Alkenyl, C₂₋₃-Alkinyl und (C₀₋₃-Alkandiyl-C₃₋₇-Cycloalkyl),
wobei Alkylreste optional mit einem Rest, ausgewählt aus der Gruppe, umfassend OH, OCH₃ und SCH₃, substituiert sein können,
***R***^{***5***} und ***R***^{***5'***} unabhängig voneinander jeweils ein Rest, ausgewählt aus der Gruppe, umfassend C₁₋₆-Alkyl, C₂₋₆-Alkenyl, C₂₋₆-Alkinyl, (C₀₋₃-Alkandiyl-C₃₋₇-Cycloalkyl), (C₀₋₃-Alkandiyl-Phenyl) und (C₀₋₃-Alkandiyl-Heteroaryl), wobei die Heteroarylgruppe fünf- oder sechsgliedrig ist und ein oder zwei Heteroatome enthält, ausgewählt aus der Gruppe, umfassend N, S und O,
wobei alle zuvor genannten Alkyl- und Cycloalkylreste mit einem Rest, ausgewählt aus der Gruppe, umfassend CF₃, C₂F₅, OH, O-C₁₋₃-Alkyl, NH₂, NH-C₁₋₃-Alkyl, NH-C₁₋₃-Alkanoyl, N(C₁₋₃-Alkyl)₂, N(C₁₋₃-Alkyl)(C₁₋₃-Alkanoyl), COOH, CONH₂ und COO-C₁₋₃-Alkyl, und alle zuvor genannten Phenyl- und Heteroarylgruppen mit bis zu zwei Resten, ausgewählt aus der Gruppe, umfassend F, Cl, Br, CH₃, C₂H₅, OH, OCH₃, OC₂H₅, NO₂, N(CH₃)₂, CF₃, C₂F₅ und SO₂NH₂, substituiert sein können und/oder auch eine anellierte Methandiylbisoxy- oder Ethan-1,2-diylbisoxygruppe tragen können, oder ***R***^{***5***} und ***R***^{***5'***} gemeinsam mit dem Amid-**N**-Atom von ***B*** einen fünf- bis siebengliedrigen, gesättigten oder ungesättigten heterocyclischen Ring bilden, der ein weiteres **N**- oder O- oder S-Atom enthalten kann und der substituiert sein kann mit C₁₋₄-Alkyl, (C₀₋₂-Alkandiyl-C₁₋₄-Alkoxy), C₁₋₄-Alkoxycarbonyl, Aminocarbonyl oder Phenyl.

8. Benzimidazolderivate nach Anspruch 7, **dadurch gekennzeichnet, dass *R***^{***5***} und ***R***^{***5'***} jeweils für einen Rest stehen, ausgewählt aus der Gruppe, umfassend C₁₋₆-Alkyl, C₂₋₆-Alkenyl, C₂₋₆-Alkinyl, (C₀₋₃-Alkandiyl-C₃₋₇-Cycloalkyl), (C₀₋₃-Alkandiyl-Aryl) und (C₀₋₃-Alkandiyl-Heteroaryl), wobei Aryl für Phenyl steht und die Alkyl- und Cycloalkylreste mit einem Rest, ausgewählt aus der Gruppe, umfassend CF₃, C₂F₅, OH, O-C₁₋₃-Alkyl, NH₂, NH-C₁₋₃-Alkyl, NH-C₁₋₃-Alkanoyl, N(C₁₋₃-Alkyl)₂, N(C₁₋₃-Alkyl)(C₁₋₃-Alkanoyl), COOH, CONH₂ und COO-C₁₋₃-Alkyl, substituiert sind.

9. Benzimidazolderivate nach Anspruch 7, **dadurch gekennzeichnet, dass** Aryl in ***R***^{***1***} für Phenyl (C₆H₅-) oder *p*-Methyl-phenyl (*p*-CH₃-C₆H₄-) steht.

10. Benzimidazolderivate nach Anspruch 7, **dadurch gekennzeichnet, dass *R***^{***2***} für C₁₋₃-Alkyl, Phenyl, Methylphenylen, Benzyl oder Heteroaryl steht.

11. Benzimidazolderivate der allgemeinen Formel I nach Anspruch 1
6-[[1-Phenyl-2-propylmercapto-1*H*-benzimidazol-6-yl]oxy]hexansäuremethylester
6-[[1-Phenyl-2-propansulfinyl-1*H*-benzimidazol-6-yl]oxy]hexansäuremethylester
6-[[1-Phenyl-2-propansulfonyl-1*H*-benzimidazol-6-yl]oxy]hexansäuremethylester
6-[[1-(4-Methylphenyl)-2-propylmercapto-1*H*-benzimidazol-6-yl]oxy]hexansäuremethylester
6-[[1-(4-Methylphenyl)-2-propansulfinyl-1*H*-benzimidazol-6-yl]oxy]hexansäuremethylester
6-[[1-(4-Methylphenyl)-2-propansulfonyl-1*H*-benzimidazol-6-yl]oxy]hexansäuremethylester
6-[[2-Benzylmercapto-1-(4-methylphenyl)-1*H*-benzimidazol-6-yl]oxy]hexansäuremethylester
6-[[1-(4-Methylphenyl)-2-(phenylmethansulfinyl)-1*H*-benzimidazol-6-yl]oxy]hexansäuremethylester
6-[[1-(4-Methylphenyl)-2-(phenylmethansulfonyl)-1*H*-benzimidazol-6-yl]oxy]hexansäuremethylester
6-[[1-(4-Methylphenyl)-2-(2-pyridinyl)mercapto-1*H*-benzimidazol-6-yl]oxy]hexansäuremethylester
*N*-(3-Methoxypropyl)-6-[[1-(4-methylphenyl)-2-propylmercapto-1*H*benzimidazol-6-yl]oxy]hexanamid
*N*-(3-Methoxypropyl)-6-[[1-(4-methylphenyl)-2-propansulfonyl-1*H*-benzimidazol-6-yl]oxy]hexanamid
*N*-(3-Methoxypropyl)-6-[[2-benzylmercapto-1-(4-methylphenyl)-1*H*-benzimidazol-6-yl]oxy]
*N*-(3-Methoxypropyl)-6-[[1-(4-Methylphenyl)-2(-phenylmethansulfonyl)-1*H* -benzimidazol-6-yl]oxy]hexanamid
6-[[2-(Morpholin-4-yl)-1-phenyl-1*H*-benzimidazol-6-yl]oxy]hexansäuremethylester
6-[[2-(Piperidin-1-yl)-1-phenyl-1*H*-benzimidazol-6-yl]oxy]hexansäuremethylester
6-[[1-(4-Methylphenyl)-2-(morpholin-4-yl)-1*H*-benzimidazol-6-yl]oxy]hexansäuremethylester
6-[[1-(4-Methylphenyl)-2-(piperidin-1-yl)-1*H*-benzimidazol-6-yl]oxy]hexansäuremethylester
*N*-(3-Methoxypropyl)-6-[[2-(morpholin-4-yl)-1-phenyl-1*H*-benzimidazol-6-yl]oxy]hexanamid
*N*-(3-Methoxypropyl)-6-[[2-(piperidin-1-yl)-1-phenyl-1*H*-benzimidazol-6-yl]oxy]hexanamid
6-[[2-Methoxy-1-phenyl-1*H*-benzimidazol-6-yl]oxy]hexansäuremethylester
6-[[2-Methoxy-1-phenyl-1*H*-benzimidazol-6-yl]oxy]hexansäure
6-[[2-Ethoxy-1-phenyl-1*H*-benzimidazol-6-yl]oxy]hexansäuremethylester
6-[[2-Ethoxy-1-phenyl-1*H*-benzimidazol-6-yl]oxy]hexansäure
6-[[1-Phenyl-2-phenylamino-1*H*-benzimidazol-6-yl]oxy]hexansäuremethylester
6-[[1-Phenyl-2-phenylamino-1*H*-benzimidazol-6-yl]oxy]hexansäure
6-[[1-(4-Methylphenyl)-2-phenylamino-1*H*-benzimidazol-6-yl]oxy]hexansäuremethylester
6-[[1-Phenyl-2-propylamino-1*H*-benzimidazol-6-yl]oxy]hexansäuremethylester
6-[[1-Phenyl-2-propylamino-1*H*-benzimidazol-6-yl]oxy]hexansäure
6-[[2-(*N*-Methyl-*N*-propyl)amino-1-phenyl-1*H*-benzimidazol-6-yl]oxy]hexansäuremethylester
6-[[1-(4-Methylphenyl)-2-phenyloxy-1*H*-benzimidazol-6-yl]oxy]hexansäure
6-[[1-(4-Methylphenyl)-2-phenylmercapto-1*H*-benzimidazol-6-yl]oxy]hexansäuremethylester
6-[[1-(4-Methylphenyl)-2-(phenylsulfinyl)-1*H*-benzimidazol-6-yl]oxy]hexansäuremethylester
6-[[1-(4-Methylphenyl)-2-(phenylsulfonyl)-1*H*-benzimidazol-6-yl]oxy]hexansäuremethylester
6-[[1-Phenyl-2-propylmercapto-1*H*-benzimidazol-6-yl]oxy]hexansäure
6-[[1-Phenyl-2-propansulfonyl-1*H*-benzimidazol-6-yl]oxy]hexansäure
6-[(2-Benzylmercapto-1-phenyl-1*H*-benzimidazol-6-yl)oxy]hexansäuremethylester
6-[[1-Phenyl-2-(phenylmethansulfonyl)-1*H*-benzimidazol-6-yl]oxy]hexansäuremethylester
6-[(2-Benzylmercapto-1-phenyl-1*H*-benzimidazol-6-yl)oxy]hexansäure
6-[[1-Phenyl-2-(phenylmethansulfonyl)-1*H*-benzimidazol-6-yl]oxy]hexansäure
6-[[1-(4-Methylphenyl)-2-propylmercapto-1*H*-benzimidazol-6-yl]oxy]hexansäure
6-[[1-(4-Methylphenyl)-2-propansulfonyl-1*H*-benzimidazol-6-yl]oxy]hexansäure
6-[[2-Benzylmercapto-1-(4-methylphenyl)-1*H*-benzimidazol-6-yl]oxy]hexansäure
6-[[1-(4-Methylphenyl)-2-(phenylmethansulfonyl)-1*H*-benzimidazol-6-yl]oxy]-hexansäure.

12. Verwendung der Benzimidazolderivate mit der allgemeinen Formel I nach einem der Ansprüche 1 bis 11 zur Herstellung von Arzneimitteln zur Behandlung von mit einer Mikroglia-Aktivierung assoziierten Erkrankungen sowie zur Prophylaxe gegen diese Erkrankungen, wobei die Benzimidazolderivate zusätzlich auch Verbindungen umfassen, bei denen B für Wasserstoff steht.

13. Pharmazeutische Präparate, enthaltend mindestens ein Benzimidazolderivat mit der allgemeinen Formel I nach einem der Ansprüche 1 bis 11 sowie mindestens einen pharmazeutisch verträglichen Träger.

14. Verwendung einer Verbindung nach einem der Ansprüche 1 - 11 zur Herstellung eines Arzneimittels zur Behandlung von inflammatorischen, allergischen, infektiösen oder autoimmunen Erkrankungen.

15. Verwendung nach Anspruch 14 zur Behandlung von Stroke.

## Claims

1. Benzimidazole derivatives having the general formula I 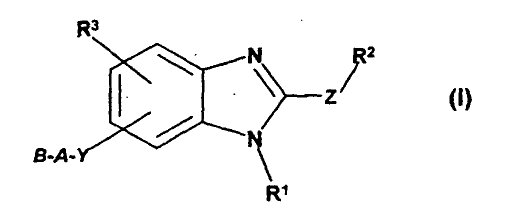 in which
*R*^{*1*} is an aryl group or a five- or six-membered heteroaryl group having one or two heteroatoms selected from the group comprising N, S and O,
where the aryl or heteroaryl group may be substituted by up to three radicals independently of one another, selected from the group comprising F, Cl, Br, C(NH)NH₂, (NH)NH*R*^{*4*},
C(NH)N***R***^{***4***}***R***^{***4'***}, C(N***R***^{***4***})NH₂, C(N***R***^{***4***})NH***R***^{***4'***}, C(N***R***^{***4***})N***R***^{***4***}***R***^{***4'***}, ***X***-OH, ***X***-O***R***^{***4***}, ***X***-OCO***R***^{***4***}, ***X***-OCONH***R***^{***4***},
***X****-*CO***R***^{***4***}, ***X***-C(NOH)***R***^{***4***}, ***X***-CN, ***X***-COOH, ***X***-COO***R***^{***4***}, ***X***-CONH₂,
***X***-CON***R***^{***4***}***R***^{***4'***}, ***X***-CONH***R***^{***4***},
***X***-CONHOH, ***X***-S***R***^{***4***}, ***X***-SO***R***^{***4***}, ***X***-SO₂***R***^{***4***}, SO₂NH₂, SO₂NH***R***^{***4***},
SO₂N***R***^{***4***}***R***^{***4'***}, NO₂, ***X***-NH₂,
***X***-NH***R***^{***4***}, ***X***-N***R***^{***4***}***R***^{***4'***}, ***X***-NHSO₂***R***^{***4***}, ***X***-N***R***^{***4***}SO₂***R***^{***4'***}, ***X***-NHCO***R***^{***4***}, ***X***-NHCOO***R***^{***4***}, ***X***-NHCONH***R***^{***4***} and ***R***^{***4***},
where X is a bond, CH₂, (CH₂)₂ or CH(CH₃), and
where furthermore *R*^{*4*} and *R*^{*4'*} have independently of one another the meanings indicated hereinafter, and
where two substituents on *R*^{*1*} may in each case be linked to one another so that together they form a methanediylbisoxy, ethane-1,2-diylbisoxy, propane-1,3-diyl or butane-1,4-diyl group when the substituents on *R*^{*1*} are in ortho positions relative to one another,
*Z* is a grouping selected from the group comprising NH, N*R*^{*2'*}, O, S, SO and SO₂, in which *R*^{*2'*} has the meaning indicated below,
*R*^{*2*} and *R*^{*2'*} are each a radical selected from the group comprising
C₁₋₄-perfluoroalkyl, C₁₋₆-alkyl, (C₀₋₃-alkanediyl-C₃₋₇ cycloalkyl), (C₀₋₃-alkanediylaryl) and (C₀₋₃-alkanediyl-heteroaryl),
where the heteroaryl group has five or six members and comprises one or two heteroatoms selected from the group comprising N, S and O, and where *R*^{*2*} and *R*^{*2'*} are chosen independently of one another,
where the aryl and/or heteroaryl group may in each case be substituted by up to two radicals selected from the group comprising F, Cl, Br, CH₃, C₂H₅, OH, OCH₃, OC₂H₅, NO₂, CF₃, C₂F₅ and SO₂NH₂, and/or may have a fused-on methanediylbisoxy and/or ethane-1,2-diylbisoxy group,
where one ring member in the cycloalkyl ring may furthermore be ring N or ring O if the cycloalkyl ring has five members, or one or two ring members in the cycloalkyl ring may each be ring N and/or ring O atoms if the cycloalkyl ring has six or seven members,
where furthermore the ring N atoms may be substituted by C₁₋₃-alkyl or C₁₋₃-alkanoyl,
or if *Z* is N*R*^{*2'*}, then *R*^{*2*} and *R*^{*2'*} may form together with *Z* a five- to seven-membered heterocyclic ring, where the heterocyclic ring may be saturated, which may comprise a further N, O or S atom, and which may be substituted by a radical selected from the group comprising C₁₋₄-alkyl, (C₀₋₃)-alkanediyl-C₁₋₃-alkoxy), C₁₋₄-alkanoyl, C₁₋₄-alkoxycarbonyl, aminocarbonyl and phenyl,
*R*^{*3*} is one or two substituents which can be chosen independently of one another, selected from the group comprising:
hydrogen, F, Cl, Br, OH, O*R*^{*4*}, OCO*R*^{*4*}, OCONH*R*^{*4*}, CO*R*^{*4*}, CN
COOH, COO***R***^{***4***}, CONH₂, CONH***R***^{***4***}, CON***R***^{***4***}***R***^{***4'***}, CONHOH,
CONHO***R***^{***4***}, S***R***^{***4***}, SO***R***^{***4***}, SO₂***R***^{***4***}, SO₂NH₂, SO₂NH***R***^{***4***}, SO₂N***R***^{***4***}***R***^{***4'***},
NO₂, NH₂, NH***R***^{***4***}, N***R***^{***4***}***R***^{***4'***}, NHSO₂***R***^{***4***}, N***R***^{***4***}SO₂***R***^{***4'***}, NNSO₂***R***^{***6***},
N***R***^{***4***}SO₂***R***^{***6***}, NHCO***R***^{***4***}, NHCOO***R***^{***4***}, NHCONH***R***^{***4***} and ***R***^{***4***},
where the radicals *R*^{*4*}, *R*^{*4'*} and *R*^{*6*} are chosen independently of one another and have the meanings indicated hereinafter,
*A* is a group selected from the group comprising C₁₋₁₀-alkanediyl, C₂₋₁₀-alkenediyl, C₂₋₁₀-alkynediyl and (C₀₋₃-alkanediyl-C₃₋₇-cycloalkanediyl-C₀₋₃-alkanediyl), where one ring member in the cycloalkyl ring may be ring N or ring O if the cycloalkyl ring has five members, or one or two ring members in the cycloalkyl ring may each be ring N and/or ring O atoms if the cycloalkyl ring has six or seven members, where the ring N atoms may be substituted by at least one radical selected from the group comprising C₁₋₃-alkyl and C₁₋₃-alkanoyl radicals, where furthermore one C atom in the aliphatic chains of the C₁₋₁₀-alkanediyl, C₂₋₁₀-alkenediyl, C₂₋₁₀-alkynediyl and (C₀₋₃-alkanediyl-C₃₋₇-cycloalkanediyl-C₀₋₃-alkanediyl) groups may be replaced by O, NH, N-C₁₋₃-alkyl or N-C₁₋₃-alkanoyl, and
where at least one of the alkyl and cycloalkyl groups may be substituted by a radical selected from the group comprising =O, OH, O-C₁₋₃-alkyl, NH₂, NH-C₁₋₃-alkyl, NH(C₁₋₃-alkanoyl, N(C₁₋₃-alkyl)₂ and N(C₁₋₃-alkyl)(C₁₋₃-alkanoyl),
*B* is a radical selected from the group comprising COOH, COO*R*^{*5*}, CONH₂, CONHNH₂, CONH*R*^{*5*}, CON*R*^{*5*}*R*^{*5'*}, CONHOH, CONHO*R*^{*5*} and tetrazolyl,
where *B* is bonded to a C atom of group *A,*
where the radicals *R*^{*5*} and *R*^{*5'*} are chosen independently of one another and have the meanings indicated hereinafter,
*Y* is O,
*R*^{*4*} and *R*^{*4'*} are each a radical selected from the group comprising CF₃, C₂F₅, C₁₋₄-alkyl, C₂₋₄-alkenyl, C₂₋₃-alkynyl and (C₀₋₃-alkanediyl-C₃₋₇-cycloalkyl),
where one ring member in the cycloalkyl ring may be ring N or ring O if the cycloalkyl ring has five members, or that one or two ring members in the cycloalkyl ring may each be ring N and/or ring O atoms if the cycloalkyl ring has six or seven members, where the ring N atoms may be substituted by C₁₋₃-alkyl or C₁₋₃-alkanoyl,
*R*^{*5*} and *R*^{*5'*} are each a radical selected from the group comprising
C₁₋₆-alkyl, C₂₋₆-alkenyl, C₂₋₆-alkynyl, where one C atom in at least one of the radicals *R*^{*5*} and *R*^{*5'*} may be replaced by O, S, SO, SO₂, NH, N-C₁₋₃-alkyl or N-C₁₋₃-alkanoyl,
furthermore (C₀₋₃-alkanediyl-C₃₋₇-cycloalkyl), where one ring member in the cycloalkyl ring may be ring N or ring O if the cycloalkyl ring has five members, or that one or two ring members in the cycloalkyl ring may each be one or two ring N and/or ring O atoms if the cycloalkyl ring has six or seven members, where the ring N atom may be substituted by at least one radical selected from the group comprising C₁₋₃-alkyl radicals and C₁₋₃-alkanoyl radicals,
furthermore (C₀₋₃-alkanediyl-aryl) and (C₀₋₃-alkanediyl-heteroaryl), where the heteroaryl group may have five or six members and may comprise one or two heteroatoms selected from the group comprising N, S and O,
where furthermore at least one of the alkyl and cycloalkyl radicals of *R*^{*5*} and *R*^{*5'*} may be substituted by up to two radicals selected from the group comprising CF₃, C₂F₅, OH, O-C₁₋₃₋alkyl, NH₂, NH-C₁₋₃-alkyl, NH-C₁₋₃-alkanoyl, N(C₁₋₃-alkyl)₂, N(C₁₋₃-alkyl)(C₁₋₃-alkanoyl), COOH, CONH₂ and COO-C₁₋₃-alkyl and that at least one of the aryl and heteroaryl radicals of *R*^{*5*} and *R*^{*5'*} may be substituted by up to two radicals selected from the group comprising F, Cl, Br, CH₃, C₂H₅, OH, OCH₃, OC₂H₅, NO₂, N(CH₃)₂, CF₃, C₂F₅ and SO₂NH₂, and/or that at least one of the alkyl, cycloalkyl, aryl and/or heteroaryl radicals of *R*^{*5*} and *R*^{*5'*} may have a fused-on methanediylbisoxy or ethane-1,2-diylbisoxy group,
or where *R*^{*5*} and *R*^{*5'*} may form together with the amide N atom of *B* a five- to seven-membered, saturated or unsaturated heterocyclic ring which may comprise a further N or O or S atom and which may be substituted by C₁₋₄-alkyl, (C₀₋₂-alkanediyl-C₁₋₄-alkoxy), C₁₋₄-alkoxycarbonyl, aminocarbonyl or aryl,
*R*^{*6*} is a radical selected from the group comprising (C₀₋₃-alkanediyl-aryl) and (C₀₋₃-alkanediyl-heteroaryl), where the heteroaryl group has five or six members and comprises one or two heteroatoms selected from the group comprising N, S and O, and
where at least one of the aryl and heteroaryl groups may be substituted in each case by up to two radicals selected from the group comprising F, Cl, Br, CH₃, C₂H₅, OH, OCH₃, OC₂H₅, NO₂, N(CH₃)₂, CF₃, C₂F₅ and SO₂NH₂, or that at least one of the aryl or heteroaryl groups may also have a fused-on methanediylbisoxy or ethane-1,2-diylbisoxy group.

2. Benzimidazole derivatives according to Claim 1, **characterized in that** *R*^{*1*} is phenyl and may be substituted by up to two radicals independently of one another, selected from the group comprising F, Cl, Br,
C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4'***},
C(N***R***^{***4***})N***R***^{***4***}***R***^{***4'***}, OH, O***R***^{***4***}, OCO***R***^{***4***}, OCONH***R***^{***4***}, CO***R***^{***4***}, C(NOH)***R***^{***4***}, CN,
COOH, COO***R***^{***4***}, CONH₂, CON***R***^{***4***}***R***^{***4'***}, CONH***R***^{***4***}, CONHOH, S***R***^{***4***}, SO***R***^{***4***},
SO₂***R***^{***4***}, SO₂NH₂, SO₂NH***R***^{***4***},SO₂N***R***^{***4***}***R***^{***4'***}, NO₂, NH₂, NH***R***^{***4***}, N***R***^{***4***}***R***^{***4'***},
NHCONH***R***^{***4***} and ***R***^{***4***}.

3. Benzimidazole derivatives according to Claim 1, **characterized in that** *R*^{*3*} is a radical selected from the group comprising hydrogen, F, Cl, Br, CH₃, C₂H₅, CF₃, C₂F₅, OH, O*R*^{*4*}, NHSO₂*R*^{*6*} and NHCO*R*^{*4*}.

4. Benzimidazole derivatives according to Claim 1, **characterized in that** *B* is a radical selected from the group comprising COOH, COO*R*^{*5*}, CONH₂, CONH*R*^{*5*} and CON*R*^{*5*}*NR*^{*5'*}.

5. Benzimidazole derivatives according to Claim 1, **characterized in that** *B-A-Y* is in position 6 of the benzimidazole.

6. Benzimidazole derivatives according to Claim 1, **characterized in that** *R*^{*6*} is a phenyl or heteroaryl group, where the heteroaryl group has five or six members and comprises one or two heteroatoms selected from the group comprising N, S and O.

7. Benzimidazoles of the general formula I according to Claim 1, in which
*R*^{*1*} is a phenyl group which may be substituted by up to two radicals independently of one another, selected from the group comprising:
F, Cl, Br,
C(NH)NH₂, C(NH)NH***R***^{***4***}, C(NH)N***R***^{***4***}***R***^{***4'***}, C(N***R***^{***4***})NH₂, C(N***R***^{***4***})NH***R***^{***4'***},
C(N***R***^{***4***})N***R***^{***4***}***R***^{***4'***},
OH, O***R***^{***4***}, OCO***R***^{***4***}, OCONH***R***^{***4***},
CO***R***^{***4***}, C(NOH)***R***^{***4***},
CN, COOH, COO***R***^{***4***}, CONH₂, CON***R***^{***4***}***R***^{***4'***}, CONH***R***^{***4***}, CONHOH,
S***R***^{***4***}, SO***R***^{***4***}, SO₂***R***^{***4***},
SO₂NH₂, SO₂NH***R***^{***4***},SO₂N***R***^{***4***}***R***^{***4'***},
NO₂, NH₂, NH***R***^{***4***}, N***R***^{***4***}***R***^{***4'***}, NHCONH***R***^{***4***} and
***R***^{***4***},
where the radicals *R*^{*4*} and *R*^{*4'*} are chosen independently of one another according to the meanings indicated below, and
where two substituents on *R*^{*1*} may be linked to one another so that together they form a methanediylbisoxy, ethane-1,2-diylbisoxy, propane-1,3-diyl or butane-1,4-diyl group when they are in ortho positions relative to one another,
*Z* NH, N*R*^{*2'*}, O, SO or SO₂,
*R*^{*2*} and *R*^{*2'*} independently of one another in each case a radical selected from the group comprising:
C₁₋₄-perfluoroalkyl, C₁₋₆-alkyl, (C₀₋₃-alkanediyl-aryl) and (C₀₋₃-alkanediyl-heteroaryl),
where the heteroaryl group has five or six members and comprises one or two heteroatoms selected from the group comprising N, S and O, and where the aryl and heteroaryl group may in each case be substituted by up to two radicals selected from the group comprising F, Cl, Br, CH₃, C₂H₅, OH, OCH₃, OC₂H₅, NO₂, CF₃, C₂F₅ and SO₂NH₂,
or if *Z* is N*R*^{*2'*}, then *R*^{*2*} and *R*^{*2'*} form together with *Z* a five- to seven-membered heterocyclic ring, where furthermore the heterocyclic ring may comprise an additional O or S atom and may optionally be substituted by a radical selected from the group comprising C₁₋₄-alkyl, (C₀₋₃-alkanediyl-C₁₋₃-alkoxy), C₁₋₄-alkanoyl, C₁₋₄-alkoxycarbonyl, aminocarbonyl and aryl,
*R*^{*3*} hydrogen,
*A* straight-chain or branched alkanediyl having up to 8 C atoms,
*B* a radical selected from the group comprising COOH, COO*R*^{*5*}, CONH₂, CONH*R*^{*5*} and CON*R*^{*5*}*R*^{*5'*}, in each case bonded to a C atom of group *A*,
where the radicals *R*^{*5*} and *R*^{*5'*} are chosen independently of one another according to the meanings indicated hereinafter,
*Y* O
in which the radicals *R*^{*4*}*, R*^{*4'*}*, R*^{*5*} and *R*^{*5'*} in the aforementioned radicals have the following meanings:
*R*^{*4*} and *R*^{*4'*} independently of one another in each case a radical selected from the group comprising CF₃, C₂F₅, C₁₋₄-alkyl, C₂₋₄-alkenyl, C₂₋₃-alkynyl and (C₀₋₃-alkanediyl-C₃₋₇-cycloalkyl),
where alkyl radicals may optionally be substituted by a radical selected from the group comprising OH, OCH₃ and SCH₃,
*R*^{*5*} and *R*^{*5'*} independently of one another in each case a radical selected from the group comprising C₁₋₆-alkyl, C₂₋₆-alkenyl, C₂₋₆-alkynyl, (C₀₋₃-alkanediyl-C₃₋₇-cycloalkyl), (C₀₋₃-alkanediyl-phenyl) and (C₀₋₃-alkanediyl-heteroaryl), where the heteroaryl group has five or six members and comprises one or two heteroatoms selected from the group comprising N, S and O,
where all the aforementioned alkyl and cycloalkyl radicals may be substituted by a radical selected from the group comprising CF₃, C₂F₅, OH, O-C₁₋₃-alkyl, NH₂, NH-C₁₋₃-alkyl, NH-C₁₋₃-alkanoyl, N(C₁₋₃-alkyl)₂, N(C₁₋₃-alkyl)(C₁₋₃-alkanoyl), COOH, CONH₂ and COO-C₁₋₃-alkyl, and all aforementioned phenyl and heteroaryl groups may be substituted by up to two radicals selected from the group comprising F, Cl, Br, CH₃, C₂H₅, OH, OCH₃, OC₂H₅, NO₂, N(CH₃)₂, CF₃, C₂F₅ and SO₂NH₂, and/or may also have a fused-on methanediylbisoxy or ethane-1,2-diylbisoxy group,
or *R*^{*5*} and *R*^{*5'*} form together with the amide N atom of *B* a five- to seven-membered, saturated or unsaturated heterocyclic ring which may comprise a further N or O or S atom and which may be substituted by C₁₋₄-alkyl, (C₀₋₂-alkanediyl-C₁₋₄-alkoxy), C₁₋₄-alkoxycarbonyl, aminocarbonyl or phenyl.

8. Benzimidazole derivatives according to Claim 7, **characterized in that** *R*^{*5*} and *R*^{*5'*} are each a radical selected from the group comprising C₁₋₆-alkyl, C₂₋₆-alkenyl, C₂₋₆-alkynyl, (C₀₋₃-alkanediyl-C₃₋₇-cycloalkyl), (C₀₋₃-alkanediyl-aryl) and (C₀₋₃-alkanediylheteroaryl), where aryl is phenyl, and the alkyl and cycloalkyl radicals are substituted by a radical selected from the group comprising CF₃, C₂F₅, OH, O-C₁₋₃-alkyl, NH₂, NH-C₁₋₃-alkyl, NH-C₁₋₃-alkanoyl, N(C₁₋₃-alkyl)₂, N(C₁₋₃-alkyl)(C₁₋₃-alkanoyl), COOH, CONH₂ and COO-C₁₋₃-alkyl.

9. Benzimidazole derivatives according to Claim 7, **characterized in that** aryl in ***R***^{***1***} is phenyl(C₆H₅) or *p*-methylphenyl (*p*-CH₃-C₆H₄).

10. Benzimidazole derivatives according to Claim 7, **characterized in that** *R*^{*2*} is C₁₋₃-alkyl, phenyl, methylphenylene, benzyl or heteroaryl.

11. Benzimidazole derivatives of the general formula I according to Claim 1
methyl 6-[[1-phenyl-2-propylmercapto-1*H*-benzimidazol-6-yl]oxy]hexanoate
methyl 6-[[1-phenyl-2-propanesulphinyl-1*H*-benzimidazol-6-yl]oxy]hexanoate
methyl 6-[[1-phenyl-2-propanesulphonyl-1*H*-benzimidazol-6-yl]oxy]hexanoate
methyl 6-[[1-(4-methylphenyl)-2-propylmercapto-1*H*-benzimidazol-6-yl]oxy]hexanoate
methyl 6-[[1-(4-methylphenyl)-2-propanesulphinyl-1*H*-benzimidazol-6-yl]oxy]hexanoate
methyl 6-[[1-(4-methylphenyl)-2-propanesulphonyl-1*H*-benzimidazol-6-yl]oxy]hexanoate
methyl 6-[[2-benzylmercapto-1-(4-methylphenyl)-1*H*-benzimidazol-6-yl]oxy]hexanoate
methyl 6-[[1-(4-methylphenyl)-2-(phenylmethanesulphinyl)-1*H*-benzimidazol-6-yl]oxy]hexanoate
methyl 6-[[1-(4-methylphenyl)-2-(phenylmethanesulphonyl)-1*H*-benzimidazol-6-yl]oxy]hexanoate
methyl 6-[[1-(4-methylphenyl)-2-(2-pyridinyl)mercapto-1*H*-benzimidazol-6-yl]oxy]hexanoate
*N*-(3-methoxypropyl)-6-[[1-(4-methylphenyl)-2-propylmercapto-1*H*-benzimidazol-6-yl]oxy]hexanamide
*N*-(3-methoxypropyl)-6-[[1-(4-methylphenyl)-2-propanesulphonyl-1*H*-benzimidazol-6-yl]oxy]hexanamide
*N*-(3-methoxypropyl)-6-[[2-benzylmercapto-1-(4-methylphenyl)-1*H*-benzimidazol-6-yl]oxy]
*N*-(3-methoxypropyl)-6-[[1-(4-methylphenyl)-2-(phenylmethanesulphonyl)-1*H*-benzimidazol-6-yl]oxy]hexanamide
methyl 6-[[2-(morpholin-4-yl)-1-phenyl-1*H*-benzimidazol-6-yl]oxy]hexanoate
methyl 6-[[2-(piperidin-1-yl)-1-phenyl-1*H*-benzimidazol-6-yl]oxy]hexanoate
methyl 6-[[1-(4-methylphenyl)-2-(morpholin-4-yl)-1*H*-benzimidazol-6-yl]oxy]hexanoate
methyl 6-[[1-(4-methylphenyl)-2-(piperidin-1-yl)-1*H*-benzimidazol-6-yl]oxy]hexanoate
*N*-(3-methoxypropyl)-6-[[2-(morpholin-4-yl)-1-phenyl-1*H*-benzimidazol-6-yl]oxy]hexanamide
*N*-(3-methoxypropyl)-6-[[2-(piperidin-1-yl)-1-phenyl-1*H*-benzimidazol-6-yl]oxy]hexanamide
methyl 6-[[2-methoxy-1-phenyl-1*H*-benzimidazol-6-yl]oxy]hexanoate 6-[[2-methoxy-1-phenyl-1*H*-benzimidazol-6-yl]oxy]hexanoic acid
methyl 6-[[2-(ethoxy-1-phenyl-1*H*-benzimidazol-6-yl]oxy]hexanoate 6-[[2-ethoxy-1-phenyl-1*H*-benzimidazol-6-yl]oxy]hexanoic acid
methyl 6-[[1-phenyl-2-phenylamino-1*H*-benzimidazol-6-yl]oxy]hexanoate 6-[[1-phenyl-2-phenylamino-1*H*-benzimidazol-6-yl]oxy]hexanoic acid
methyl 6-[[1-(4-methylphenyl)-2-phenylamino-1*H*-benzimidazol-6-yl]oxy]hexanoate
methyl 6-[[1-phenyl-2-propylamino-1*H*-benzimidazol-6-yl]oxy]hexanoate 6-[[1-phenyl-2-propylamino-1*H*-benzimidazol-6-yl]oxy]hexanoic acid
methyl 6-[[2-(*N*-methyl-*N*-propyl)amino-1-phenyl-1*H*-benzimidazol-6-yl]oxy]hexanoate 6-[[1-(4-methylphenyl)-2-phenyloxy-1*H*-benzimidazol-6-yl]oxy]hexanoic acid
methyl 6-[[1-(4-methylphenyl)-2-phenylmercapto-1*H*-benzimidazol-6-yl]oxy]hexanoate
methyl 6-[[1-(4-methylphenyl)-2-(phenylsulphinyl)-1*H*-benzimidaozl-6-yl]oxy]hexanoate
methyl 6-[[1-(4-methylphenyl)-2-(phenylsulphonyl)-1*H*-benzimidazol-6-yl]oxy]hexanoate
6-[[1-phenyl-2-propylmercapto-1*H*-benzimidazol-6-yl]oxy]hexanoic acid
6-[[1-phenyl-2-propanesulphonyl-1*H*-benzimidazol-6-yl]oxy]hexanoic acid
methyl 6-[(2-benzylmercapto-1-phenyl-1*H*-benzimidazol-6-yl)oxy]hexanoate
methyl 6-[[1-phenyl-2-(phenylmethanesulphonyl)-1*H*-benzimidazol-6-yl]oxy]hexanoate
6-[[2-benzylmercapto-1-phenyl-1*H*-benzimidazol-6-yl)oxy]hexanoic acid
6-[[1-phenyl-2-(phenylmethanesulphonyl)-1*H*-benzimidazol-6-yl]oxy]hexanoic acid
6-[[1-(4-methylphenyl)-2-propylmercapto-1*H*-benzimidazol-6-yl]oxy]hexanoic acid
6-[[1-(4-methylphenyl)-2-propanesulphonyl-1*H*-benzimidazol-6-yl]oxy]hexanoic acid
6-[[2-benzylmercapto-1-(4-methylphenyl)-1*H*-benzimidazol-6-yl]oxy]hexanoic acid
6-[[1-(4-methylphenyl)-2-(phenylmethanesulphonyl)-1*H*-benzimidazol-6-yl]oxy]hexanoic acid.

12. Use of the benzimidazole derivatives having the general formula I as claimed in any of claims 1 to 11 for producing medicaments for treating diseases associated with microglia activation, and for prophylaxis against these diseases, where the benzimidazole derivatives additionally also include compounds in which *B* is hydrogen.

13. Pharmaceutical products comprising at least one benzimidazole derivative having the general formula I as claimed in any of claims 1 to 11 and at least one pharmaceutically acceptable carrier.

14. Use of a compound according to any of claims 1-11 for producing a medicament for treating inflammatory, allergic, infectious or autoimmune diseases.

15. Use according to Claim 14 for treating strokes.

## Revendications

1. Dérivés de benzimidazole de formule générale I 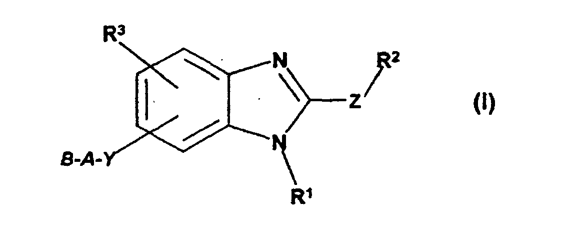 dans laquelle
*R*^{*1*} représente un groupe aryle ou un groupe hétéroaryle à cinq ou six chaînons, renfermant un ou deux hétéroatomes, choisis parmi le groupe constitué de N, S et O,
où le groupe aryle ou hétéroaryle peut être substitué par jusqu'à trois radicaux indépendamment les uns des autres, choisis parmi le groupe constitué de F, CI, Br, C(NH)NH₂, C(NH)NH*R*^{*4*}, C(NH)N*R*^{*4*}*R*^{*4*}, C(N*R*^{*4*})NH₂, C(N*R*^{*4*})NH*R*^{*4*}, C(N*R*^{*4*})N*R*^{*4*}*R*^{*4*}*, X*-OH, *X-*O*R*^{*4*}, *X*-OCO*R*^{*4*}, *X*-OCONH*R*^{*4*},
*X*-CO*R*^{*4*}, *X-*C(NOH)*R*^{*4*}, *X*-CN, *X*-COOH, *X*-COO*R*^{*4*}, *X*-CONH₂, *X-* CON*R*^{*4*}*R*^{*4'*}, *X*-CONH*R*^{*4*},
*X*-CONHOH, *X*-S*R*^{*4*}, *X*-SO*R*^{*4*}, *X*-SO₂*R*^{*4*}, SO₂NH₂, SO₂NH*R*^{*4*}, SO₂N*R*^{*4*}*R*^{*4'*}, NO₂, *X*-NH₂,
*X-*NH*R*^{*4*}, *X*-N*R*^{*4*}*R*^{*4'*}, *X-*NHSO₂*R*^{*4*}, *X*-N*R*^{*4*}SO₂*R*^{*4'*}, *X*-NHCO*R*^{*4*}, *X*-NHCOO*R*^{*4*}, *X*-NHCONH*R*^{*4*} et *R*^{*4*},
où *X* est une liaison, CH₂, (CH₂)₂ ou CH(CH₃) et
où, en outre, *R*^{*4*} et *R*^{*4'*} présentent, indépendamment les uns des autres, les significations indiquées ci-dessous, et
où deux substituants sur *R*^{*1*} peuvent, dans chaque cas, être liés l'un à l'autre de telle sorte qu'ils forment conjointement un groupe méthanediylbisoxy, éthane-1,2-diylbisoxy, propan-1,3-diyle ou butan-1,4-diyle,
lorsque les substituants sur *R*^{*1*} sont en position ortho l'un par rapport à l'autre,
Z représente un groupement choisi parmi le groupe constitué de NH, N*R*^{*2'*}, O, S, SO et SO₂, où *R*^{*2'*} représente la signification indiquée ci-après,
*R*^{*2*} et *R*^{*2'*} représentent chacun un radical choisi parmi le groupe constitué de :
un perfluoroalkyle en C₁₋₄, un alkyle en C₁₋₆, un (C₀₋₃-alcanediyle-C₃₋₇-cycloalkyle), un (C₀₋₃-alcanediyl-aryle) et un (C₀₋₃-alcanediyl-hétéroaryle),
où le groupe hétéroaryle est à cinq ou six chaînons et renferme un ou deux hétéroatomes choisis parmi le groupe constitué de N, S et O, et où *R*^{*2*} et *R*^{*2'*} sont choisis indépendamment l'un de l'autre,
où le groupe aryle et/ou hétéroaryle peut, dans chaque cas, être substitué par jusqu'à deux radicaux choisis parmi le groupe constitué de F, Cl, Br, CH₃, C₂H₅, OH, OCH₃, OC₂H₅, NO₂, CF₃, C₂F₅ et SO₂NH₂, et/ou peut porter un groupe méthandiylbisoxy et/ou éthan-1,2-diylbisoxy cyclisé,
où un chaînon cyclique dans le noyau cycloalkyle peut en outre être un azote cyclique ou un oxygène cyclique, lorsque le noyau cycloalkyle est à cinq chaînons, ou un ou deux chaînons cycliques dans le noyau cycloalkyle peuvent chacun être des atomes d'azote cycliques et/ou d'oxygène cycliques, lorsque le noyau cycloalkyle est à six ou sept chaînons,
où, en outre, les atomes d'azote cycliques peuvent être substitués par un alkyle en C₁₋₃ ou un alcanoyle en C₁₋₃,
ou lorsque *Z* est N*R*^{*2'*}, *R*^{*2*} et *R*^{*2'*} peuvent former, conjointement avec *Z*, un noyau hétérocyclique à cinq à sept chaînons, où le noyau hétérocyclique peut être saturé, lequel peut renfermer un atome d'azote, d'oxygène ou de soufre supplémentaire et peut être substitué par un radical choisi parmi le groupe constitué d'un alkyle en C₁₋₄, d'un (C₀₋₃-alcandiyl-C₁₋₃-alcoxy), d'un alcanoyle en C₁₋₄, d'un C₁₋₄-alcoxycarbonyle, d'un aminocarbonyle et d'un phényle,
*R*^{*3*} représente un ou deux substituants pouvant être choisis indépendamment l'un de l'autre, choisis parmi le groupe constitué de : un hydrogène, F, Cl, Br, OH, O*R*^{*4*}, OCO*R*^{*4*}, OCONH*R*^{*4*}, CO*R*^{*4*}, CN, COOH, COO*R*^{*4*}, CONH₂, CONH*R*^{*4*}, CON*R*^{*4*}*R*^{*4'*}, CONHOH, CONHO*R*^{*4*}, S*R*^{*4*}, SO*R*^{*4*}, SO₂*R*^{*4*}, SO₂NH₂, SO₂NH*R*^{*4*}, SO₂N*R*^{*4*}*R*^{*4'*}, NO₂, NH₂, NH*R*^{*4*}, N*R*^{*4*}*R*^{*4'*}, NHSO₂*R*^{*4*}, N*R*^{*4*}SO₂*R*^{*4'*}, NHSO₂*R*^{*6*}, N*R*^{*4*}SO₂*R*^{*6*}, NHCO*R*^{*4*}, NHCOO*R*^{*4*}, NHCONH*R*^{*4*} et *R*^{*4*},
où les radicaux *R*^{*4*}, *R*^{*4'*} et *R*^{*6*} sont choisis indépendamment les uns des autres et présentent les significations indiquées ci-dessous,
*A* représente un groupe choisi parmi le groupe constitué d'un C₁₋₁₀-alcanediyle, d'un C₂₋₁₀-alcénediyle, d'un C₂₋₁₀-alcynediyle et d'un (C₀₋₃-alcanediyl-C₃₋₇-cycloalcanediyl-C₀₋₃-alcanediyle),
où un chaînon cyclique dans le noyau cycloalkyle peut être un azote cyclique ou un oxygène cyclique, lorsque le noyau cycloalkyle est à cinq chaînons, ou un ou deux chaînons cycliques dans le noyau cycloalkyle peuvent chacun être des atomes d'azote cycliques et/ou d'oxygène cycliques, lorsque le noyau cycloalkyle est à six ou sept chaînons, où les atomes d'azote cycliques peuvent être substitués par au moins un radical choisi parmi le groupe constitué de radicaux alkyle en C₁₋₃ et alcanoyle en C₁₋₃,
où, dans les chaînes aliphatiques des groupes C₁₋₁₀-alcanediyle, C₂₋₁₀-alcénediyle, C₂₋₁₀-alcynediyle et (C₀₋₃-alcanediyl-C₃₋₇-cycloalcanediyle-C₀₋₃-alcanediyle), en outre un atome de carbone peut être remplacé par O, NH, un N-C₁₋₃-alkyle ou un N-C₁₋₃-alcanoyle, et
où au moins l'un des groupes alkyle et cycloalkyle peut être substitué par un radical choisi parmi le groupe constitué de =O, OH, d'un O-C₁₋₃-alkyle, NH₂, d'un NH-C₁₋₃-alkyle, d'un NH-C₁₋₃-alcanoyle, d'un N(C₁₋₃-alkyle)₂ et d'un N(C₁₋₃-alkyl)(C₁₋₃-alcanoyle),
*B* représente un radical choisi parmi le groupe constitué de COOH, COO*R*^{*5*}, CONH₂, CONHNH₂, CONH*R*^{*5*}, CON*R*^{*5*}*R*^{*5'*}, CONHOH, CONHO*R*^{*5*} et d'un tétrazolyle,
où *B* est lié à un atome de carbone du groupe *A,*
où les radicaux *R*^{*5*} et *R*^{*5'*} sont choisis indépendamment l'un de l'autre et présentent les significations indiquées ci-dessous,
*Y* représente O,
*R*^{*4*} et *R*^{*4'*} représentent un radical choisi parmi le groupe constitué de CF₃, C₂F₅, d'un alkyle en C₁₋₄, d'un alcényle en C₂₋₄, d'un alcynyle en C₂₋₃ et d'un (C₀₋₃-alcanediyl-C₃₋₇-cycloalkyle),
où un chaînon cyclique dans le noyau cycloalkyle peut être un azote cyclique ou un oxygène cyclique, lorsque le noyau cycloalkyle est à cinq chaînons, ou un ou deux chaînons cycliques dans le noyau cycloalkyle peuvent chacun être des atomes d'azote cycliques et/ou d'oxygène cycliques, lorsque le noyau cycloalkyle est à six ou sept chaînons, où les atomes d'azote cyclique peuvent être substitués par un alkyle en C₁₋₃ ou un alcanoyle en C₁₋₃,
*R*^{*5*} et *R*^{*5'*} représentent chacun un radical choisi parmi le groupe constitué d'un alkyle en C₁₋₆, d'un alcényle en C₂₋₆, d'un alcynyle en C₂₋₆, où un atome de carbone dans au moins l'un des radicaux *R*^{*5*} et *R*^{*5'*} peut être remplacé par O, S, SO, SO₂, NH, un N-C₁₋₃-alkyle ou un N-C₁₋₃-alcanoyle,
en outre un (C₀₋₃-alcanediyl-C₃₋₇-cycloalkyle), où un chaînon cyclique dans le noyau cycloalkyle peut être un azote cyclique ou un oxygène cyclique, lorsque le noyau cycloalkyle est à cinq chaînons, ou un ou deux chaînons cycliques dans le noyau cycloalkyle peuvent chacun être un ou deux atomes d'azote cycliques et/ou d'oxygène cycliques, lorsque le noyau cycloalkyle est à six ou sept chaînons, où les atomes d'azote cyclique peuvent être substitués par au moins un radical choisi parmi le groupe constitué de radicaux alkyle en C₁₋₃ et de radicaux alcanoyle en C₁₋₃,
en outre un (C₀₋₃-alcanediyl-aryle) et un (C₀₋₃-alcanediyl-hétéroaryle), où le groupe hétéroaryle peut être à cinq ou six chaînons et peut renfermer un ou deux hétéroatomes choisis parmi le groupe constitué de N, S et O,
où, en outre, au moins l'un des radicaux alkyle et cycloalkyle de *R*^{*5*} et *R*^{*5'*} peut être substitué par jusqu'à deux radicaux choisis parmi le groupe constitué de CF₃, C₂F₅, OH, d'un O-C₁₋₃-alkyle, NH₂, d'un NH-C₁₋₃-alkyle, d'un NH-C₁₋₃-alcanoyle, d'un N(C₁₋₃-alkyle)₂, d'un N(C₁₋₃-alkyl)(C₁₋₃-alcanoyle), COOH, CONH₂ et d'un COO-C₁₋₃-alkyle, et au moins l'un des radicaux aryle et hétéroaryle de *R*^{*5*} et *R*^{*5'*} peut être substitué par jusqu'à deux radicaux choisis parmi le groupe constitué de F, CI, Br, CH₃, C₂H₅, OH, OCH₃, OC₂H₅, NO₂, N(CH₃)₂, CF₃, C₂F₅ et SO₂NH₂, et/ou au moins l'un des radicaux alkyle, cycloalkyle, aryle et/ou hétéroaryle de *R*^{*5*} et *R*^{*5'*} peut porter un groupe méthanediylbisoxy ou éthane-1,2-diylbisoxy cyclisé,
ou où *R*^{*5*} et *R*^{*5'*} peuvent former, conjointement avec l'atome d'azote d'amide de *B*, un noyau hétérocyclique saturé ou insaturé à cinq à sept chaînons, qui peut renfermer un atome d'azote, d'oxygène ou de soufre supplémentaire et peut être substitué par un alkyle en C₁₋₄, un (C₀₋₂-alcanediyl-C₁₋₄-alcoxy), un C₁₋₄-alcoxycarbonyle, un aminocarbonyle ou un aryle,
*R*^{*6*} représente un radical choisi parmi le groupe constitué d'un (C₀₋₃-alcanediyl-aryle) et d'un (C₀₋₃-alcanediyl-hétéroaryle), où le groupe hétéroaryle est à cinq ou six chaînons et renferme un ou deux hétéroatomes choisis parmi le groupe constitué de N, S et O, et
où au moins l'un des groupes aryle et hétéroaryle peut être substitué dans chaque cas par jusqu'à deux radicaux choisis parmi le groupe constitué de F, Cl, Br, CH₃, C₂H₅, OH, OCH₃, OC₂H₅, NO₂, N(CH₃)₂, CF₃, C₂F₅ et SO₂NH₂, ou au moins l'un des groupes aryle ou hétéroaryle peut également porter un groupe méthanediylbisoxy ou éthane-1,2-diylbisoxy cyclisé.

2. Dérivés de benzimidazole selon la revendication 1, **caractérisés en ce que** *R*^{*1*} représente un phényle et peut être substitué par jusqu'à deux radicaux indépendamment l'un de l'autre, choisis parmi le groupe constitué de F, Cl, Br, C(NH)NH₂, C(NH)NH*R*^{*4*}, C(NH)NH*R*^{*4*}*R*^{*4'*}, C(N*R*^{*4*})NH₂, C(N*R*^{*4*})NH*R*^{*4'*}, C(N*R*⁴)N*R*^{*4*}*R*^{4'}, OH, O*R*^{*4*}, OCO*R*^{*4*}, OCONH*R*^{*4*}, CO*R*^{*4*}, C(NOH)*R*⁴, CN, COOH, COO*R*^{*4*}, CONH₂, CON*R*^{*4*}*R*^{*4'*}, CONH*R*^{*4*}, CONHOH, S*R*^{*4*}, SO*R*^{*4*}, SO₂*R*^{*4*}, SO₂NH₂, SO₂NH*R*^{*4*}, SO₂N*R*^{*4*}*R*^{*4'*}, NO₂, NH₂, NH*R*^{*4*}, N*R*^{*4*}*R*^{*4'*}, NHCONH*R*^{*4*} et *R*^{*4*}.

3. Dérivés de benzimidazole selon la revendication 1, **caractérisés en ce que** *R*^{*3*} est un radical choisi parmi le groupe constitué d'un hydrogène, de F, Cl, Br, CH₃, C₂H₅, CF₃, C₂F₅, OH, O*R*^{*4*}, NHSO₂*R*^{*6*} et NHCO*R*^{*4*}.

4. Dérivés de benzimidazole selon la revendication 1, **caractérisés en ce que** *B* représente un radical choisi parmi le groupe constitué de COOH, COO*R*^{*5*}, CONH₂, CONH*R*^{*5*} et CON*R*^{*5*}*NR*^{*5'*}.

5. Dérivés de benzimidazole selon la revendication 1, **caractérisés en ce que** *B-A-Y* se trouve en position 6 du benzimidazole.

6. Dérivés de benzimidazole selon la revendication 1, **caractérisés en ce que** *R*^{*6*} représente un groupe phényle ou hétéroaryle, où le groupe hétéroaryle est à cinq ou six chaînons et renferme un ou deux hétéroatomes choisis parmi le groupe constitué de N, S et O.

7. Benzimidazoles de formule générale I selon la revendication 1, dans laquelle
*R*^{*1*} représente un groupe phényle qui peut être substitué par jusqu'à deux radicaux indépendamment l'un de l'autre, choisis parmi le groupe constitué de :
F, Cl, Br,
C(NH)NH₂, C(NH)NH*R*^{*4*}, C(NH)N*R*^{*4*}*R*^{*4'*}*,* C(N*R*^{*4*})NH₂, C(N*R*^{*4*})NH*R*^{*4'*},
C(N*R*^{*4*})N*R*^{*4*}*R*^{*4'*},
OH, O*R*^{*4*}, OCO*R*^{*4*}, OCONH*R*^{*4*},
CO*R*^{*4*}, C(NOH)*R*^{*4*},
CN, COOH, COO*R*^{*4*}, CONH₂, CON*R*^{*4*}*R*^{*4'*}, CONH*R*^{*4*}, CONHOH,
S*R*^{*4*}, SO*R*^{*4*}, SO₂*R*^{*4*},
SO₂NH₂, SO₂NH*R*^{*4*}, SO₂N*R*^{*4*}*R*^{*4'*},
NO₂, NH₂, NH*R*^{*4*}, N*R*^{*4*}*R*^{*4'*}, NHCONH*R*^{*4*} et
*R*^{*4*},
où les radicaux *R*^{*4*} et *R*^{*4'*} sont choisis indépendamment l'un de l'autre conformément aux significations indiquées ci-après et
où deux substituants sur *R*^{*1*} sont liés l'un à l'autre de telle sorte qu'ils forment conjointement un groupe méthanediylbisoxy, éthane-1,2-diylbisoxy, propan-1,3-diyle ou butan-1,4-diyle, lorsqu'ils sont en position ortho l'un par rapport à l'autre,
*Z* représente NH, N*R*^{*2*}, N*R*^{*2'*}, O, SO ou SO₂,
*R*^{*2*} et *R*^{*2'*} représentent chacun, indépendamment l'un de l'autre, un radical choisi parmi le groupe constitué de :
un perfluoroalkyle en C₁₋₄, un alkyle en C₁₋₆, un (C₀₋₃-alcanediyl-aryle) et un (C₀₋₃-alcanediyl-hétéroaryle),
où le groupe hétéroaryle est à cinq ou six chaînons et renferme un ou deux hétéroatomes choisis parmi le groupe constitué de N, S et O, et
où les groupes aryle et hétéroaryle peuvent chacun être substitués par jusqu'à deux radicaux choisis parmi le groupe constitué de F, Cl, Br, CH₃, C₂H₅, OH, OCH₃, OC₂H₅, NO₂, CF₃, C₂F₅ et SO₂NH₂,
ou lorsque *Z* est N*R*^{*2'*}, *R*^{*2*} et *R*^{*2'*} forment, conjointement avec *Z*, un noyau hétérocyclique à cinq à sept chaînons, où en outre le noyau hétérocyclique peut renfermer un atome d'oxygène ou de soufre supplémentaire et peut être éventuellement substitué par un radical choisi parmi le groupe constitué d'un alkyle en C₁₋₄, d'un (C₀₋₃-alkanediyl-C₁₋₃-alcoxy), d'un alcanoyle en C₁₋₄, d'un C₁₋₄-alcoxycarbonyle, d'un aminocarbonyle et d'un aryle,
*R*^{*3*} représente un hydrogène,
*A* représente un alcanediyle linéaire ou ramifié renfermant jusqu'à 8 atomes de carbone,
*B* représente un radical choisi parmi le groupe constitué de COOH, COO*R*⁵, CONH₂, CONH*R*^{*5*} et CON*R*^{*5*}*R*^{*5'*}, dans chaque cas lié à un atome de carbone du groupe *A*,
où les radicaux *R*^{*5*} et *R*^{*5'*} sont choisis indépendamment l'un de l'autre conformément aux significations indiquées ci-dessous,
*Y* représente *O*
où, dans les radicaux ci-dessus, les radicaux *R*^{*4*}, *R*^{*4'*}, *R*^{*5*} et *R*^{*5'*} présentent les significations suivantes :
*R*^{*4*} et *R*^{*4*}*'* représentent chacun, indépendamment l'un de l'autre, un radical choisi parmi le groupe constitué de CF₃, C₂F₅, d'un alkyle en C₁₋₄, d'un alcényle en C₂₋₄, d'un alcynyle en C₂₋₃ et d'un (C₀₋₃-alcanediyl-C₃₋₇-cycloalkyle),
où les radicaux alkyle peuvent éventuellement être substitués par un radical choisi parmi le groupe constitué de OH, OCH₃ et SCH₃,
*R*^{*5*} et *R*^{*5'*} représentent chacun, indépendamment l'un de l'autre, un radical choisi parmi le groupe constitué d'un alkyle en C₁₋₆, d'un alcényle en C₂₋₆, d'un alcynyle en C₂₋₆, d'un (C₀₋₃-alcanediyl-C₃₋₇-cycloalkyle), d'un (C₀₋₃-alcanediyl-phényle) et d'un (C₀₋₃-alcanediyl-hétéroaryle), où le groupe hétéroaryle est à cinq ou six chaînons et renferme un ou deux hétéroatomes choisis parmi le groupe constitué de N, S et O,
où tous les radicaux alkyle et cycloalkyle mentionnés précédemment peuvent être substitués par un radical choisi parmi le groupe constitué de CF₃, C₂F₅, OH, d'un O-C₁₋₃-alkyle, NH₂, d'un NH-C₁₋₃-alkyle, d'un NH-C₁₋₃-alcanoyle, d'un N(C₁₋₃-alkyle)₂, d'un N(C₁₋₃-alkyl)(C₁₋₃-alcanoyle), COOH, CONH₂ et d'un COO-C₁₋₃-alkyle, et tous les groupes phényle et hétéroaryle mentionnés précédemment peuvent être substitués par jusqu'à deux radicaux choisis parmi le groupe constitué de F, Cl, Br, CH₃, C₂H₅, OH, OCH₃, OC₂H₅, NO₂, N(CH₃)₂, CF₃, C₂F₅ et SO₂NH₂, et/ou peuvent également porter un groupe méthanediylbisoxy ou éthan-1,2-diylbisoxy cyclisé,
ou *R*^{*5*} et *R*^{*5'*} forment, conjointement avec l'atome d'azote d'amide de *B*, un noyau hétérocyclique saturé ou insaturé, à cinq à sept chaînons, qui peut renfermer un atome d'azote, d'oxygène ou de soufre supplémentaire qui peut être substitué par un alkyle en C₁₋₄, un (C₀₋₂-alcanediyl-C₁₋₄-alcoxy), un C₁₋₄-alcoxycarbonyle, un aminocarbonyle ou un phényle.

8. Dérivés de benzimidazole selon la revendication 7, **caractérisés en ce que** *R*^{*5*} et *R*^{*5'*} représentent chacun un radical choisi parmi le groupe constitué d'un alkyle en C₁₋₆, d'un alcényle en C₂₋₆, d'un alcynyle en C₂₋₆, d'un (C₀₋₃-alcanediyl-C₃₋₇-cycloalkyle), d'un (C₀₋₃-alcanediyl-aryle) et d'un (C₀₋₃-alcanediyl-hétéroaryle), où aryle représente un phényle et les radicaux alkyle et cycloalkyle peuvent être substitués par un radical choisi parmi le groupe constitué de CF₃, C₂F₅, OH, d'un O-C₁₋₃-alkyle, NH₂, d'un NH-C₁₋₃-alkyle, d'un NH-C₁₋₃-alcanoyle, d'un N(C₁₋₃-alkyle)₂, d'un N(C₁₋₃-alkyl)(C₁₋₃-alcanoyle), COOH, CONH₂ et d'un COO-C₁₋₃-alkyle.

9. Dérivés de benzimidazole selon la revendication 7, **caractérisés en ce que** aryle dans *R*^{*1*} représente un phényle (C₆H₅₋) ou un *p*-méthylphényle (*p*-CH₃-C₆H₄-).

10. Dérivés de benzimidazole selon la revendication 7, **caractérisés en ce que** *R*^{*2*} représente un alkyle en C₁₋₃, un phényle, un méthylphénylène, un benzyle ou un hétéroaryle.

11. Dérivés de benzimidazole de formule générale 1 selon la revendication 1, à savoir
l'ester méthylique de l'acide 6-[[1-phényl-2-propylmercapto-1*H*-benzimidazol-6-yl]oxy]hexanoïque,
l'ester méthylique de l'acide 6-[[1-phényl-2-propanesulfinyl-1*H*-benzimidazol-6-yl]oxy]hexanoïque,
l'ester méthylique de l'acide 6-[[1-phényl-2-propanesulfonyl-1*H*-benzimidazol-6-yl]oxy]hexanoïque,
l'ester méthylique de l'acide 6-[[1-méthylphényl)-2-propylmercapto-1*H*-benzimidazol-6-yl]oxy]hexanoïque,
l'ester méthylique de l'acide 6-[[1-(4-méthylphényl)-2-propanesulfinyl-1*H*-benzimidazol-6-yl]oxy]hexanoïque,
l'ester méthylique de l'acide 6-[[1-(4-méthylphényl-2-propanesulfonyl-1*H*-benzimidazol-6-yl]oxy]hexanoïque,
l'ester méthylique de l'acide 6-[[2-benzylmercapto-1-(4-méthylphényl)-1*H*-benzimidazol-6-yl]oxy]hexanoïque,
l'ester méthylique de l'acide 6-[[1-(4-méthylphényl)-2-(phénylméthanesulfinyl)-1*H*-benzimidazol-6-yl]oxy]hexanoïque,
l'ester méthylique de l'acide 6-[[1-(4-méthylphényl)-2-(phénylméthanesulfonyl)-1*H*-benzimidazol-6-yl]oxy]hexanoïque,
l'ester méthylique de l'acide 6-[[1-(4-méthylphényl)-2-(2-pyridinyl)mercapto-1*H*-benzimidazol-6-yl]oxy]hexanoïque,
le *N*-(3-méthoxypropyl)-6-[[1-(4-méthylphényl)-2-propylmercapto-1*H*-benzimidazol-6-yl]oxy]hexanamide,
le *N*-(3-méthoxypropyl)-6-[[1-(4-méthylphényl)-2-propanesulfonyl-1*H* -benzimidazol-6-yl]oxy]hexanamide,
le *N*-(3-méthoxypropyl)-6-[[2-benzylmercapto-1-(4-méthylphényl)-1*H*-benzimidazol-6-yl]oxy],
le *N*-(3-méthoxypropyl)-6-[[1-(4-méthylphényl)-2-(phénylméthanesulfonyl)-1*H*-benzimidazol-6-yl]oxy]hexanamide,
l'ester méthylique de l'acide 6-[[2-(morpholin-4-yl)-1-phényl-1*H*-benzimidazol-6-yl]oxy]hexanoïque,
l'ester méthylique de l'acide 6-[[2-(pipéridin-1-yl)-1-phényl-1*H*-benzimidazol-6-yl]oxy]hexanoïque,
l'ester méthylique de l'acide 6-[[1-(4-méthylphényl)-2-(morpholin-4-yl)-1*H*-benzimidazol-6-yl]oxy]hexanoïque,
l'ester méthylique de l'acide 6-[[1-(4-méthylphényl)-2-(pipéridin-1-yl)-1*H*-benzimidazol-6-yl]oxy]hexanoïque,
le *N*-(3-méthoxypropyl)-6-[[2-(morpholin-4-yl)-1-phényl-1*H*-benzimidazol-6-yl]oxy]hexanamide,
le *N*-(3-méthoxypropyl)-6-[[2-(pipéridin-1-yl)-1- phényl-1*H*-benzimidazol-6-yl]oxy]hexanamide,
l'ester méthylique de l'acide 6-[[2-méthoxy-1-phényl-1*H*-benzimidazol-6-yl]oxy]hexanoïque,
l'acide 6-[[2-méthoxy-1-phényl-1*H*-benzimidazol-6-yl]oxy]hexanoïque,
l'ester méthylique de l'acide 6-[[2-éthoxy-1-phényl-1*H*-benzimidazol-6-yl]oxy]hexanoïque,
l'acide 6-[[2-éthoxy-1-phényl-1*H*-benzimidazol-6-yl]oxy]hexanoïque,
l'ester méthylique de l'acide 6-[[1-phényl-2-phénylamino-1*H*-benzimidazol-6-yl]oxy]hexanoïque,
l'acide 6-[[1-phényl-2-phénylamino-1*H*-benzimidazol-6-yl]oxy]hexanoïque,
l'ester méthylique de l'acide 6-[[1-(4-méthylphényl)-2-phénylamino-1*H*-benzimidazol-6-yl]oxy]hexanoïque,
l'ester méthylique de l'acide 6-[[1-phényl-2-propylamino-1*H*-benzimidazol-6-yl]oxy]hexanoïque,
l'acide 6-[[1-phényl-2-propylamino-1*H*-benzimidazol-6-yl]oxy]hexanoïque,
l'ester méthylique de l'acide 6-[[2-(*N*-méthyl-*N*-propyl)amino-1-phényl-1*H*-benzimidazol-6-yl]oxy]hexanoïque,
l'acide 6-[[1-(4-méthylphényl)-2-phényloxy-1*H*-benzimidazol-6-yl]oxy]hexanoïque,
l'ester méthylique de l'acide 6-[[1-(4-méthylphényl)-2-phénylmercapto-1*H*-benzimidazol-6-yl]oxy]hexanoïque,
l'ester méthylique de l'acide 6-[[1-(4-méthylphényl)-2-(phénylsulfinyl)-1*H*-benzimidazol-6-yl]oxy]hexanoïque,
l'ester méthylique de l'acide 6-[[1-(4-méthylphényl)-2-(phénylsulfonyl)-1*H*-benzimidazol-6-yl]oxy]hexanoïque,
l'acide 6-[[1-phényl-2-propylmercapto-1*H*-benzimidazol-6-yl]oxy]hexanoïque,
l'acide 6-[[1-phényl-2-propanesulfonyl-1*H*-benzimidazol-6-yl]oxy]hexanoïque,
l'ester méthylique de l'acide 6-[[2-benzylmercapto-1-phényl-1*H*-benzimidazol-6-yl)oxy]hexanoïque,
l'ester méthylique de l'acide 6-[[1-phényl-2-(phénylméthanesulfonyl)-1*H*-benzimidazol-6-yl]oxy]hexanoïque,
l'acide 6-[(2-benzylmercapto-1-phényl-1*H*-benzimidazol-6-yl)oxy]hexanoïque,
l'acide 6-[[1-phényl-2-(phénylméthanesulfonyl)-1*H*-benzimidazol-6-yl]oxy]hexanoïque,
l'acide 6-[[1-(4-méthylphényl)-2-propylmercapto-1*H*-benzimidazol-6-yl]oxy]hexanoïque,
l'acide 6-[[1-(4-méthylphényl)-2-propanesulfonyl-1*H*-benzimidazol-6-yl]oxy]hexanoïque,
l'acide 6-[[2-benzylmercapto-1-(4-méthylphényl)-1*H*-benzimidazol-6-yl]oxy]hexanoïque,
l'acide 6-[[1-(4-méthylphényl)-2-(phénylméthanesulfonyl)-1*H*-benzimidazol-6-yl]oxy]hexanoïque.

12. Utilisation des dérivés de benzimidazole de formule générale I selon l'une quelconque des revendications 1 à 11 pour la production de médicaments destinés au traitement de maladies associées à une activation de la microglie ainsi qu'à la prophylaxie contre ces maladies, où les dérivés de benzimidazole comprennent en outre également des composés dans lesquels *B* représente un hydrogène.

13. Préparations pharmaceutiques comprenant au moins un dérivé de benzimidazole de formule générale I selon l'une quelconque des revendications 1 à 11 ainsi qu'au moins un support pharmaceutiquement acceptable.

14. Utilisation d'un composé selon l'une quelconque des revendications 1 à 11 pour la production d'un médicament destiné au traitement de maladies inflammatoires, allergiques, infectieuses ou auto-immunes.

15. Utilisation selon la revendication 14 pour le traitement d'une attaque cérébrale.
